# EUROPEAN PATENT APPLICATION

(11) **EP 3 088 521 A1**
(43) Date of publication of application: **02.11.2016**
(21) Application number: 14874196.0
(22) Date of filing: 25.12.2014
(51) Int. Cl.: C12N 15/09, C12N 15/01, C12Q 1/68

(54) **METHOD FOR DETECTING LUNG CANCER**

(30) Priority: 25.12.2013 JP 2013267568
(71) Applicant: Saga University, Saga-shi, Saga 840-8502 (JP); ARKRAY, Inc., Minami-ku Kyoto-shi Kyoto 601-8045 (JP)
(72) Inventor: ARAGANE, Naoko, Saga-shi Saga 840-8502 (JP); KIMURA, Shinya, Saga-shi Saga 840-8502 (JP); WATANABE, Naomi, Saga-shi Saga 840-8502 (JP)
(74) Representative: Jones, Elizabeth Louise
(86) International application number: PCT/JP2014/084755
(87) International publication number: WO 2015/099197

(57) **Abstract**

Provided is a lung cancer biomarker containing a DDR2 protein or a DDR2 gene, or a mutant thereof, i.e., the following (a), (b) and (c): (a) overexpressed DDR2 protein or DDR2 gene; (b) overexpressed DDR2 protein or DDR2 gene mutant; and (c) DDR2 protein or DDR2 gene mutant.

## Description

### TECHNICAL FIELD

The present invention relates to a method for examining lung cancer using a DDR2 protein, a DDR2 gene or a mutant thereof as an indicator.

### BACKGROUND ART

Primary lung cancers are diseases with poor prognosis that still account for the highest number of deaths, among the various incidence regions, in Japan. Although prognosis for adenocarcinoma that accounts for about 40% of the primary lung cancers is improving due to the recent progress in elucidation of genes as therapeutic targets, genes as therapeutic targets for squamous cell carcinoma that accounts for about 35% of the primary lung cancers have hardly been elucidated.

Recently, Peter S Hammerman et al. reported that about 3.8% of the squamous cell lung carcinomas had a mutation in discoidin domain receptor tyrosine kinase 2 (DDR2), which was associated with the effect of dasatinib, i.e., a therapeutic agent for chronic myeloid leukemia (Cancer Discovery 2011;1(1):78-89, Non-Patent Document 1).

DDR2 is a receptor tyrosine kinase that binds with collagen as the ligand, and plays a role in cell adhesion, cell proliferation, extracellular remodeling and the like. Besides squamous cell lung carcinoma, mutations associated with 18 types of amino-acid changes at 17 sites have also been reported in large cell lung carcinoma, clear cell renal carcinoma and else, but its role in cancer cells has not yet been fully understood.

### RELATED ART DOCUMENTS

### Non-Patent Document

Non-Patent Document 1: Cancer Discovery 2011;1(1):78-89

### SUMMARY OF THE INVENTION

### Problems to be Solved by the Invention

The present invention has an objective in providing a method for examining lung cancer using an amino acid mutation of a DDR2 protein or a mutation of a DDR2 gene as an indicator.

### Means for Solving the Problems

In order to solve the above-described problem, the present inventors have gone through keen studies, as a result of which found that overexpression of a DDR2 protein or a DDR2 gene, as well as a mutation of a predetermined amino acid residue of a DDR2 protein and a mutation of a predetermined base of a DDR2 gene are associated with lung cancer, in particular, squamous cell lung carcinoma, thereby accomplishing the present invention.

Thus, the present invention is as follows.
(1) A biomarker for lung cancer, comprising a DDR2 protein, a DDR2 gene or a mutant thereof of (a), (b) or (c) below:
   (a) an overexpressed DDR2 protein or DDR2 gene;
   (b) a mutant of an overexpressed DDR2 protein or DDR2 gene; or
   (c) a mutant of a DDR2 protein or a DDR2 gene.
(2) The biomarker according to (1), wherein the DDR2 protein is a protein of (a) or (b) below:
   (a) a protein containing the amino acid sequence represented by SEQ ID NO:3; or
   (b) a protein that contains an amino acid sequence having one or several amino acids, other than the 681st amino acid, deleted, substituted or added in the amino acid sequence represented by SEQ ID NO:3, and that has DDR2 activity.
(3) The marker according to (1), wherein the DDR2 gene comprises DNA of (a) or (b) below:
   (a) DNA containing the nucleotide sequence represented by SEQ ID NO:2; or
   (b) DNA that hybridizes with a nucleotide sequence complementary to the nucleotide sequence represented by SEQ ID NO:2 under stringent conditions, and that codes for a protein having DDR2 activity.
(4) The biomarker according to (1), wherein the mutant of the DDR2 protein is a protein of (a) or (b) below:
   (a) a protein containing the amino acid sequence represented by SEQ ID NO:5; or
   (b) a protein that contains an amino acid sequence having one or several amino acids, other than the 681st amino acid, deleted, substituted or added in the amino acid sequence represented by SEQ ID NO:5, and that has DDR2 activity.
(5) The biomarker according to (4), wherein the mutation of the 681 st amino acid residue is a mutation from threonine to isoleucine (T681I).
(6) The biomarker according to (1), wherein the mutant of the DDR2 gene is one shown in (a), (b) or (c) below:
   (a) DNA coding for the amino acid sequence represented by SEQ ID NO:5;
   (b) DNA coding for a protein that contains an amino acid sequence having one or several amino acids, other than the 681st amino acid, deleted, substituted or added in the amino acid sequence represented by SEQ ID NO:5, and that has DDR2 activity; or
   (c) DNA having a mutation of the second nucleotide of the codon coding for the 681st amino acid residue of the nucleotide sequence of DDR2 gene.
(7) The biomarker according to (6), wherein the mutation of the second nucleotide is a mutation at the 148400th base of the nucleotide sequence of the DDR2 gene.
(8) The biomarker according to (6), wherein the mutation of the second nucleotide is a mutation from nucleotide C to nucleotide T.
(9) A method for examining lung cancer, comprising a step of determining an expression level of a DDR2 protein, a DDR2 gene or a mutant thereof in a test sample collected from a test subject, wherein, when the expression level indicates overexpression as compared to a control, the determination result is used as an indicator of lung cancer or a risk of lung cancer.
(10) A method for examining lung cancer, comprising a step of analyzing the presence or absence of a mutation in a DDR2 protein or a DDR2 gene in a test sample collected from a test subject, wherein, when a mutation is present in a predetermined amino acid sequence of the DDR2 protein or in a predetermined nucleotide sequence of the DDR2 gene, the analysis result is used as an indicator of lung cancer or a risk of lung cancer.
(11) The method according to (9) or (10), wherein the test sample is at least one selected from the group consisting of a surgically resected analyte, a bronchoscopic analyte and a bronchial lavage fluid analyte.
(12) A method for examining cancer, wherein a test subject is judged to have lung cancer or a risk of lung cancer when the expression level of a DDR2 protein, a DDR2 gene or a mutant thereof indicates overexpression by the method according to (9) or (11).
(13) A method for examining cancer, wherein the test subject is judged to have lung cancer or a risk of lung cancer when a mutation is present in an amino acid sequence of a DDR2 protein or in a nucleotide sequence of a DDR2 gene by the method according to (10) or (11).
(14) The method according to any one of (9)-(13), wherein an examination of lung cancer is detection of lung cancer or estimation of a nature of lung cancer.
(15) The method according to (9) or (10), wherein the mutant of the DDR2 protein results from a mutation of the 681 st amino acid residue in the amino acid sequence of the DDR2.
(16) The method according to (15), wherein the mutation of the 681st amino acid residue is a mutation from threonine to isoleucine (T681I).
(17) The method according to (15), wherein the analysis of the presence or absence of the mutation of the 681 st amino acid residue is an analysis of the presence or absence of a mutation of the second nucleotide of the codon coding for the 681 st amino acid residue in the nucleotide sequence of the DDR2 gene.
(18) The method according to (17), wherein the mutation of the second nucleotide is a mutation from nucleotide C to nucleotide T.
(19) The method according to (9) or (10), wherein the mutation of the DDR2 gene is a mutation of the 148400th base in the nucleotide sequence of the DDR2 gene.
(20) The method according to (19), wherein when the gene mutation is T/C heterozygous, it is judged that lung cancer or a risk of lung cancer is present.
(21) The method according to any one of (9)-(20), wherein the lung cancer is squamous cell lung carcinoma.
(22) An oligonucleotide comprising a sequence of at least 10 bases containing the 148400th base of the nucleotide sequence of the DDR2 gene, or a sequence complementary thereto.
(23) The oligonucleotide according to (22), having a length of 10-2565 bases.
(24) A probe having the oligonucleotide according to (22) or (23) labeled.
(25) The probe according to (24), wherein the oligonucleotide has a length of 10-30 consecutive bases.
(26) A kit for examining lung cancer, comprising at least one selected from the group consisting of the oligonucleotides according to (22) and (23) and the probes according to (24) and (25).
(27) A method for treating lung cancer, comprising a step of administering an anticancer drug to a test subject who has been judged to have lung cancer by the method according to (12) or (13).
(28) A method for treating lung cancer, comprising a step of administering a substance that inhibits the expression of a DDR2 protein, a DDR2 gene or a mutant thereof to a patient with the lung cancer.
(29) The method according to (28), wherein the substance that inhibits the expression is a substance shown in (a) or (b) below:
   (a) an antibody for a DDR2 protein or a mutant thereof; or
   (b) an antisense nucleic acid, a decoy nucleic acid, microRNA, siRNA or shRNA for a DDR2 gene or a mutant thereof.
(30) A pharmaceutical composition for treating lung cancer, comprising a substance that inhibits the expression of a DDR2 protein, a DDR2 gene or a mutant thereof.
(31) The pharmaceutical composition according to (30), wherein the substance that inhibits the expression is a substance shown in (a) or (b) below:
   (a) an antibody for a DDR2 protein or a mutant thereof; or
   (b) an antisense nucleic acid, a decoy nucleic acid, microRNA, siRNA or shRNA for a DDR2 gene or a mutant thereof.

### EFFECT OF THE INVENTION

The present invention provides a method and a kit for examining lung cancer. By using the method of the present invention, a patient with lung cancer can easily and unfailingly be detected or diagnosed. Accordingly, the method of the present invention is useful for detecting lung cancer, understanding pathological condition, diagnosing, and developing a molecularly targeted therapy.

### BRIEF DESCRIPTION OF DRAWINGS

Figure 1 gives diagrams showing a mutation of a gene in a lung cancer tissue and a lung cancer cell line.
Figure 2 gives images showing results from an Invasion assay.
Figure 3 gives diagrams showing results from an Invasion assay.
Figure 4 gives a diagram showing results from analyzing TGFβ1 expression levels by real-time PCR.
Figure 5 gives a diagram showing results from analyzing TGFβ1 expression levels by ELISA.
Figure 6 gives images showing results from a cell transplantation experiment with metastatic mouse models.

### MODES FOR CARRYING OUT THE INVENTION

Hereinafter, the present invention will be described in detail. The following embodiments are illustrations for explaining the present invention, and the present invention should not be limited thereto. The present invention may be carried out in various embodiments without departing from the scope thereof.

The documents and patent documents such as patent publications cited herein are incorporated herein by reference. The present specification also includes the content of the specification of Japanese patent Application No. 2013-267568 (filed on December 25, 2013) based on which the present application claims priority.

The present inventors found that a predetermined mutation in an amino acid sequence of a DDR2 (discoidin domain receptor tyrosine kinase 2) protein or in a nucleotide sequence of a DDR2 gene collected from a test subject is associated with lung cancer. Moreover, as the present inventors determined the expression level of a DDR2 protein, a DDR2 gene or a mutant thereof, they found that overexpression was associated with lung cancer. Specifically, they found that when a predetermined mutation is present in an amino acid sequence of a DDR2 protein or in a nucleotide sequence of a DDR2 gene collected from a test subject, this mutation can be used as an indicator representing that the test subject has lung cancer or a risk thereof, or when a DDR2 protein, a DDR2 gene or a mutant thereof is overexpressed as compared to a control, this overexpression can be used as an indicator representing that the test subject has lung cancer or a risk thereof.

Hence, the present invention provides a biomarker for lung cancer, comprising a DDR2 protein, a DDR2 gene or a mutant thereof of (a), (b) or (c) below:
(a) an overexpressed DDR2 protein or DDR2 gene;
(b) a mutant of an overexpressed DDR2 protein or DDR2 gene; or
(c) a mutant of a DDR2 protein or a DDR2 gene.

In addition, the present invention provides a method for examining lung cancer, comprising a step of determining an expression level of a DDR2 protein, a DDR2 gene or a mutant thereof in a test sample collected from a test subject, wherein, when the expression level indicates overexpression as compared to a control, the determination result is judged to represent lung cancer or a risk of lung cancer. Furthermore, the present invention provides a method for examining lung cancer, comprising a step of analyzing the presence or absence of a mutation in a DDR2 protein or a DDR2 gene in a test sample collected from a test subject, wherein, when the mutation is present in the amino acid sequence of the DDR2 protein or in the nucleotide sequence of the DDR2 gene, the analysis result is judged to represent lung cancer or a risk of lung cancer.

According to one aspect of the present invention, a method for examining lung cancer comprises a step of analyzing the presence or absence of the mutation of the 681 st amino acid residue in an amino acid sequence of a DDR2 (discoidin domain receptor tyrosine kinase 2) collected from a test subject to examine lung cancer of the test subject based on this analysis result.

The present inventors used a total of 81 surgically resected analytes and bronchoscopic analytes as well as 26 lung cancer cell lines for sequencing all exon regions of DDR2. In one analyte out of the 81 clinical analyte and in one cell line out of the 26 cell lines, a mutation from threonine to isoleucine was found at the 681 st amino acid residue

(T681I). This mutation was found on a kinase domain, and was a previously unreported novel mutation. The present invention was accomplished by focusing on this novel mutation as a novel marker of lung cancer. Accordingly, the present invention provides a method for examining lung cancer by analyzing the presence or absence of the mutation of the 681st amino acid residue in the amino acid sequence of a DDR2 protein, or the mutation of the gene coding for the 681 st amino acid residue in a DDR2 gene to correlate this analysis result with lung cancer of a test subject.

According to the present invention, "examination" of lung cancer refers to detection of lung cancer and/or estimation of a nature of lung cancer using the expression level of a DDR2 protein, a DDR2 gene or a mutant thereof as an indicator, or using the presence of the mutation in a DDR2 protein or a DDR2 gene as an indicator.

Specifically, it refers to detection of lung cancer and/or estimation of a nature of lung cancer by using a mutation of the 681 st amino acid residue in an amino acid sequence of DDR2 (for example, SEQ ID NO:3) as an indicator, or by using a mutation in a nucleotide sequence of a gene coding for DDR2, for example, a mutation of the 148400th base in the nucleotide sequence represented by SEQ ID NO:1 or a mutation of the 2042nd base in the nucleotide sequence represented by SEQ ID NO:2, as an indicator. The term "examination" also includes an aspect of analysis and an aspect of mutational analysis.

Herein, the ordinal numerals "681 st" and "148400th" represent the positions of the amino acid residue and the base targeted for the analysis, respectively, by numbers in the full-length sequences of the DDR2 protein and the DDR2 gene. Therefore, even when a DDR2 protein or a DDR2 gene used in the actual analysis is, for example, a partial fragment that is shorter than the full length, the targeted position refers to the "681 st" and the "148400th", respectively. In SEQ ID NOS:2 and 4, the 2042nd base corresponds to the above-described "148400th" base.

The phrase "to detect lung cancer" includes judgment, diagnosis or preliminary diagnosis of whether a test subject has lung cancer, and also includes judgment, diagnosis or preliminary diagnosis of whether a test subject who does not actually suffer from a lung cancer has a risk of suffering from lung cancer in the future. The term "preliminary diagnosis" also includes an aid for a physician to make a final diagnosis, in other words, an aid for diagnosing lung cancer.

Furthermore, the phrase "to estimate a nature of lung cancer" refers to estimation of the type, whether it is primary, or else of the lung cancer detected in a test subject. For example, since T681I mutation of a DDR2 protein is found in squamous cell lung carcinoma, lung cancer with the same mutation can be estimated, for example, to be squamous cell lung carcinoma. The above-described estimation also includes an aid for a physician to make a final diagnosis, in other words, an aid for diagnosing lung cancer.

### 1. DDR2 protein

A protein targeted by the examination of the present invention is a wild-type protein or a mutant protein of a DDR2 protein. A wild-type DDR2 protein is targeted by an examination that uses overexpression as an indicator. A mutant protein is targeted by an examination that uses either overexpression or a mutation as an indicator.

According to the present invention, a wild-type DDR2 protein is specifically a protein of (a) or (b) below:
(a) a protein containing the amino acid sequence represented by SEQ ID NO:3; or
(b) a protein that contains an amino acid sequence having one or several amino acids, other than the (681st) amino acid at the above-described substitution site, deleted, substituted or added in the amino acid sequence represented by SEQ ID NO:3, and that has DDR2 activity.

Herein, the protein of (b) is referred to as a "modified wild-type protein".

A mutant protein is a mutant DDR2 protein (hereinafter, also referred to as a "mutant DDR2 protein") in which the 681st amino acid residue has been mutated from threonine to an amino acid other than threonine (for example, isoleucine) in the amino acid sequence of DDR2, which is represented by SEQ ID NO:5. In the amino acid sequence represented by SEQ ID NO:5, the mutation of the 681st amino acid residue is based on a mutation of the second nucleotide of the codon coding for the 681 st amino acid residue in the nucleotide sequence of DDR2 gene (SEQ ID NO:1 or 2). In the case where the 681st amino acid residue is mutated, for example, from threonine to isoleucine, the mutation would be ACT to ATT at the 148399th-148401st bases of the nucleotide sequence represented by SEQ ID NO:1, or the mutation would be from ACT to ATT at the 2041st-2043rd bases of the nucleotide sequence represented by SEQ ID NO:2. The present invention provides both of such mutant DDR2 protein and such gene coding for said protein.

Specifically, a mutant DDR2 protein of the present invention is a protein of (a) or (b) below:
(a) a protein containing the amino acid sequence represented by SEQ ID NO:5 (a protein containing the amino acid sequence having the 681st amino acid substituted into an amino acid other than threonine in the amino acid sequence represented by SEQ ID NO:3); or
(b) a protein including an amino acid sequence having one or several amino acids, other than the 681st amino acid, deleted, substituted or added in the amino acid sequence represented by SEQ ID NO:5, and having DDR2 activity.

Herein, the protein of (b) is referred to as a "modified mutant protein".

The above-mentioned amino acid other than threonine is not particularly limited as long as it is an amino acid other than threonine but it is preferably a hydrophobic amino acid (tryptophan, methionine, proline, phenylalanine, alanine, valine, leucine, isoleucine), and more preferably isoleucine.

### 2. DDR2 gene and mutant thereof

A gene targeted by the examination of the present invention is a DDR2 gene (wild-type) or a mutant gene thereof. A wild-type DDR2 gene is targeted by an examination that uses overexpression as an indicator. A mutant DDR2 gene is targeted by an examination that uses either overexpression or a mutation as an indicator.

Examples of a wild-type DDR2 gene include those containing DNA of (a) or (b) below:
(a) DNA containing the nucleotide sequence represented by SEQ ID NO:1 or 2; or
(b) DNA that hybridizes with a nucleotide sequence complementary to the nucleotide sequence represented by SEQ ID NO:1 or 2 under stringent conditions, and that codes for a protein having DDR2 activity protein.

In the above-described DNA of (b), the 148399th-148401st bases of the nucleotide sequence represented by SEQ ID NO:1 (the 2041st-2043rd bases in case of SEQ ID NO:2) are identical to the DNA of (a).

Herein, this DNA of (b) is referred to as a "modified wild-type DNA".

In addition, the present invention provides a gene coding for a mutant DDR2 protein (hereinafter, also referred to as a "mutant DDR2 gene").

Specifically, a mutant DDR2 gene of the present invention contains DNA having a nucleotide sequence of (a), (b), (c) or (d) below:
(a) a nucleotide sequence in which the 148399th-148401st bases in the nucleotide sequence represented by SEQ ID NO:1 have been substituted into a codon of an amino acid other than threonine;
(b) DNA that hybridizes with DNA consisting of a nucleotide sequence complementary to DNA containing the nucleotide sequence of (a) above under stringent conditions (providing that the 148399th-148401st bases are identical to (a) above), and that codes for a protein having DDR2 activity;
(c) the nucleotide sequence represented by SEQ ID NO:4 (a nucleotide sequence in which the 2041st-2043 bases in the nucleotide sequence represented by SEQ ID NO:2 have been substituted into a codon of an amino acid other than threonine); or
(d) DNA that hybridizes with DNA consisting of a nucleotide sequence complementary to DNA containing the nucleotide sequence of (c) above under stringent conditions (providing that the 2041st-2043rd bases are identical to (c) above), and that codes for a protein having DDR2 activity.

Herein, DNA of (b) and (d) are referred to as "modified mutant DNA".

Here, the phrase "under stringent conditions" refers to, but not limited to, conditions such as: (1) 5 x SSC, 5 x Denhardt's solution, 0.5% SDS, 50% formamide, 50°C; (2) 0.2 x SSC, 0.1% SDS, 60°C; (3) 0.2 x SSC, 0.1% SDS, 62°C; (4) 0.2 x SSC, 0.1% SDS, 65°C; or (5) 0.1 x SSC, 0.1% SDS, 65°C. Under these conditions, DNA having high sequence identity can be expected to be obtained efficiently by increasing the temperature. However, there are multiple factors that are considered to have an effect on the stringency of hybridization such as the temperature, the probe concentration, the probe length, the ion intensity, the time, the salt concentration and the like, which can suitably be selected by those skilled in the art to realize similar stringency.

The above-mentioned amino acid other than threonine is not particularly limited as long as it is an amino acid other than threonine but it is preferably a hydrophobic amino acid (tryptophan, methionine, proline, phenylalanine, alanine, valine, leucine, isoleucine), and more preferably isoleucine. While codons of isoleucine are ATA, ATC or ATT, it is preferably ATT.

The present inventors found that the genotype of the 148400th base of the DDR2 gene (SEQ ID NO:1) derived from a patient with lung cancer was T/C heterozygous.

Hence, the present invention relates to a method for examining lung cancer of an individual by using a base substitution of a DDR2 gene (T681I in the case of an amino acid sequence) as an indicator. Specifically, the present invention provides a method for examining lung cancer by analyzing a gene mutation of the 148399th-148401st bases, preferably the 148400th base, in a nucleotide sequence of a DDR2 gene collected from a test subject, or a mutation of the 2041st-2043rd bases, preferably the 2042nd base, in the nucleotide sequence represented by SEQ ID NO:2 to examine the lung cancer of the test subject based on said analysis result.

In a preferable aspect of the present invention, a mutation of the second nucleotide of the codon coding for the 681 st amino acid residue of the nucleotide sequence of the DDR2 gene, that is, a mutation of the 148400th base of a nucleotide sequence (for example, SEQ ID NO:1) of a gene coding for DDR2 (also referred to as a DDR2 gene), is a mutation where the base of the nucleotide is mutated from cytosine (C) to thymine (T) (mutation from ACT to ATT). In the nucleotide sequence represented by SEQ ID NO:2, the 2042nd base is mutated from C to T.

According to the examination method based on the above-described DDR2 gene, the test subject is judged to have lung cancer or have a risk of lung cancer when the gene mutation is T/C heterozygous.

Furthermore, the test subject can be judged to have squamous cell lung carcinoma or have a risk of squamous cell lung carcinoma when the gene mutation is T/C heterozygous, where this judgment result is useful for estimating the nature of the lung cancer.

The location of the mutation of a DDR2 gene in a patient with lung cancer is not limited to a specific site and the mutation has been reported in any domains, while it is frequently found in squamous cell lung carcinoma among lung cancers (Examples). Accordingly, the above-mentioned mutation can be used as basic information that is adaptable to diagnosis or treatment of lung cancer. According to the present invention, it was confirmed that this mutation can specifically be detected in DNA derived from a sample (tissue) of a test subject.

In the present invention, genes and proteins used for the detection are DDR2 genes and mutant genes thereof, and DDR2 proteins and mutant proteins thereof (including modified proteins and modified DNA, respectively). The DDR2 gene is located in the q23,3 region of human chromosome 1, and codes for DDR2, i.e., a protein belonging to receptor tyrosine kinases. A human DDR2 gene has a nucleotide sequence with a length of 155028 bp (SEQ ID NO:1), while a DDR2 protein consists of a sequence with 855 amino acids as represented by SEQ ID NO:3 (and coded by the nucleotide sequence represented by SEQ ID NO:2). The nucleotide sequence information of human DDR2 gene and the amino acid sequence information of DDR2 protein are available, for example, under Accession No. NG-016290.

### 3. Biomarker

A gene or a protein that serves as a biomarker in the present invention is a DDR2 gene or a mutant gene thereof, or a DDR2 protein or a mutant protein thereof. Information of a DDR2 gene mutation refers to the mutation of the 148400th base of the nucleotide sequence of the DDR2 gene, where, for example, ACT is mutated to ATT. Since ATT is a codon of isoleucine, this mutation will result substitution from threonine to isoleucine (T681I) at position 681 of the amino acid sequence of a DDR2 protein (the 681 st amino acid residue of the amino acid sequence represented by SEQ ID NO:3).

According to the present invention, a gene mutation principally includes a single-base mutation in the 148399th-148401st bases, preferably the 148400th base, of the nucleotide sequence of a DDR2 gene (SEQ ID NO:1), or the 2041st-2043rd bases, preferably the 2042nd base, of the nucleotide sequence represented by SEQ ID NO:2.

In a preferable aspect of the present invention, the above-described mutation of a DDR2 gene or a DDR2 protein can be utilized as a marker for assessing lung cancer of a test subject. In addition, overexpression of a DDR2 gene, a DDR2 protein or a mutant thereof can also be utilized as a marker for assessing lung cancer of a test subject.

According to the present invention, the primarily targeted lung cancer is squamous cell carcinoma or adenosquamous cell carcinoma.

By analyzing this mutation or overexpression, whether or not lung cancer, in particular, squamous cell carcinoma is present can be assessed.

The T681I mutation found by the present invention can be detected in a lung cancer tissue, where the correlation between the mutation and the phenotype is such that lung cancer or a risk of lung cancer is judged to be present when the gene mutation of the 148400th base in the analyzed DDR2 gene is T/C heterozygous. According to a statistical analysis carried out by the present inventors using 107 cases, the mutation was detected in 1.9% of the lung cancer cases.

Although the detection rate of the T681I mutation found by the present invention is not high, it can be expected to be a novel target molecule for a molecular target drug by estimating from the site of the mutation. A cell having this mutation can be used to establish a system for screening a novel molecular target drug.

### 4. Detection method

### (1) Detection of mutation in mutant DDR2 protein (including modified mutant protein, which similarly applies hereafter)

According to the present invention, a DDR2 protein derived from a sample of a test subject such as a surgically resected analyte, a bronchoscopic analyte or a bronchial lavage fluid analyte (including a cancer tissue, a normal lung tissue or the like) is subjected, for example, to direct sequencing of the amino acid sequence to analyze the 681st amino acid for detecting whether it is threonine or other amino acid (isoleucine). Moreover, a sample to be used is not limited to the above-mentioned samples, and sputum, blood, urine, saliva or the like can also be used.

In order to derive a DDR2 protein from the above-mentioned sample, for example, a procedure well known in the art such as solubilization of a protein with a surfactant, dialysis, gel filtration, ion-exchange chromatography, affinity chromatography or the like may be performed in a suitable combination.

### (2) Detection of mutation in mutant DDR2 gene (including modified mutant DNA, which similarly applies hereafter)

In addition, the present invention analyzes a mutation in a nucleotide sequence of DNA derived from the above-described sample collected from the test subject.

A genome sample from a test subject that is used for the above-described mutation analysis may be extracted from a surgically resected analyte, a bronchoscopic analyte, a bronchial lavage fluid analyte or the like (including a cancer tissue, a normal lung tissue or the like). Methods for extracting and purifying genomic DNA are well known. For example, a sample can be extracted from a surgically resected analyte, a bronchoscopic lung biopsy analyte or a bronchial lavage fluid analyte collected from human using a kit such as QIAamp DNA mini kit (QIAGEN). In the case of the present invention, since a mutation exists in a coding region (open reading frame) of a DDR2 protein, mRNA or total RNA may be extracted instead of genomic DNA. In order to extract mRNA or total RNA, a commercially available kit, for example, RNeasy Protect Mini Kit from QIAGEN, RNA purification kit from Roche (kit of HighPure series) or the like can be used, whose extraction procedure is well known.

Hereinafter, an exemplary method for detecting gene mutation in the above-described test sample will be described.

### (2-1) Detection using PCR method

In order to amplify a test sample by PCR, a high fidelity DNA polymerase, for example, but not limited to, Discoverase™ DHPLC DNA (Invitrogen) is preferably used. The primers used are designed and synthesized such that a gene mutation is contained at a predetermined position of the primers so that the targeted mutation site in the test sample is amplified.

At the end of the amplification reaction, an amplification product is detected to judge of the presence or absence of the mutation. For example, a TaqMan PCR method is a method that utilizes a fluorescently labeled allele-specific oligonucleotide and PCR reaction using a Taq DNA polymerase. An allele-specific oligonucleotide (also referred to as a TaqMan probe) used in the TaqMan PCR method can be designed based on the above-described gene mutation information. Alternatively, upon amplification using allele-specific primers, a SYBR Green PCR method can be used to incorporate a fluorescently labeled base into the amplified product so as to quantify the mutated gene.

### (2-2) Detection by nucleotide sequencing method

According to the present invention, a mutation can also be detected by direct sequencing. As a sequencer used for nucleotide sequencing, a commercially available ABI series (Life Technologies) or the like is used.

### (2-3) Detection with DNA microarray

A DNA microarray has nucleotide probes fixed on a support, and includes a DNA chip, a microchip, a bead array and the like. First, a polynucleotide of a test sample is isolated, amplified by PCR, and labeled with a fluorescent reporter group. Subsequently, labeled DNA/mRNA and total RNA are incubated with the array. Then, this array is inserted into a scanner to detect the hybridization pattern. The hybridization data is collected as luminescence from the fluorescent reporter group bound to the probe array (i.e., incorporated into the target sequence). A probe that completely matched the target sequence generates a signal that is stronger than one having a part that does not match the target sequence. Since the sequences and the positions of the respective probes on the array are known, the sequence of the target polynucleotide reacted with the probe array can be determined according to complementarity.

### (2-4) Others

Other than the above-described method, a gene mutation can be detected by a MBP-QP method. The MBP-QP method is a method in which a gene is amplified by mutation biased PCR and then a gene mutation is detected by a quenched probe method. The MBP-QP method was established by the present inventors for the purpose of detecting other gene mutations. This method allows a gene mutation be detected easily.

### (3) Detection of overexpressed DDR2 protein or DDR2 gene, or mutant thereof

An overexpressed protein (including a modified wild-type protein, which similarly applies hereafter) may be detected, for example, by ELISA, western blotting or the like.

Moreover, an overexpressed gene (including a modified wild-type DNA, which similarly applies hereafter) can be detected, for example, by quantitative PCR, Digital PCR or the like.

The above-described procedures can also be applied to mutant proteins and mutant genes (including modified mutant proteins and modified mutant DNAs, which similarly applies hereafter).

### (4) Judgment of examination results

When an expression level of a DDR2 protein, a DDR2 gene or a mutant thereof (collectively referred to as "DDR2s") in a test sample collected from a test subject is overexpressed as compared to a control, the value of this expression level can be used as an indicator that represents that the test subject has lung cancer or has a risk of lung cancer. When the value of this expression level is higher than that of the control, the test subject has lung cancer or a risk of lung cancer.

Herein, the term "control" refers to an expression value or level of DDR2s derived from a healthy individual, or a standardized normal value or level based on the data of DDR2s derived from a healthy individual.

Moreover, when an amino acid sequence of a DDR2 protein or a nucleotide sequence of a DDR2 gene of a test sample collected from a test subject has the above-described mutation, the test subject is judged to have lung cancer or a risk of lung cancer.

### (5) Handling of examination results

Now, according to the method of the present invention, samples derived from multiple test subjects are used to analyze the presence or absence of a mutation. Therefore, in order to detect lung cancer of an individual test subject, the gene mutation in genomic DNA is statistically analyzed, for example, by an allele-specific PCR method, from the results of which, the position where the individual is located or belongs to is examined to find out the possibility of lung cancer of each individual test subject. Moreover, an analysis between T681I mutation and a phenotype (lung cancer or a type thereof) can be carried out for a predetermined number of test subjects (primary population), where the resulting determined values are used as basic data that can be compared with the mutations derived from single or multiple test subjects targeted for the detection so as to correlate with the phenotype, i.e., lung cancer. The analysis results of overexpression levels of wild-type genes and proteins can also be handled in a similar manner to the above-described analysis of the presence or absence of mutations.

The above-determined data derived from the test subjects can be incorporated into the values of the above-mentioned population to reprocess the data between the mutation or the expression level and the risk level of the lung cancer. By doing so, the number of the cases of the targeted test subjects (population) can be increased to improve the accuracy of the analysis.

### 5. Probe or PCR primer for detecting lung cancer

Hereinafter, a probe or a PCR primer for detecting lung cancer will be described. A method for acquiring a genomic DNA sample from a targeted test subject is as described above.

According to the present invention, an oligonucleotide used as a primer and/or a probe is an oligonucleotide (hereinafter, referred to as an "oligonucleotide of the present invention") that consists of a nucleotide sequence having at least 10 bases designed to contain an oligonucleotide containing the 148400th base of the nucleotide sequence of a DDR2 gene (for example, SEQ ID NO:1), or a nucleotide sequence complementary thereto.

A nucleotide sequence (partial) near the mutation site is shown in Table 1.

**[Table 1]**

| Nucleotide sequence near mutation site | |
|---|---|
| 5’........tgtacgca c/t tgtcag.......3’ ↑ 148400 | SEQ ID NO:6 |

In Table 1, "c/t" means that "C" was mutated to "T". The present invention provides an oligonucleotide having 10-50 bases that contains the mutation site (c/t) of the above-described nucleotide sequence of the DDR2 gene (SEQ ID NO:1). These oligonucleotides may be used as a probe or a PCR primer for detecting lung cancer. The lung cancer can be detected using the above-described mutation as an indicator.

The length of the oligonucleotide of the present invention is not particularly limited as long as it has at least 10 consecutive bases, preferably a length of 10-2565 bases, more preferably a length of 10-100 bases, still more preferably a length of 10-50 bases, and yet still more preferably a length of 10-30 bases. Preferably, an oligonucleotide having a length of 10-2565 bases is designed and synthesized from the region (SEQ ID NO:2 or 4) coding for DDR2 in the nucleotide sequence represented by SEQ ID NO:1.

The oligonucleotide of the present invention can be obtained by a general chemical synthesis based on the nucleotide sequence represented by SEQ ID NO:1 or 2. According to the present invention, a complementary strand (complementary sequence) thereof are also contained. In addition, the oligonucleotides of the present invention can be designed and synthesized based on the information of the above-described nucleotide sequence represented by SEQ ID NO:4. In this case, the mutation site may be designed to lie on the 5' end or the 3' end of the nucleotide sequence, but it is not limited thereto and may be designed to lie inside the 5' or 3' end.

In addition, the probe of the present invention may contain a labeling moiety bound to the oligonucleotide of the present invention. While the label may be a fluorescent label, a radioisotope label, a digoxigenin (DIG) label or the like, but it is preferably a fluorescent label.

When the prepared oligonucleotide is used as a probe, whether or not this probe has hybridized with the test DNA can be utilized to determine or detect the mutation.

The above-described fluorescent dye is not particularly limited. Examples include fluorescein, phosphor, rhodamine and a polymethine dye derivative. Examples of commercially available products of these fluorescent dyes include Pacific Blue, BODIPY FL (brand name, Molecular Probes), FluorePrime (trade name, Amersham Pharmacia), Fluoredite (trade name, Millipore), FAM (ABI), Cy3 and Cy5 (Amersham Pharmacia) and TAMRA (Molecular Probes). Alternatively, the probe of the present invention can be used as a TaqMan (registered trademark) probe.

Conditions for detecting a fluorescently labeled oligonucleotide are not particularly limited, and may suitably be determined according to a fluorescent dye used. For example, Pacific Blue can be detected at a detection wavelength of 445-480 nm, TAMRA can be detected at a detection wavelength of 585-700 nm, and BODIPY FL can be detected at a detection wavelength of 520-555 nm. By using a prove having such a fluorescent dye, hybridization and dissociation can easily be confirmed according to the variations of the respective fluorescence signals.

Preferably, a probe of the present invention is designed to contain the 148400th base of the nucleotide sequence represented by SEQ ID NO:1 (in the case of SEQ ID NO:2 or 4, the base that corresponds to this 148000th base), and to contain the bases both upstream and downstream from the 148400th base. Provided that the 148400th base with an allele of "C" (normal sequence) is referred to as "allele C" and the 148400th base with an allele of "T" (a sequence having a mutation at the 148400th base) is referred to as "allele T", hybridization is performed under conditions such that the above-described probe hybridizes with the sequence of allele C while it does not hybridize with the sequence of allele T. Since the probe hybridizes with DNA having the sequence of allele T, whether the allele is C or T can be judged (detected) according to the presence or absence of a band.

In order to design an oligonucleotide as a primer for detecting allele T, they are designed from the upstream side of the 148400th base as well as from the downstream side of the 148400th base. Accordingly, fragments amplified by PCR will have different sizes depending on the presence or absence of the mutation and thus the mutation can be detected according to the difference in the sizes of the fragments. The primer is designed to have a length of at least 15 bases, preferably 15-30 bases, and more preferably 18-24 bases. Of course, the design of the primer is not limited to a region within 50 bases upstream and downstream from the 148400th base. It may suitably be selected from the region of the template DNA such that the amplified fragment is 1000 bp or less, preferably 500 bp or less, and more preferably 200 bp or less (for example, 50-100 bp) based on the sequence of genomic DNA.

While the oligonucleotide primer and the oligonucleotide probe designed as described above can chemically be synthesized by a known means and method, in general, they are synthesized using a commercially available chemical synthesizer. Additionally, an appropriate fluorescent label (for example, FAM, VIC, etc.) may be added to the probe in advance so as to allow automation of the operation.

### 6. Kit

The present invention provides a kit for detecting lung cancer. Specifically, the kit of the present invention includes at least one component selected from the group consisting of the nucleotide of the present invention and the probe of the present invention. For example, in addition to the above-mentioned component, the kit of the present invention may include an enzyme buffer, dNTP, a reagent as a control (for example, a tissue sample, a target oligonucleotide as a positive and negative control, or the like), a labeling and/or detecting reagent, a solid-phase support, an instruction and the like. In addition, the kit of the present invention may be a partial kit that only includes some of the required components, in which case the user can prepare the rest of the components.

The kit of the present invention can be provided as a microarray having the above-described oligonucleotide fixed on a support. The microarray has an oligonucleotide of the present invention fixed on a support, and includes a DNA chip, a microchip, a bead array and the like.

Preferably, the kit of the present invention includes an oligonucleotide that specifically hybridizes with a DNA fragment containing a mutation of a DDR2 gene.

In a case where a gene mutation or overexpression is judged with the kit of the present invention, for example, DNA containing the above-described DDR2 gene is isolated from a surgically resected analyte, a bronchoscopic lung biopsy analyte or a bronchial lavage fluid analyte collected from a human upon diagnosis or treatment of the disease, a fixed sample prepared therefrom, or the like, and then this isolated DNA is reacted with the oligonucleotide of the kit to judge a genotype or an overexpression level.

Based on the judged genotype, gene mutation or overexpression level, whether or not lung cancer (in particular, squamous cell carcinoma) or a risk of lung cancer is present is judged.

### 7. Substance that inhibits expression of DDR2

The phrase "to inhibit expression" means to suppress gene expression of DDR2, or to suppress a function of DDR2 (proliferation, migration, invasion or metastasis of a tumor cell).

Therefore, there is no limitation on the "substance" as long as it has an activity of inhibiting the expression of the DDR2 protein or the DDR2 gene. Examples of the substance that inhibits the expression of a DDR2 protein include an antibody for DDR2, and inhibitory nucleic acids for a gene coding for DDR2 such as antisense nucleic acids, decoy nucleic acids, microRNA, shRNA or siRNA.

### (1) Antibody for DDR2 or mutant thereof

An antibody contained in a pharmaceutical composition of the present invention may be either a polyclonal antibody or a monoclonal antibody, whose preparation process is well known.

### (1-1) Preparation of polyclonal antibody

In order to prepare the above-described antibody, first, a purified DDR2 or a purified DDR2 mutant is used as an immunogen (antigen).

Next, the obtained purified protein is dissolved in a buffer to prepare an antigen solution, to which a known adjuvant is added if necessary. Immunization is carried out by administering a solution containing the above-described purified DDR2 or a mutant thereof into a mammal animal (for example, a mouse, a rat, a rabbit, etc.).

An antiserum of interest is screened from the antisera collected from the immunized animals. The screening method can be carried out by a known immunoassay following a routine procedure. If purification of the antibody is necessary, a known method such as ammonium sulfate precipitation, ion-exchange chromatography or affinity chromatography may be employed.

### (1-2) Preparation of monoclonal antibody

A method for preparing an antigen and a solution thereof, an immunization method, as well as the dosage, the dosing interval and the number of doses of the above-described antigen are well known in the art.

For example, an antibody-producing cell is preferably a spleen cell, a lymph node cell, a peripheral blood cell or the like, and more preferably a spleen cell.

Through cell fusion between the collected antibody-producing cell and a myeloma cell, a fusion cell (hybridoma) can be obtained.

The myeloma cell, the cell fusion technique, the mixture ratio of the antibody-producing cell and the myeloma cell, culture with a HAT selection medium, screening of the hybridoma of interest, a technique for cloning the hybridoma and else are well known in the art.

According to the present invention, a humanized antibody, a human antibody, or a fragment thereof can also be used.

A humanized antibody (CDR-grafted antibody) is a genetically-engineered monoclonal antibody, specifically an antibody whose complementarity determining region of the hypervariable region is partially or entirely a complementarity determining region of a mouse monoclonal antibody-derived hypervariable region, whose framework region of the variable region is a framework region of a human immunoglobulin-derived variable region, and whose constant region is a human immunoglobulin-derived constant region. A humanized antibody can be prepared by a method well known in the art.

A human antibody refers to an antibody whose entire regions, including the variable region of the H chain and the constant region of the H chain as well as the variable region of the L chain and the constant region of the L chain that make up an immunoglobulin, originate from a gene coding for a human immunoglobulin. A human antibody can be produced in the same manner as the above-described method for producing a polyclonal antibody or a monoclonal antibody by producing a transgenic animal that generates a human antibody, and immunizing this transgenic animal with an antigen by a method well known in the art.

An antibody fragment refers to a part containing an antigen-binding region of the above-described antibody or a part derived from this region, specific examples being F(ab')₂, Fab', Fab, Fv (variable fragment of antibody), scFv (single chain Fv) and dsFv (disulphide stabilized Fv).

### (2) Inhibitory nucleic acid for gene

An inhibitory nucleic acid for a DDR2 gene or a mutant thereof refers to a nucleic acid that suppresses the gene function or the gene expression thereof, examples being an antisense nucleic acid, siRNA, shRNA, a decoy nucleic acid and microRNA. With such an inhibitory nucleic acid, expression of the above-described gene can be suppressed.

### (2-1) Antisense nucleic acids

Antisense nucleic acids are a single-stranded nucleic acid sequence (RNA or DNA) that can bind to a mRNA (sense) or DNA (antisense) sequence of a DDR2 gene. The length of the antisense nucleic acid sequence is at least about 17 nucleotides, and preferably 15-30 nucleotides. The antisense nucleic acids bind to the above-described gene sequence to form a double strand and suppress transcription or translation.

The antisense nucleic acids can be produced by employing a chemical synthesis method or a biochemical synthesis method known in the art.

The antisense nucleic acids can be introduced into a cell, for example, by a gene transfer method such as DNA transfection or electroporation, or by using a virus vector.

### (2-2) Decoy nucleic acids

According to the present invention, decoy nucleic acids refer to "decoy" nucleic acids that bind to a transcription factor of a DDR2 gene and suppress the promoter activity. By introducing these nucleic acids into a cell, the transcription factor binds to these nucleic acids, by which the primary binding between the transcription factor and the genomic binding site is competitively inhibited, thereby suppressing the expression of that transcription factor. Specifically, decoy nucleic acids are nucleic acids that can bind to a target-binding sequence, or an analog thereof.

An example of preferable decoy nucleic acids of the present invention includes nucleic acids (either DNA or RNA) that can bind to a transcription factor that binds to a promoter of a DDR2 gene. The decoy nucleic acids can be designed as a single strand or a double strand including a complementary strand thereof, based on the promoter sequence of the above-described gene. The length is not particularly limited.

The decoy nucleic acids used in the present invention can be produced by employing a chemical synthesis method or a biochemical synthesis method known in the art. Alternatively, a nucleotide sequence as a template can be isolated or synthesized and then subjected to a PCR method or a gene amplification method using a cloning vector. Furthermore, nucleic acids obtained by these methods can be cleaved with, for example, a restriction enzyme or the like, and linked using a DNA ligase to produce nucleic acids of interest. Moreover, in order to obtain decoy nucleic acids that are more stable in the cell, bases can chemically be modified, for example, by alkylation or acylation. A mutant of decoy nucleic acids can also be synthesized by a method known in the art, for example, by site-directed mutagenesis or the like. Site-directed mutagenesis is well known in the art, where a commercially available kit such as GeneTailor™ Site-Directed Mutagenesis System (Invitrogen), TaKaRa Site-Directed Mutagenesis System (Takara Bio) or the like can be used.

The transcription activity of the promoter upon use of the decoy nucleic acids can be analyzed by employing commonly performed luciferase assay, gel shift assay, western blotting, FACS assay, RT-PCR or the like. Kits for performing these assays are also commercially available (for example, promega dual luciferase assay kit).

### (2-3) RNA interference

According to the present invention, a synthesized small nucleic acid molecule that can regulate gene expression through RNA interference (RNAi) in the cell, for example, a siRNA, microRNA (miRNA) or shRNA molecule, can be used.

When a siRNA (small interfering RNA) molecule is used, various RNA corresponding to the target gene may be targeted. Examples of such RNA include mRNA, variants obtained by alternative splicing of the target gene, and post-transcriptionally modified RNA of the target gene. In a case where a family of transcription products that can be distinguished by alternative splicing using suitable exons arises, a siRNA molecule can be used to inhibit the expression of the exon parts or the conserved sequences.

A siRNA molecule can be designed based on a standard well known in the art. For example, as a target segment of target mRNA, a segment with 15-30 consecutive bases, preferably 19-25 bases that preferably starts from AA (most preferable), TA, GA or CA can be selected. The GC-content of the siRNA molecule is 30-70%, and preferably 35-55%.

A siRNA can be generated as a single-stranded hairpin RNA molecule that folds back on its own nucleic acids to generate a double-stranded part. A siRNA molecule can be obtained by a general chemical synthesis. Alternatively, a siRNA molecule can biologically be generated using an expression vector containing sense and antisense siRNA sequences.

Moreover, the present invention is capable of suppressing expression of the above-describe gene by using a microRNA. A microRNA (miRNA) is a single-stranded RNA with a length of about 20-25 bases that is present in a cell, and is one type of ncRNA (non coding RNA) that is considered to have a function of regulating expressions of other genes. A miRNA results by undergoing processing upon transcription into RNA, and exists as nucleic acids that form a hairpin structure that suppresses the expression of the target sequence.

Since miRNA is also an inhibitory nucleic acid based on RNAi, it can be designed and synthesized like shRNA or siRNA.

### 8. Therapeutic method and pharmaceutical composition

The present invention provides a method for treating lung cancer, the method comprising a step of administering an anticancer drug to a test subject who has been judged to have lung cancer by the above-described method.

The present invention also provides a method for treating lung cancer, the method comprising a step of administering a substance that suppresses expression of a DDR2 protein, a DDR2 gene or a mutant thereof to a patient with lung cancer.

The present invention further provides a pharmaceutical composition for treating lung cancer, the composition comprising a substance that suppresses expression of a DDR2 protein, a DDR2 gene or a mutant thereof.

An anticancer drug or a pharmaceutical composition of the present invention may be administered into a body through a known usage, for example, but not limited to, a parenteral or oral usage, preferably by parenteral administration.

A formulation used in these various usages (a parenteral agent, an oral agent or the like) can be prepared according to a routine method by appropriately selecting and using an excipient, a filler, an extender, a binder, a wetting agent, a disintegrant, a lubricant, a surfactant, a dispersant, a buffer, a preservative, a solubilizing agent, an antiseptic, a flavoring agent, a soothing agent, a stabilizer, a tonicity agent or the like that are generally employed in pharmaceutical manufacturing.

In general, a dosage of an anticancer drug or a pharmaceutical composition of the present invention, can suitably be determined considering the mixing ratio of an active element (an antibody for DDR2 or a mutant thereof, or an inhibitory nucleic acid for DDR2 or a mutant thereof) in the formulation, as well as age and weight of the administration target (patient), stage/progress of the disease, administration route, number of doses, administration period and the like.

When an anticancer drug or a pharmaceutical composition of the present invention is used as a parenteral agent, the form thereof is generally not limited, and may be, for example, any of an intravenously injectable agent (including drip infusion), an intramuscularly injectable agent, an intraperitoneally injectable agent, a subcutaneously injectable agent, a suppository or the like.

The various injectable agents may be provided, for example, in a form of a unit dosage ampule or a multiple-dose container, or in a form of freeze-dried powder that can be redissolved in a dissolving liquid upon use. Besides the above-described active element, the parenteral agent may also contain various known diluents and additives according to its form within a range that does not impair the effect of the above-described active element. For example, the various injectable agents may contain water, glycerol, aliphatic polyalcohols such as propylene glycol or polyethylene glycol, or the like.

When used as an oral agent, the form thereof is generally not limited, and may be, for example, any of a tablet, a capsule, granule, powdered drug, a pill, a lozenge, liquid medication, a suspension agent, an emulsion formulation, syrup or the like, or a dried product that can be redissolved upon use. Besides the above-described active element, the oral agent may also contain various known diluents and additives according to its form within a range that does not impair the effect of the above-described active element. Examples include a binder (syrup, gum arabic, gelatin, sorbitol, tragacanth, polyvinylpyrrolidone, etc.), a filler (lactose, sugar, cornstarch, potato starch, calcium phosphate, sorbitol, glycine, etc.), a lubricant (magnesium stearate, talc, polyethylene glycol, silica, etc.), a disintegrant (various starches, etc.) and a wetting agent (sodium lauryl sulfate, etc.).

An oral agent can be given continuously or once to several times a day. Preferably, the administration route is parenteral (for example, intravenous injection).

When siRNA, shRNA or miRNA is given, the effective amount thereof is not particularly limited as long as the amount is sufficient to cause RNAi-mediated decomposition of the target mRNA. Those skilled in the art can readily determine the effective amount to be given to a test subject by considering factors such as the height, weight, age and sex of the test subject, the administration route, or whether it is local or systemic administration.

Hereinafter, the present invention will be described more specifically by way of examples. The present invention, however, should not be limited to these examples.

### Example 1

### Method

26 lung cancer cell lines including 9 squamous cell lung carcinoma cell lines and 80 cases of patient analytes were used for an analysis. The patient analytes were the 80 cases of surgically resected analytes and bronchoscopic analytes of primary lung cancer cases (other than adenocarcinoma) under treatments at Saga University Hospital from May, 2004 to February, 2012, among which 54 cases were diagnosed as squamous cell carcinoma. Informed consent was received from all patients. All of the analytes were subjected to all exon sequencing of DDR2 using DNA direct sequencing.

DNAs were extracted from the surgically resected analytes, the bronchopulmonary biopsy analytes and the bronchial lavage fluids with QIAamp (registered trademark) DNA mini kit (QIAGEN, Hilden, Germany). Detections of mutations were analyzed for all exons 1-19 by direct sequencing. PCR amplification was conducted using Discoverase™ DHPLC DNA polymerase (Invitrogen Inc., CA). Thereafter, Amicon Ultra-0.5 (Millipore Inc. MA) was used for purification and direct sequencing was performed by cycle sequencing with Applied Biosystems 3130 Genetic Analyzer (Applied Biosystems, Foster City, CA).

### Results

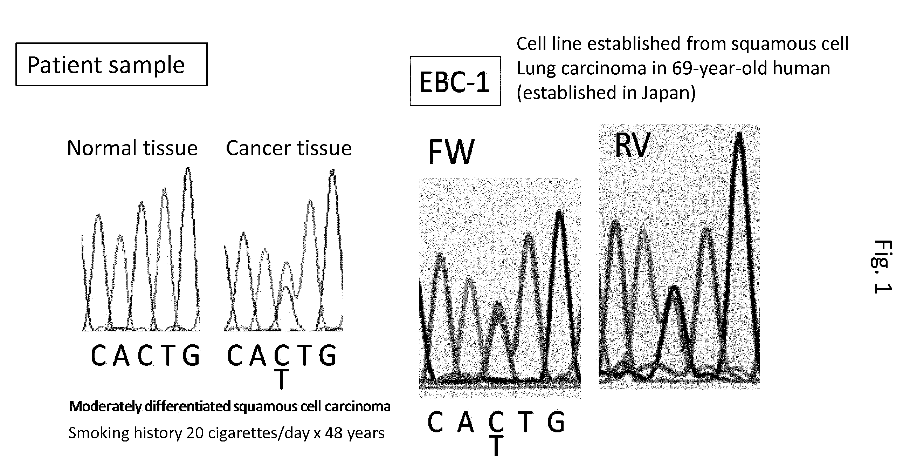

As a result of analyzing the 80 cases of patient analytes, three types of gene polymorphisms without an amino acid mutation (H136H; 11%, L420L; 17.5%, H520H; 7.5%) and one type of gene mutation associated with an amino acid mutation (T681I; 1.3%) were found (Figure 1). Among the lung cancer cell lines, T681I mutation was also found in the squamous cell lung carcinoma cell line EBC-1, where the mutation frequency was 2.2% in whole and 3.2% among the squamous cell carcinomas (Figure 1).

From the above-described results, a method for detecting DDR2 gene mutation (T681I) was established for diagnosing lung cancer.

### Example 2

### Invasion assay using cell lines

BD BioCoat Matrigel invasion chamber 354480 was used to perform Invasion assay with the cell lines.

Cells used:
H226B - WT (D#68)
H226B - mutant (M#73)
H226B - empty (E#4)
Number of cells: 5 x 10⁵/well
Invasion time: 22 hours

After an invasion chamber was hydrated for 2 hours, cells were added to an insert, followed by 22 hours of incubation.

Cells on the top surface were removed and the invaded cells were fixed and stained with Diff-Quik and encapsulated.

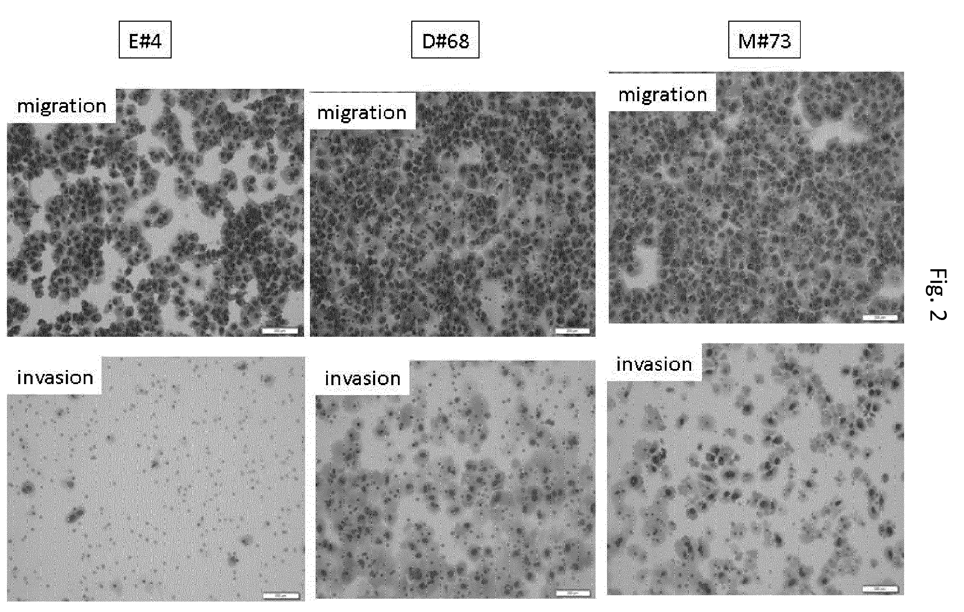

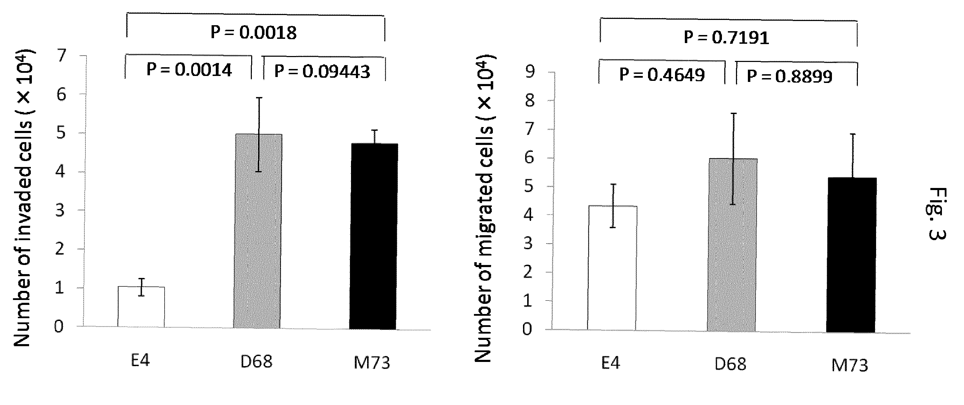

### The results are shown in Figures 2 and 3.

As can be appreciated from Figures 2 and 3, when DDR2 wild-type, T681I mutation was forcibly expressed in lung cancer cell lines H226B, invasive capacity of the cancer cells was found to significantly increase.

### Example 3

### Analysis of mRNA expression level of TGFb1

### (1) Analysis of expression by real-time PCR

### Method:

A primer probe of TaqMan Gene Expression Assays (Assay ID Hs 00998133_ml) from Applied biosystems was used to perform PCR under the following conditions.
Cycle conditions; Initial denaturation: 95°C, 60 seconds → PCR: 40 cycles of 95°C for 15 seconds and 60°C for 30 seconds
PCR reagent; THUNDERBIRD Probe qPCR Mix (Code No. QPS-101) from TOYOBO
GAPDH and 18S rRNA were used as internal controls.

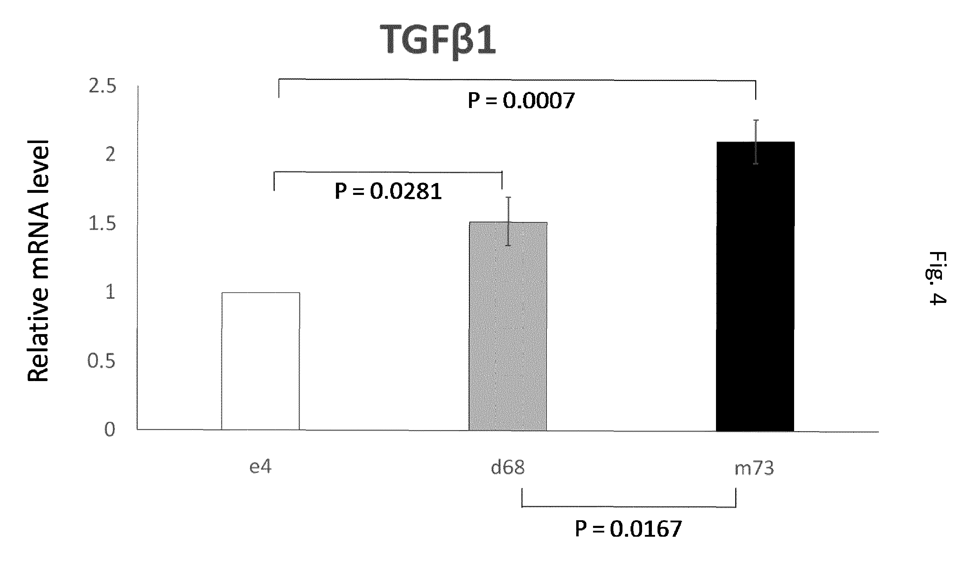

### The results are shown in Figure 4.

With respect to the mRNA expression levels of TGFβ1 obtained by real-time PCR, the expression level of TGFβ1 increased in the cell line highly expressing DDR2 (Wild type) than in empty cell line. In addition, the expression level was significantly increased further in the clone highly expressing mutant (T681I) than in the Wild type. These results show that the overexpressed version of DDR2 or a mutant thereof promotes invasion/metastatic capacity of the cancer.

### (2) Expression analysis by ELISA

### Method:

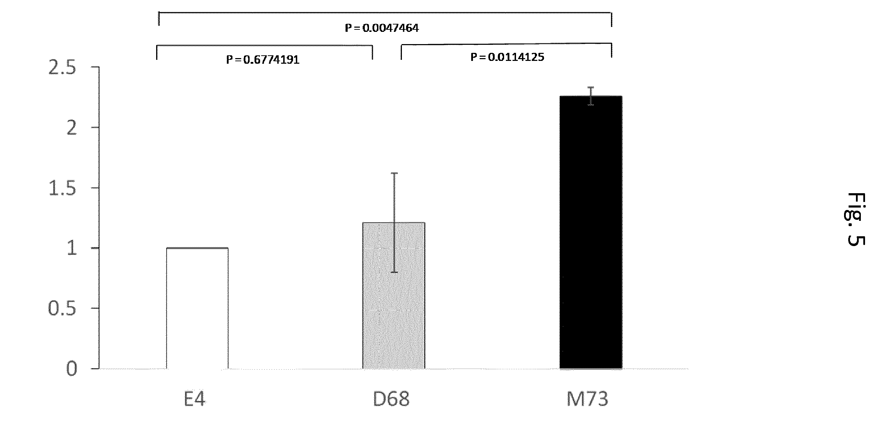

Supernatants of H226B-WT (D#68), H226B-mutant (M#73) and H226B-empty (E#4) were determined with R and D systems Quantikine ELISA Human TGF-β1 Immunoassay (Catalog Number DB100B).
The results are shown in Figure 5.

Referring to Figure 5, although there was no significant difference in the protein expression level of TGFβ1 between the cell line highly expressing DDR2 (Wild type) and the empty cell line, the expression level was significantly increased further in the clone highly expressing mutant (T681I) than in the Wild type. Hence, the mutant (T681I) was shown to promote invasion/metastatic capacity of the cancer.

### Example 4

### Transplantation experiment with metastatic mouse models

### Method:

### Mice:

8-week-old male NOJ/SCID mice

### Cells:

H226B-empty E#4
H226B-wild D#68
H226B-T681I M#73

Each of the above-mentioned cells were suspended in a phosphate buffer saline, and the cells were subcutaneously transplanted into abdomens of 5 mice per group at a dose of 5 x 10⁵/total/100 µl. After the transplantation, the weights and the tumor masses were determined once a week. The tumor mass was calculated according to the following formula.

### Tumor mass = (minor diameter)² x (major diameter)/2

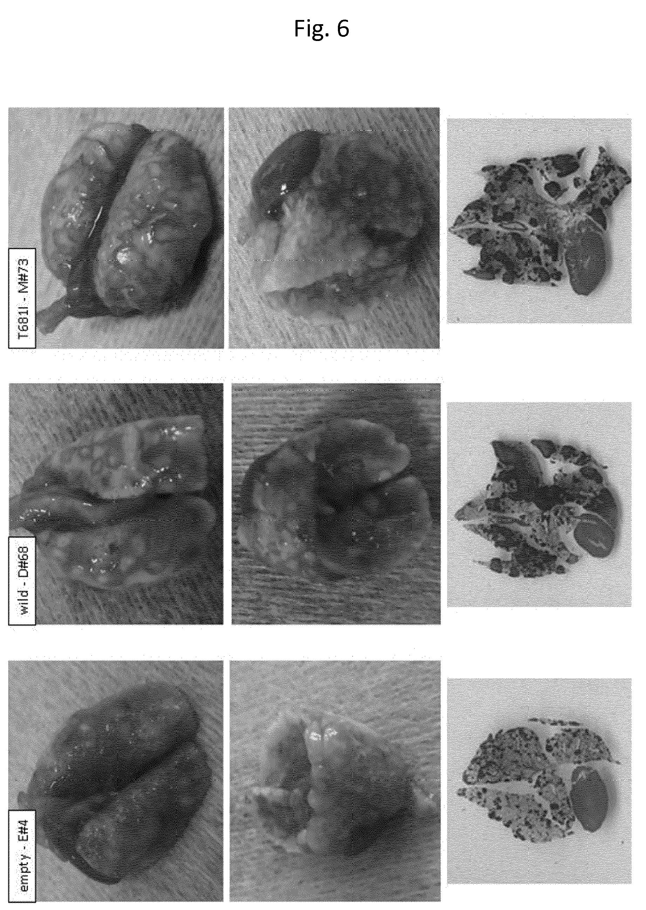

Figure 6 gives images showing macroscopic findings of lung metastasis and results from HE staining. As can be appreciated from Figure 6, the lung metastasis was large in total in the cell line highly expressing DDR2 (Wild type) and the cell line highly expressing mutant (T681I).

Hence, the cell line highly expressing DDR2 and the mutant of DDR2 (T681I) were shown to promote invasion/metastatic capacity of the cancer.

### SEQUENCE LISTING: Free Text

SEQ ID NO:4: n represents a, g, c or t (positions of presence: 2041..2043).
SEQ ID NO:5: Xaa represents Lys, Asn, Arg, Ser, Ile, Met, Glu, Asp, Gly, Ala, Val, Gln, His, Pro, Leu, Tyr, Trp, Cys or Phe (position of presence: 681).

### INDUSTRIAL APPLICABILITY

The method and the kit of the present invention are useful for examining lung cancer.

## Claims

1. A biomarker for lung cancer, comprising a DDR2 protein, a DDR2 gene or a mutant thereof of (a), (b) or (c) below:
(a) an overexpressed DDR2 protein or DDR2 gene;
(b) a mutant of an overexpressed DDR2 protein or DDR2 gene; or
(c) a mutant of a DDR2 protein or a DDR2 gene.

2. The biomarker according to Claim 1, wherein the DDR2 protein is a protein of (a) or (b) below:
(a) a protein containing the amino acid sequence represented by SEQ ID NO:3; or
(b) a protein that contains an amino acid sequence having one or several amino acids, other than the 681st amino acid, deleted, substituted or added in the amino acid sequence represented by SEQ ID NO:3, and that has DDR2 activity.

3. The marker according to Claim 1, wherein the DDR2 gene comprises DNA of (a) or (b) below:
(a) DNA containing the nucleotide sequence represented by SEQ ID NO:2; or
(b) DNA that hybridizes with a nucleotide sequence complementary to the nucleotide sequence represented by SEQ ID NO:2 under stringent conditions, and that codes for a protein having DDR2 activity.

4. The biomarker according to Claim 1, wherein the mutant of the DDR2 protein is a protein of (a) or (b) below:
(a) a protein containing the amino acid sequence represented by SEQ ID NO:5; or
(b) a protein that contains an amino acid sequence having one or several amino acids, other than the 681st amino acid, deleted, substituted or added in the amino acid sequence represented by SEQ ID NO:5, and that has DDR2 activity.

5. The biomarker according to Claim 4, wherein the mutation of the 681 st amino acid residue is a mutation from threonine to isoleucine (T681I).

6. The biomarker according to Claim 1, wherein the mutant of the DDR2 gene is one shown in (a), (b) or (c) below:
(a) DNA coding for the amino acid sequence represented by SEQ ID NO:5;
(b) DNA coding for a protein that contains an amino acid sequence having one or several amino acids, other than the 681st amino acid, deleted, substituted or added in the amino acid sequence represented by SEQ ID NO:5, and that has DDR2 activity; or
(c) DNA having a mutation of the second nucleotide of the codon coding for the 681st amino acid residue of the nucleotide sequence of DDR2 gene.

7. The biomarker according to Claim 6, wherein the mutation of the second nucleotide is a mutation at the 148400th base of the nucleotide sequence of the DDR2 gene.

8. The biomarker according to Claim 6, wherein the mutation of the second nucleotide is a mutation from nucleotide C to nucleotide T.

9. A method for examining lung cancer, comprising a step of determining an expression level of a DDR2 protein, a DDR2 gene or a mutant thereof in a test sample collected from a test subject, wherein, when the expression level indicates overexpression as compared to a control, the determination result is used as an indicator of lung cancer or a risk of lung cancer.

10. A method for examining lung cancer, comprising a step of analyzing the presence or absence of a mutation in a DDR2 protein or a DDR2 gene in a test sample collected from a test subject, wherein, when a mutation is present in a predetermined amino acid sequence of the DDR2 protein or in a predetermined nucleotide sequence of the DDR2 gene, the analysis result is used as an indicator of lung cancer or a risk of lung cancer.

11. The method according to Claim 9 or 10, wherein the test sample is at least one selected from the group consisting of a surgically resected analyte, a bronchoscopic analyte and a bronchial lavage fluid analyte.

12. A method for examining cancer, wherein a test subject is judged to have lung cancer or a risk of lung cancer when the expression level of a DDR2 protein, a DDR2 gene or a mutant thereof indicates overexpression by the method according to Claim 9 or 11.

13. A method for examining cancer, wherein the test subject is judged to have lung cancer or a risk of lung cancer when a mutation is present in an amino acid sequence of a DDR2 protein or in a nucleotide sequence of a DDR2 gene by the method according to Claim 10 or 11.

14. The method according to any one of Claims 9-13, wherein an examination of lung cancer is detection of lung cancer or estimation of a nature of lung cancer.

15. The method according to Claim 9 or 10, wherein the mutant of the DDR2 protein results from a mutation of the 681 st amino acid residue in the amino acid sequence of the DDR2.

16. The method according to Claim 15, wherein the mutation of the 681st amino acid residue is a mutation from threonine to isoleucine (T681I).

17. The method according to Claim 15, wherein the analysis of the presence or absence of the mutation of the 681st amino acid residue is an analysis of the presence or absence of a mutation of the second nucleotide of the codon coding for the 681 st amino acid residue in the nucleotide sequence of the DDR2 gene.

18. The method according to Claim 17, wherein the mutation of the second nucleotide is a mutation from nucleotide C to nucleotide T.

19. The method according to Claim 9 or 10, wherein the mutation of the DDR2 gene is a mutation of the 148400th base in the nucleotide sequence of the DDR2 gene.

20. The method according to Claim 19, wherein when the gene mutation is T/C heterozygous, it is judged that lung cancer or a risk of lung cancer is present.

21. The method according to any one of Claims 9-20, wherein the lung cancer is squamous cell lung carcinoma.

22. An oligonucleotide comprising a sequence of at least 10 bases containing the 148400th base of the nucleotide sequence of the DDR2 gene, or a sequence complementary thereto.

23. The oligonucleotide according to Claim 22, having a length of 10-2565 bases.

24. A probe having the oligonucleotide according to Claim 22 or 23 labeled.

25. The probe according to Claim 24, wherein the oligonucleotide has a length of 10-30 consecutive bases.

26. A kit for examining lung cancer, comprising at least one selected from the group consisting of the oligonucleotides according to Claims 22 and 23 and the probes according to Claims 24 and 25.

27. A method for treating lung cancer, comprising a step of administering an anticancer drug to a test subject who has been judged to have lung cancer by the method according to Claim 12 or 13.

28. A method for treating lung cancer, comprising a step of administering a substance that inhibits the expression of a DDR2 protein, a DDR2 gene or a mutant thereof to a patient with the lung cancer.

29. The method according to Claim 28, wherein the substance that inhibits the expression is a substance shown in (a) or (b) below:
(a) an antibody for a DDR2 protein or a mutant thereof; or
(b) an antisense nucleic acid, a decoy nucleic acid, microRNA, siRNA or shRNA for a DDR2 gene or a mutant thereof.

30. A pharmaceutical composition for treating lung cancer, comprising a substance that inhibits the expression of a DDR2 protein, a DDR2 gene or a mutant thereof.

31. The pharmaceutical composition according to Claim 30, wherein the substance that inhibits the expression is a substance shown in (a) or (b) below:
(a) an antibody for a DDR2 protein or a mutant thereof; or
(b) an antisense nucleic acid, a decoy nucleic acid, microRNA, siRNA or shRNA for a DDR2 gene or a mutant thereof.


SEQUENCE LISTING

<110> Saga University
ARKRAY, Inc.

<120> A method of examining a lung cancer

<130> G1280WO

<150> JP2013-267568
<151> 2013-12-25

<160> 6

<170> PatentIn version 3.5

<210> 1
<211> 155028
<212> DNA
<213> Homo sapiens

<400> 1
aaactaagaa ttaactattt cctaactgct ctgaaatttt tcctgtacac tacagaaaca 60

cacagcatat tattgtcaca cagacacaca ttttgttgtc acacatacac acacgtgttc 120

tagctctggc aggtatgatc ttgggcaaat taacttctct gtgcattggt tttctcatat 180

ggccattggg aaaataatag tacccatccc ataaaattct tgtgagtgtt aaatgaaata 240

ataaatgtgg aattcttata acagtgtctg gtatacagtg tcagaagttt tatatgtcaa 300

aagttctgct ttatatatcc tgaatctccc ctcctgctaa ttgtactcaa taaattatta 360

tttctggatc ttatttgatc ttcacaaccc tgtgaatttt cagttcatat tgtcccccat 420

tttacaatta gaaacgtgaa tgacttgttc aaggtgactc tgccccctag gggtttgtaa 480

ttggatcact ttccatgaaa gtgtccaaca ggagaacagc acctagtagt gtgcagggtt 540

aggacccaga agataaagtg aacactctct tagagttaaa attatctttg aaagaccttt 600

aaatcactta cagtatacat acttctgtgg atatgacttc ataggataat tcttaaggac 660

actaaaattc gagaatcgtg gggtaaattc aggaagacac taaaagcaag tctatatgca 720

gctgtctgaa cattcagact attttgcttt ttagaaaact aaggtgtttc tattgtctac 780

aagagttcct ccacactgat ttttaaagac tattaagcct ttatgcctta atatatgtca 840

atggatttgt ctattatgga tagaacaacc aggaacaggg agtcttaaga attaggttct 900

aaaattggag gccgggcgcg gtggctcatg cttgtaatcc tagcactgtg ggaggctgag 960

gcgggtggat cacttgaggt cagcggtttg agagcagcct ggccagcatg gtgaaacacc 1020

gtctctacta aaaatacaaa aatttgctgg gcgtggtggt gtgcacctgt aatcccagct 1080

actcaggagg ctgaggcagg agaatctctt gaacctggga ggcagaggtt gctgtgagcc 1140
aagaacgtgc cattgcactc cagcctgggc gacaagagcg aaactgtctc aaaaataaat 1200
aaataaataa ataaaaataa aagaaaaaag aaaaagaatt aggttctaaa attggtgctt 1260
aagttaaatg tgtgcagaat ataactccaa agtacacaat attctctgct tttgtgttag 1320
taaggcataa tttctttaaa atccagggat tgccatgctt gtctaattat tggtgtaaca 1380
gacaattttg gggtgtcttc taatgtatga catccctttt ctgagaattg attcatgtat 1440
gcgtatgagc tcttggagcc atattcatat tatatgatcc attgccctct acagtttaga 1500
gatagaggat gcacataaga aacacatttg accagtaaaa ttctccatcc cagcattcta 1560
gaactggact cccataatat ctgtcattct ctcatgatcg tttgaagcaa ggagagataa 1620
atttgagaac taaggcactg ttacaggcgc ctgctataac cagctataat attatgcgaa 1680
aataagcaga aaaggctggt ttactgaaag agaatgggct aaagcaaaat gagcagcggt 1740
aagcagagga gagagacaag aaatgaatga gagagagaag aaataagaga atgagaggag 1800
aagaaaagag gagaggaaag aagagaagag aggaggaaag gagaagagaa gacaggacag 1860
gacaggagag aagaaaaacc tgttgactaa gttactggtg gtggctaatt tgtggcttaa 1920
attccttgtg aagttaaatg taccacatgg gtttggtttg tgtgaatatc catgtatcct 1980
tttaaggtat gtgtgtgttt ctcaaactaa gttaagttaa ttcattttac tttcatcctt 2040
cattttatgt cctgaggcat cacaaaagca tccagctcat agctatttag agacagaaat 2100
attatagtta tttcaatgct caacttttgt atttacttga accttggttt gtagactaga 2160
aaaggtcttg aaaagtcatc ttgcacatct tacttctaat taacactgca tcttgaccat 2220
tccggataga tctgtcctat ttttgtaaaa tcccagtaat ggaattctat attgtttttc 2280
agtcatttgt tttcagtgta aaacagtcct cataacaaaa atacattact tcctatggaa 2340
cagaaataat tctgaatgca gtgactcaca tttcccaagc cagaccaacc agggggtaaa 2400
atctgttcac agaagagtaa taaaatggta tttctccaca tctgttcctg caatattagg 2460
aattccagtc ttactcaaaa ctctaccaaa tagctctgag agttcccagg acatttcttc 2520
tgtgtgttcc atgggttagt cccgtcagct ttgaagttag catttgccat tttcttactt 2580
tccagctctg gcttcctaca gcagtggctg gtgtcaaggc tgaacactgg ttgtcactca 2640
atcagagttg tctctgaaaa ctgaggcttc aatctaatga atcctcctca tgctttaaat 2700
atccctgaag tattttagta aaatatcact ttcagctttt ccttgctctt ttctttgtag 2760

aaccttcttc ctagagttca agattagacc ccagctttgg ctgagaatag cctgagtgat 2820
ggagatggtg ttttgagtga ttgatagaca tgctattttc ctttgagcta ctccttactg 2880
ttgacgtagc ttagtttttc ttgggacttt tatcccttgg tcctttctgt gttcagtgag 2940
ttcttcccca gaagtgtcct ggtctatata caaatagact cttgactgcc tagtccgcct 3000
cagacaaacc caggtgtgtc tgggttttgc agtctacatc actggctcag tcctggctca 3060
attaagacta caacttgagt tctaattata catacacaga gaaaaaacaa tctcacctat 3120
ggagtagaac caccttaaaa tgaaaaagga aaacacatac ctatatgtac aggccctgtc 3180
ctttatcagg ctcagtctgg ttttatgaag ttgttactgt tttccacatt tgacctatag 3240
tatcaccaaa tctgtgaaat cacagattgt atttttggta cattctttat agtaatgcca 3300
aagaggtgaa ctactacagg catggacatt tgaggaggat ttctagttgt ctctggcaag 3360
cacccagtaa agtgcctgat aagagcccaa cacatattta ttgagttgag ctgcatgtct 3420
ggcctgagtc accctcaggc ctgacttcag atgctgtaag ttatttgaag agtcttctta 3480
ataagttgat ctctgcagtg tcttttctct tcataaacta accctagttc tagttttttc 3540
tctaaggtct tgcttgctgc ctttaatcat ttccatggct gccctctgaa ccattcccat 3600
gttctccgtg tccttttctg taacagctgt gaggcacaga tgttaacggc tgctttctga 3660
ccctcagact ggtgctgagc ttatgagact tttagggact taacactgac tcaacactag 3720
ctctcagttt catggccacc ctcattgtgt gtgtgtgtgt gtgtgtgtgt gtgtgtgtgt 3780
gtgtgtgtgc gcttttccat tccccttatg aaacatctcc tgcccctaac ttactatggg 3840
aacgttcctc catgatctct ttccatcctc cctttcctgt ttgctcactt cttttcttgc 3900
tcatcttgga gactgtgcaa tcccagatta actacaaaca gagaagagct ggtgatagct 3960
ccagagctca gagaaaggag gtctctttac aagaagtctg gctctcaaag gtaagctcgg 4020
tcttcttgac ctgccttttg ccatctacct ctctctttgg atctcctgcc cctgggtccc 4080
tgcttgccct gcattctcca tactcacgga gaatcaggac ctccagaaca gcccagcgtg 4140
ggcttggtca ggaatgatcc aggctctgaa gggttaaggc tggagcctgg atggatggag 4200
agggttatta gttatcgttt ggagagtgga gtgaaggctc ctcccctgtt gcctacccgc 4260
ccctttcaca tgatctctga ctatagctgt ggaagcgggg agaagataaa ggagggaaaa 4320
aaaagttcac aaaataattt agaaagtcct tataaatgct tgtgttttca tttagcagca 4380
tgaatccagg acagcaacag accctcagag gcagcagcca actgggaatt gagtggaaaa 4440
aaataggaat cctggattct ggcagtttcc agttgttttc ttggtggggg gagccgtggt 4500

gggaagggat taaatcaaat ctttcggggg agagctcagc tctaggcttt catggtcaaa 4560
atactaaagg tcagctccgc gtgtgtgccc caactgtcct ggaagcttca attggaaaac 4620
tccaaacttt aagttttgct tatagcttca gctcaagtaa gaaaaatgag gtgtggggta 4680
ggcggcgggg gggtggggat cacaggaagg ggatcaagtc ttagatggag gggaaggaag 4740
tggagatgga tatgggtggg aataaggcag aagggagcgg tgtctttaag tctgtcctat 4800
ggttatacat atttttcttt ctatgaatgg gttttgctgt aaattatagc tttgcccata 4860
gtcccttggg tcaaaagaaa tagtctccaa acagaaaaca gctctgattg gtgactttcc 4920
ttcttccctc cccaaccccc ttcctaattg aaaccaaatg tgcaatatat tggatgctgg 4980
gatccctggc gctttcccag gaatcctcag aatgtgaggt ttctctcaaa ggcatcttgc 5040
atcagcctgt ggatgtatgc ctaccaccgg gctccttcac cagcaaagtg gaaaaagaag 5100
cgtttcacaa caaattcttc tttttgggtt ggggaaacgc agtggattat agctctgttt 5160
tcttctttcc aaaactgtgc acccctggat gaaagtaagt tatgtgggtc tttttctttt 5220
attatttttt cttttttgaa aatgaaagct ccgcatatac ttcttttggt gtttcaggaa 5280
aagttttaaa aaggaaacag aagggcgttt taagtcagac aaggaaggga acgtaatgag 5340
gcaccacaga ctcgagaaat cagatcccat gtttttgggg gtctggattt atgctatgaa 5400
gggaagaata aagagcagta ataaccaaag aagcctgaag caaaataaat gaatctatag 5460
cagctgcaag atacaataaa cagtgttagc agagttgaaa ttgttggcag gcaagacagg 5520
ccgattgcag agttagtttc aggatgaaga aggcagagca ggagagctaa gtttttagac 5580
atttagtagt ggtgttagaa ggtcctgagt tttggagata gtgtagacca agcacttttt 5640
gtttttcagg ggagttgctg atttacttgt gagaagggta actttggatg aggggtggct 5700
tccccctgag atttggggtg gctaggggga ggctgggtgc atggaagatt tttcagttgt 5760
gacagtgaaa tacagcttcc cagactcatc tcctcgctgg ctctgggtgc ctttcccttt 5820
gttatggaat gactggggct gttttcattt gggacctctg agtcctggcc taggttcaag 5880
agtcccatcc aaagggaaag agaactgttc ccagtggctg aaactctcag accagccatt 5940
tgaggcaaga gttcgtactg ttcaagccag tagggggttc agcaccattt tgccgatcat 6000
tggcttccta caacacagag ttaagagtcc tgaaaagggc cttaggcctc tcatctaacc 6060
ccttcgctcc acagatgtaa aaaatgatag gaaggggacc gacctgttca agtcccacag 6120
cacatctgta gtggagctga aaatgaaatt gagattctct ctactgctag tccagtgttc 6180

tttccactcc atacctctgc tttcatttcc tttttaaggc ttattctggc tgagacaata 6240
gaggcagtat gatgagcaag tcaccacaag gacgttaatt atcagtaata gagaggacgc 6300
caatggcggc tcatcagcta atgatgtcta tggttatatt tgtgtgtgtg tgtaaaataa 6360
aaagaagcta aattctgagt attttctatg cagcaggctc aaaataatac ataattcatg 6420
ctcagagctc ttcatacata atttgattga ctctcacagc tatgtaaggc attatttcta 6480
tcgtacatgt aaggacatta acgcttagag aagcgaaatg agttgctcaa ggaaaggtag 6540
ctaatacatt tgaaactcat gtatatctga ctctaaaacc tatgcttgga atcactattc 6600
aatgatactc ccctcatctc ccttgcattt ggttactttt ggggtcaaat tccatatttc 6660
actggctgat caggaggtga aggtgtcagg ctatgggagc agtatggatt ctctctcaga 6720
ggccacttct gcacttggct gaacatttgt ctggatatca gatgtggggt tgatttctgg 6780
gtctttggta tatacttacc tttgttatgc aaagagcctg ggacttagag tcagaagatt 6840
caggataagt tctggctcta cctttactac cttagcaaag ttgctttgcc tttctgcacc 6900
ttgacttctg tatttattat atgggaacaa ttgcagtacc tttcagtgtg attgctgcaa 6960
gaggtattgg acatgcatgc atgctgaagg ctatcaaggg cttacccata tcagctcttt 7020
cgattaggat cggtaaacac agaacgtcat atgaggcagc ccctgtggtg ctgggtctgg 7080
cagttggtgc tgatgctggc aggagggcag atggatttgg atgtgtgtga aactctgaaa 7140
tgggtggcct cagtcttagt gcccatctcc tttgaatgga agcaggcctt cctccctccc 7200
gggggaatat tcctgagggc tctgtcttaa attctgtctc ttaaattctc ctccactggc 7260
ctggtttgat atgattcctt gcccaaagca gtgcaggctg aaggacactg tgcttggtga 7320
agcatcccct gtctctgaaa aggaagtgtt tagtcaggac cagtcccctg attgctcctc 7380
agacaagact gacatttgag gagcttcgta tctagctgag ggcagatcct tgacccagga 7440
ccttcttgtc actggcaggc acatctccct tcatcctttc cctggcattc cctattctgt 7500
gcctgcagtc agaagggagg ctgctgctcc tggggtgggg gtggagagcc agcctgcagt 7560
ggggaggcct cagcctctgt ttctgggtaa gatccatttg ctttagaaac cttaaatgta 7620
agggaagtgg gtaaggtgtt tccggaatgc ctttcactta gattcctcaa cagagtctcc 7680
atgtccaagc acaggacctt atcacctgct tattctcccc tagccactct gtgaactcgc 7740
acttttcttt ccttcttgta taattgctcc ccagtcttga agtcatctga attctctatc 7800
acacacagtg aatttacttc aaacctttcc atgtatttgc atttttaata gacacatgaa 7860
aaaattggag cggccacccc ccatacattc atgtcacacc attttcaatt cgcatgagct 7920

ctcagatatc cttcccaagc tcccttacaa gatctgcttt caggcacttc acatcaccct 7980
cacctcaaac acacaatcca aacacatgtc tcgcaggcat ccaaaaagca aacacacgtc 8040
taaaagtgga cctccacaga catgctaaca ttcttgtgcc agctcacata aatggtgacc 8100
tgtccgcaac acagacacac tggactcttt atacctcttg ccctgtttga agctgtgcaa 8160
catatgaatt gatcgactct gtcccaacaa tgactagtca gtccagaaat gtgtccagca 8220
tccccaggca tcccagggct tctctgaatg cagcaaccat ccataatgag gactgttcac 8280
caggaaatta gaaaccccag gtagacctta aaagattttc agaccaatca aatcagaata 8340
tctgaggttg gagattggga atcagtactt tgaactgctc cccaggtgat tctgatgtga 8400
aacagaatta agtacctctg ctgataaatc tccaactgtg ttgtctttgg caattcaatt 8460
ttattccatt tgatttagtt ctattcatca aagatttagt aacactgtgc taataacata 8520
gtttagtaaa gaagctgaga tgtccagata cctgtcaagg atatttattg gaacatcaat 8580
cacctcgtgt ctaaagtgat gatctctgta aatttttctc tcttttaatt atatttgtat 8640
atgtttaaat gtgctattat taagcctgca ttattcatgt acgatttggc taaaaatggt 8700
tctcttattt tctaattgtt ttgcatagat atatttgatg tttcctaatt ttataaaccc 8760
cagcatttgg caaattgtgt acttattagc tggaatctta ctttggcttt gaaatgctga 8820
agaaggtcag ttttgagcca gcccaagagt tgctaaggcc tggaagggca atgcaaaggt 8880
agaggaattc taaacatggg tttggactca ctacacagct cctgagttta gctgggcttc 8940
tcctggaaat tggatattgt ctgtaatgga tccactcatt aagtcgtctg tggcttgtga 9000
tttgtgccta aaagaacccc acaaaaatct gtgaggggcc ctttggatct atagctccga 9060
ggttagtgct ggctggtgct ctgtagtgtg agtgagaggg agagggcatg tgataaatag 9120
aacttcagat gcgtgatgta gtgaaaagaa catgtagtga ggctctagtc ccaccaccat 9180
cattttctag ggtttcgacc tcagaggagt tttttaactt cattggtcct caatttcccc 9240
ttgagttttt atccccttca tttgagttta gataagataa tgtataggaa actggaaagc 9300
actgtacctg tgaagatggt aggtaatgca agataatcct tagggcctta atagcttagg 9360
tagccaaaat aattgagatg ggagatggat taaaaatgag ggcaaataat ctagcatttt 9420
tgcatttttt ccttttaaaa tttgaatgct cttatgaatc caaaaaaaca tgaaacaata 9480
caaatctcag tgaactctca tgtctaagat agttttccac tgattttatt tttctcttga 9540
atagtggtga tttaaaaaaa aatcgcaggt atatttttta tctattaaaa atatcatagc 9600

atcctggtaa actctgtcat aattttgttt tgctgtattt atttatttac tcctcagtaa 9660
gtagttattg gatgcctagt ctctgctgga cactgttcta tgttttatga tgaacaaacc 9720
ccatgaagtt acaagcattt ataagcattc attgctttag gttctaggga gtataatatg 9780
aatttgactg tgtgaaactt gatttagaaa attggtatcc acattgggtt aataaaattt 9840
agtttttaaa gtttactgaa ctgtaattta aaaataatac agaaatagtt attaaatggt 9900
gattttcagt ggagcaggtg ttatgccata aataggaccc caaattttat attttttata 9960
atttttaaaa attatagagc aactttagct ttacagaaaa actgagaaca aaatgcagag 10020
ttctcatgaa ttccttctac cctttgcaca atttctctat tattacaacc ttgtattagt 10080
attatgtttt atttgctatc agttactcaa tgtgtattta ttaaacataa caaaataata 10140
aataaattta ttatttatgc cctatttgtt attatgaaaa gaatacatca tattgctgcc 10200
tctcaagctg tttataatgt tatgtagcta cagtttgagg tttctcattt attagaaaaa 10260
ttttttttct gtgcacttag tgcatgcctt attcagtgct agggcttaac acattatctg 10320
gacatagtag acatttaata aaatatctgc tgagtataca gacaaatgaa atgaaggatc 10380
cagaagtaaa agacgcaatt cttgtcctta ggaacttagg aaacaacaga cacacatagc 10440
agtgactgga gaaaatttca gaaatacata aaatatgatt aaatctctga taaaggagac 10500
attggtatag gcaaatgagg cataaagcca ttaacaagag ccccatcctc tttttcctcc 10560
tttttgggac tgtacttttt tttaattttt tttatttgtt tgagacggag tctcactctg 10620
tcacccaggc tggagtgcaa tggtgcgatc ttggctcact gcaccctctg cctcctgggt 10680
tcaagtgatt ctcctgcctc agcctcctga gtagctggga ttacagacac ccgccaccaa 10740
gcccagcaaa tttttgtatt tttagtagag ttggggtttc accaggttgg ccaggctggt 10800
ctcaaactcc tgacctcagg tgatccacct gcctcggcct cccaaagtgt tgggattaca 10860
ggcatgagcc actgcgcccg gcctgggact gtacttctta tcatcaccat tgtctttgtt 10920
gccatggtct tcattattat catcataatt atggctatcg ttctatgtct aaatccaagc 10980
attaaagaac aaactcccat atccccatgg taaggttgga gaagtgcgtg caagtgtgtc 11040
tatgtgtgtt tttggcaatt ttaaagtaca gtcacaccaa tatatgccac ttccatttct 11100
tgctggaagt aaaatgagtg actacttggg ctttgtaaaa gaaaatcgca ctttggaagt 11160
ccttgtggga gccataccaa aatgccttgc agtactgtaa cttctcagag gcatgtaaaa 11220
gttagaggat tggtgaagtt ttcaaaagct actggcactt gtccttcagg atgaaccctc 11280
ttgagatgca ggggagcaaa ttgacctcaa taagagagta gtgggagcta aattcactca 11340

agaatagcaa tctgttcctc agacagatgc agttgtaatt ttagaatgtg tacataacag 11400
acagtttaaa atggacataa taacgctaaa atagtatcaa tggaaattaa aaataagtcc 11460
taatcccaac caaaattcta gcaagttgtt ttgtggatat caacgaactg attctttttt 11520
tttttctttt ttctttttct atctttgaga cggagtcttg ctctgttgcc cagactggag 11580
tgcagtggcg caatctcggc tcagtgcaac ctccacctcc caggtccaag tgattctcct 11640
gtctcagcct cctgagtagc tgggattaca gacatgcacc accatgactg gcctcaacca 11700
gctgattcta aagtttatat agacagttaa aagaccagat tagctactgc aatattgaat 11760
gagaacaaag tcaaaggact gacagtacct gacttcaagg cttactgtaa agctctggta 11820
atcaatacat tgtggtactg gtgaaaaaat aaacaaatag atcaagaaaa cagaatagag 11880
agcccagata gacccatata atgtagccaa ctgttctttg acaaaggagc aaaggcaatc 11940
tgatggagaa aaggtagtat tttcaacaaa tggtgctgga acaattggac atccacatgc 12000
aaaaataatg aatcttaggt ataaacctta catccttcac aaaagcaact caaagtggat 12060
catagaccaa aatgtaaatg caaaacaatg aaactcatag aaaataacat aggaaaaaat 12120
ctcagtaatc ttggatattg tgatgacttt ttagttacag caccaaaggc actatctgtg 12180
aaagaaatcc ctgattagct ggacttcatt aaaattgtaa acttccactc tgtgaaagac 12240
aatgtcaaga gaatgagaat acaaaccaca ggttgagaaa aatacagatg gtgctgactt 12300
aaaacggttc aacttacgat ttttcaactt tacgacagtg caaaagtgat actcattcag 12360
tagaaatagt acttcgaatt ctgaatttca ttggacaaaa ctatggaaac agtaaaaact 12420
attcgtagtt gccaggaatt aggggtgagg gagggataaa ttgaacacag aggattttta 12480
gggtagtgaa actgttctgt ggttactaca atggtggatg tatgtcatta tacctttgtc 12540
aaaacccata gaatgtacaa cactgagagt gaaccctaag acaaacaatg tactttgggt 12600
gaaatgattt tttttttttt ttgagatgaa gtcttgctct gtcgcccagg ctggagtgca 12660
gtggtgtgat ctcggctcac tgcaacctct gcctcctggg ttcaagcgat tttcctgcct 12720
cagcctcctg agtagctggg attacaggcg tgtgctatca cgcccaacta atttttgtat 12780
ttttagtaca gacagggttt caccatgttg gccaggctgg tttcgaactc ctgacctcat 12840
gatctgcccc cctcagcctc ccaacgtgct gggattgcaa gtgtgagcca ccgcgcccag 12900
ctgaaaatga tgtgttgata tcacagacca ttaggtttat tgataggctt attgattgta 12960
ataaatgtgc caatgtcgtg agggatgtca atagtgaggg aggttttgta gcagaggaca 13020

agggtgtata tgggaacaat ctgtactttc tgcttaattt tgctgtgaac ctacaactgc 13080
tttaaaaaga attaaagttt aatttaaaaa atctgcccaa tctccaaatt ctaatacttt 13140
gattccatct tggctacttc tttcttattt gaaggcagtc attgtaatat agttataatc 13200
atgagcatct gtattttaaa atgcttaatt ttgtttttca gataagtacc ctaaacttaa 13260
atgatattat tttatttatt aaagctacct gaggcagaaa tgtttcaaaa tcttgaaaaa 13320
gactaattca ataggttaat agcagataag taggtatttt tgtgtcatac tcttcaaatt 13380
ataataacta tcacttaaag aatagtcatt tttgttcgtt tgtgatcctt ccacctcagc 13440
ccctcgacta gctgggacta tatgtgcgta ctaccacacc tggctaactg ttgttttttg 13500
tagagatgag gtttcactat gttcttcagg caggtctcaa actcctgggc tcaagcaatc 13560
ctcccacctt ggcttctcaa aatgctagta ttacaaacat gagccactgg acccaccctc 13620
attgagctgt tttaactaca acttggtatg gtagaaagag tgatgggcca agggttaata 13680
gggtgagatt ctaattttaa ctttgccacc tacaagcagt gggactttgt gcaacaatgt 13740
tttaaatctt tctgcattta aatttttctc atctgtaaag ggcaatatac aatttctgta 13800
ttctccagtt cagttatgct aatcgctgtc ctgtcagcac ccacatcccc atattcactt 13860
tcacagataa gacttgagtc gctatgatct tcaccctagt tgtcaccctg tccccatact 13920
ctctgaatat gctcctctct cctctcattt tagccaaata ccactcttct ttgaaggcca 13980
gcttaagttt cagatcctcc atccatccca gatcttcttg gaatccctag ttcttttggt 14040
cagtatcact tagtttagca aagaccatgt cttaggtgat tgctctaact caccttatag 14100
cattaaccca taagtgagta cttactgaac tctcactgta tattagatta agggcttatt 14160
ttatatgcct ggcacagttc ttatttgttg ttggaaaaat ttttagaggg cagtaattga 14220
aatgtcatat actatagtct ttgaggatgg aaaagagctc agtaagtttc tattaattgg 14280
ttatgccgtt aagatcagca tctaaagaga gctaggtcat ggaaaaaatg gcctaagtac 14340
tggacttact atcagaagta ctggacttaa attttaattc ttatgatttg tgaacattgt 14400
agaaatatac taatgtcttt gtgagtacag ataaggagga acagataagc ttatgtgaaa 14460
actcggtttg tattgggtct atattatata cagggctatt ttattttatt tatatttatt 14520
tatttatttt tgagacagag tctctccctg tcgcccaggc tagagtgcag tggcgggatc 14580
tcggatcact gcaacctctg cctcctgggt tcaagccatt ctccatcctc ctgcctcagc 14640
ctccgaagta gctgggatta caggcaccca caccacgccc agctaatttt tgtattttta 14700
gtagagacag ggtttcacca tgttggccag gctggtcttg aactcctgac ctcaggtgat 14760

ccacccgcca tggcttccca aagtgttggg attacaggcg taagccacca tgcctggcct 14820

attttattta tttatctatc tatctatttt ttgaggtgga atttcgctct tgtttcccag 14880

gctggagtgc aatggcgcca tcctggctca ccacaacctc cacctcccag gttcaagcga 14940

ttgtcctgcc tcagcctccc gagtagctgg gattacaggc atgcgtcacc atgcccggct 15000

aattttctgt gttttttttt tttttttctt tcagtagaga tggggttttt tcatgttgat 15060

taggctggtc ttgaattctc gacctcaggt gatctacccg cctcagcctc ccaaaatgct 15120

gggattacag gcgtgagcca ccgtgcctgg cctgttttat tttattttaa caacaaatag 15180

aaggtggtta ttccaggagg gtttcagaat actaccccag atttggtttc tcctggtttg 15240

actacaggca gtgctggtgt ctttctcttg atcatactcc tttcctcatg ggtgtgttca 15300

gtcctctgat gatagaagtg cttaccttta tttaaccttt ccctctctca ctgagtgttc 15360

tctctttcct gaaatacttt tcatttgtta aagcttttaa gaaattctta tttgaacatt 15420

cagtgttatg taaaaaacaa attaactgct gcttaatatc atgtttggag gtgaaaaatt 15480

ttctaagcct tttgcttagc catttattac caaaggggta tcaagcttgg atgtcaagct 15540

aggatgtaaa ttctagacct atacaattcc ttcaagggcg gctgaaaagt tgctttctaa 15600

tccttttgct atatttggtt ggcaagcttt gttttcacag tttaaagagg attgatggtt 15660

ttcctattgt ctttccttca caactttctg aagttcactt gcctgtttca ttttcatgac 15720

ttaaggtacc agcttcttct gcccctctcc tagatccacc agcgcccccc atgctccaga 15780

aaaattgagc tacactgtgc ctttccctga gctgtagaaa gttccacctc taggtttcca 15840

ggtgcttctg atcatcattt ttcaatggat tgagttgcca acatctacaa aatatattct 15900

aacaaagatg ctcagaatgt ctggtgttga cctatctata caggtaaact tcaatgactc 15960

tgcatttccc tttcactgct gacctcatgc cttttgcaga agtaatatag gaaagaagag 16020

cattgaaata gggcagaaga tctaattttc tttttctttc ttgagatgga gtctcgctct 16080

gtcacccagg ctggagtgca gaggctcggt cttggctcac tgtaactccg cctccccggc 16140

tcaggtgatt ctcctgcctc agccttccga gtagctggga ttacaggcac ccaccaccat 16200

gcccagctaa tttttgtatt tttagtagag acgaagcttt gccctgttgg ccagtctggt 16260

ctcgaactcc tgacctcagg tgatccacct gcctcggcct cccaaagtgc tgggattaca 16320

ggtgtgagcc actgcacccg gccagaagat ctaattttct aacttacaat gtactcgaat 16380

attgaaagga atttcatatt tttagaacta agccctcatt ggggttgggt acggttaggg 16440

caaggagaag cctgggaagg agttcttggg tagttaaatt ggaaaaagag agtctggcac 16500
attcagaatg gctccatatt tgttacccgc agactgtaca taagaaatga gacctggcta 16560
ttaagagata tgatgcctgt gaaaaccctg gatacacttg caacttgctt tttcccaagt 16620
gtttaggact ctagtaggga cacctccagt tccttttgga aataagctaa tatttggttt 16680
ccaaaaatgt attgaagtag tcttaatgag acaaatagaa aatagaaatt tgattgaatg 16740
ttcttacatt cttaaaaatg ctacttttta gcatttttaa aaataatgaa gtacatgttg 16800
gggtgggagt ggagaagaga ctgtaatatt tctgtatctg aagcctccat aaggtcttaa 16860
tccagccctc cataggaaca taagccataa ttgtcatcta caacttgttc ctaaatttac 16920
tcagctacag tttctcagag ctattgaggg aagtactacg atgctattta aaaatacttt 16980
caaatccatc cttatgatga tgaagttatt atagttacaa atgcatgatt cagcaatagg 17040
cattaggaaa acatatccac cagacagcct gatgatcatg gtgtatgaca ctttcttctg 17100
aatacatgag ggttagatga aagtggtgac aaacgattga gatatctaga tccacattat 17160
ttaccttttt tttttttttg agacagagtc tcgctctgtt gcccaggctg gattgcaatg 17220
acacacttgg cttattgcaa cctctgcctc ctgggttcaa gtgattctcc tgcctcagcc 17280
tcctgaatag ctgggattac aggcatgcac taccacacct ggctaatttt gtatttttag 17340
cagacagggg gtttcactct gttggccagg ctggtctcga actcctgacc tcaggttatc 17400
tgcccacctc agcctcccaa agtgctggga ttacaggcat gagtcaccac gcccagccaa 17460
ccacacttct taaggctaga taaatacata gctttaagtg tagagtaaat ggtgtctcac 17520
ttgcaaagaa gaggatctct tggtcctctc ctttccccaa ccccaaataa tggctttgct 17580
attggcaatg taaggtgggt cggaaagttt gtgaagcaat ggccaagggc cttatggaac 17640
aggcatactg caggggtggc gtggtcataa gttgctgatg aagcagattc tctgtcactc 17700
atgcctggtg aacaggcggg gactcccttc ttcaagagag acaggtgatg atagaagagt 17760
agacagccct gaacaaacct ttcctcagga ttgtcccttt tggtattact cagacctgaa 17820
taatacatgg acatgtttta tagggaattt tttttcagct ttttgggtga tctattatta 17880
ggatcttatt aaaatatgta taattttaaa actaggtata cacttcttat gcttatagtt 17940
cttatgcaac tatagagcca aaacaaatta ataaatgaaa tcaactatat ctgagcagac 18000
ctcaggattt gagagagaga gagaaaaaga aatcaaatat aactatgaaa cccaataaaa 18060
tttaaattat tttaatgtga tagataatgt ggttttgttg aaactaatca ctagtgtgga 18120
acttaaagat agacacagta gaaccatgta tcctcagggt ctggtctatg tttaaatctg 18180

ttatttaaat gtattaattt catgcaaagc atgcctgtta gtgtaagtat gttgaacaaa 18240
ttgttattaa tagctttgag ggttcataag ttcaggaaaa taagatctta acccaaactc 18300
tgtagtaagc aatcatcgaa tgtcagtttc tatgaggtgt catttgataa ttttttaaat 18360
tgtttactca cttgaagata gggttagcat tgtgttgata ttaaaattgg tactaaatgg 18420
atatctaatc catttactaa cagaatcagg gacagaacca ttcttctttg tgtgtttatg 18480
cctcaagttt ctgcttataa gctggtatgt gttagtatgt aaaaagccaa agtccccata 18540
gcacatctgc tcacagcgtg aagatggcat cgaatcctat ggctgagtag attgaactgc 18600
ctcatgccat gcaacgactc agttttccca acatttcctc tatgtccaga ttgatccttg 18660
acttcatctg gctttcactg ggcatttgtg caattgtaaa caccaatgaa aagccaggca 18720
gtgaaattgc atgatggtta ctgcttgtat taaaatgtta aattataatc gaagacagaa 18780
aattttttaa atcctagaac tcttgtggac tgcaagatat ttatcctttg agacaaactg 18840
ctttaaaatt aaatttgggc tccacggttc tcaggaaaac ttttctcatt tgtggtcttt 18900
cctttaagtt ccattaataa ttaaaaatga caattgtgag gacttctgag taatgagtgt 18960
ttattatgct ctaaaattta agataagcac tttacatatg taatctcatg aaaaccttgt 19020
aattggtgtg aggtgagtgc tttggctatt ttacatgtga ggacacatga caccaagaaa 19080
aggtcaatca catgacaagt aagtggcagc agcggaattt gaacctatat ctttctcatt 19140
gcaacgccca agccttacac actaaagcct cttcttatgt ctccatttca cattgtgaaa 19200
acctgtttaa ctgcaactag gaaccagtgc tctacaatta tcctcttaca ttcctgtctt 19260
ttccattggg ccagtgggtc tcaatcttta atatgcatca gaatcacctg gagtgctttt 19320
tgaaaaatgc aggtcccaga tgccactccg aatttactga ataagaaatt gtctgtgcaa 19380
atgtccaggg ggcctgaatc ttttaaaaca acctccctca ggtgattctg ttgcaggtgt 19440
ctgtctgatc acattttgtg taaaactgca ttagactgtg gatgcctcaa gaaccatgac 19500
ttttgaaaaa atccatcctt gtacctcaag gactcactca ctaaatgctt gtcaaataag 19560
taaaagaatg aatgaagggg caattgtcaa gaaagatgtg ttcttgggaa gaatacgctt 19620
ttggccagac ccttggaaaa tttagagatg catgtttaaa tatcacttaa tatactcggt 19680
ctagactctc cttcctggag gccaagcatg gcaaagagcc gatgagaggg tggtagaaag 19740
tacaaatcta tctataaaaa aaagaatgaa aatgctcctg accatccagg atgagataga 19800
ataaatcttc ccctgcgttt ctaaaactaa agtcaattga actagaaata tttaaatgca 19860

cttgaggccc agaaagagat tcatttcctt aatgttttgt ggacttgacg ttttctactt 19920
aaatcatagc tctaaggtca atatggattt gttaccggca aggggtccca atctacaccc 19980
caagagaggg ttcttagacc tcacgtgaga aagagttctg ggcaagtcta taaagtgaaa 20040
gcaagtttat tagagaagga aagaaacaaa agaatggcta ctccataggc agagcagtgg 20100
catgagctgc tcaattgggt atacttgtag ttatttcttg attatatgct aaataaggag 20160
tggataattc gtgagttttc tggaaaaggg gcagggattt cctttggaac tgaggatccc 20220
tacccttttt agactatatg aggtaacttc cagatgttgc catggcattt gtaaactgtt 20280
atagcgctgg tgggaatgtc ttttagcatg ttaatgcatt ataattagcg tctaatgagc 20340
agtgaggatg accacaggtc actttcatca ccatcttggt tttggtggga tttggtaggt 20400
ttctttactg catcctgctt tatcagcagg gtctttgtga cctgtatgtg tcttgtgcca 20460
acgtcctgtc tcatcctggg actaagaatg ccttaacctc ctgggaatgc cacccagcag 20520
gtctcagtct cattttaccc agtccctatt caagatggag ttgctctggt tcaaatgcct 20580
ctgacagatt cattcctgta tttagcaaga ggttttttcc tttttttttt ttttgagtac 20640
ttactaagag tcttgtattt aataagacat agtcctttcc ctttctgagt ttattttcag 20700
ttagaaaatt ccgggagcag taaaagagca atgcaagtaa atggggggct agttgctcag 20760
aatctgcaaa tgtaagagtg catggaaggg tttgtatagc caggggactg gtgctggatg 20820
ctgcaaattg aataagaatt ggaataaatt ggatagccag gtagcaaaga gcagtggaac 20880
agacacttgg cagtcagatt gcttatggct ggctggttca gttaacagtg aggacaggag 20940
cctagggtgg tgggatattt gccttgggga gcatggggaa ataacattgc atagatgggt 21000
tttctgacat taaatagagc tttggatgtt gtggaatttt gaacatttcc tttatctgaa 21060
agccggtgaa gtgttgtctt aagatgtgac agcagtatgt aagcaaattg caggggataa 21120
gaaatcaagt cagggagacc cattagaggg ctgcattctc agaacaagga gcactgagga 21180
aaaaattaaa aaacaaacaa acaaaaacaa aaaaagaagg ttgctttcgg tagtccagat 21240
gtgaagggac agggtttaga ccagggtggt ggagtgcaga aagaaaagcc agagggatgt 21300
ggaaggacaa gatggcatga ccacgaatgg tggcaaaggc agcgtggaac aagcacatca 21360
gctgatgtgg aatgtatgag aacaaaaaca ctcattttcc caccagaacc cttagtagga 21420
gaaagtgcct taagcttaca gtgaggattc caaattgaaa ttgctcagta gctgtttcac 21480
cctttgttac tggcagagca gatggctctg gaagagacta ggactacttg ttgcttctct 21540
ttcccatctc tcaccactcc ccaaccttat cccatagtga ggaccactca ctagtgacat 21600

ttctgcttga gacctacaaa gcaagtcttg ctaatgagac ttaggcactt tttcccaaag 21660
tgcaattctt ggaaatctgt gggttttaag tagattacag ggccacagtt ccgtggtcaa 21720
attagttcaa aaaatattgt tagagaatta agcaagtttc tttactgcag gatgtcccac 21780
agtctttaat atggtaatca acattttgac tatccaagag agggcgagct tagagcactc 21840
cacaaacttt ttggatcatg gagctctttt taaaagaagc accttgagag aagaggatgg 21900
cacagcatat atttaaagaa attagggact agaaagtttg tctcttgcac tagaggttgg 21960
tagtgtcatg caaagaaaca agagttggga tttgggtcca ggctttgcca ctaactggtg 22020
atcttgggta agtcattgaa tgtctctgag cctcagtttc cccaggggtt atgttcattc 22080
taaatgaact cttcagctcc tttcaaaagt ctgtcttcat attcaaaaag gaaaataatc 22140
tgcaatttct ctactctgca ataactctgg tggtggaggc agtggtagga aaagagagag 22200
tagagcggac agtgacaaga agatgaataa gatacggtct gaacccctgc actgattaga 22260
ctgttgggca caattacagt gctgggctga tgtaacaaag ataaatgaag tttgtagtgt 22320
gttctcaagc ttctctgact ccaaccacat catgtgatat acagcaacta gttcagttgc 22380
taagcaacac taattcccaa tggtcactta atccatctag tctcagttct gctactaaga 22440
agctgtgagg ccttggataa atcagttaac cctttgggtt tgggtttctt tcatccgtaa 22500
aataaggaag ttgggttagg tctggaacca tttttaagtt gacctgagtc ttacctggaa 22560
gaaggtaaag gaaacacagc tattctcctc tcacccaccc catgcagatg agccccaaca 22620
ttctgtcttc gcttttcttc tcttccttcc tcctggatca cttaatctgt ctcagacttt 22680
aattattgca tttatgtagt tgaagttcaa atgtggatct ctaagaccaa cccttctctt 22740
gaatgccaaa atcaagatgt gtctacctgt tttcattcta aggaaaccaa attacttatg 22800
tttcaaacca aactcacaat ctccccttac aaaccaattc ctcctccacg ttaaaaaaaa 22860
aataataata ataacaggcc aggtgtggtg gctcacgcct gtaatcccaa catttgggga 22920
ggccaaggaa ggcagatcac ttgaggtcag gagttcgaga acagcctggc caacatggtg 22980
aaagcccatc tctactaaaa gtacaaaaat tagcagggta gggtggtgcg cacctgtaat 23040
ctcagctact caggaggctg aggcaggaga atcgcttgaa cctgggaggc agaggttgca 23100
gtgaactaag actgcaccac tgccctccag cctgggcaac agagtgagac tccatctcaa 23160
aaataaatac ataaataaat aaataactat gtgctcctgg tcacatgcag cccagactcc 23220
caggcttttc gactccctcc ttcttccctc ctgtgctagt cattggtcaa ttctcaatcc 23280

tctccacatt attattatta ttttttgaga cagagtcttg ctctgttacc ctggctgtag 23340
tgcagtggct cgatctcagc tcactgcagc ctaagccttc caggttcaag taattctcat 23400
gtctcagcct cccaagtagc tgagattaca gtggatgtgt gccaccatgc ccagctaatt 23460
tttgtatttt tagtagagac atgttttcat catgttggcc aaggtggtct tgaactcctg 23520
gcctcaagtg atccacctgc cttggcctcc caaagtgctg ggattacagg catgaaccac 23580
cgcacccagc ccctctccac attattatat gtccacatta tttatttccc tttcctgatg 23640
tcattattgt acatcaggct ttcattggtc atcctgcccc aggtctctcc tccaaagcaa 23700
cccacatgcc aggttcagcc tcacagagct tctctgagcc tgccatccca ctctccaaag 23760
cctttcccgg ctctcttgtg ctcactcctt gctggcattt ggagccctgc gttttcccac 23820
ctcaatcatc ttccagcttc cagctttgtc ttcgattacc tgtgtgttcc tgtcctccca 23880
ccaactattt attaatcttc ttatcccata acttgcccat tcaaattgtt cccttttctt 23940
agacttttgt ctgcattctt tctatctgac aaaatcctag tgatatttca agagtcagct 24000
caaataatgg ctcctcactc caacaaatct ttagggaaca ctcaactggc ttgatttctc 24060
cttcttttag caccttgttc tttcctttct tactacctta ccaacccaga catatatatc 24120
tactatatct tgtaatatcc attatatctt ttttgaggcc aaggaacatg tttccttcat 24180
ctttctatca actatagtac atattacata ctaggaactc agaaacgtgg ttgaacaaat 24240
tactattaaa ttattaagct attaaaacaa taatctattg aaataatgta ataatcttaa 24300
tctcctaaca aaatactaat aaaatttaag taaaataaat aatctacaat aatccaaaaa 24360
tgagtcgaat cttggtttac tctttggtgt tttgtcattc tactttcgtg tcaagcttag 24420
aaataatttt ctgaagaatt ttgattttga ttttagttat aactttgcgt tagtaagacc 24480
atcagcctac ggttaaggat atttgtctca cctgtgaaat ctcactatct ccatgaaacc 24540
gactggcttc gctacatcat gggaagggtg ttatctttcc tagttggagc atcatatgat 24600
gatgtggttg aaaacatgca ctttggcata agacagactt ggtttggaat tctgactctg 24660
cccttgttgg ggctgcttgg ggaatttagt tagcatctct aaatattagt ttgctcattt 24720
gtaaatggag gatgtaattg caggtgctgg ttctactctt ggaaggattc aaaaattgtc 24780
atgcttaccc gaggggctcc tccaaacctg ggcggggttt tctgtatgcc ttatggacct 24840
acaaatgcaa accacactga ccatgtaatt ttgggggcag attggaaagc tgaaacagaa 24900
gtgcagggac tgttggtgcc catcttcctg gcctggtggg gaggaatgta ggaaagcaga 24960
ctcgtgcttc tcatcttgaa tcagacagac gacagacact ccaggaaatt ccttttgttt 25020

ggaaaagaaa aatgctctgc catcttctct taccccagtt ttctacattc ccagacttcc 25080
taaaagagta gaccactggg gttccctgaa aagccagcta agtccagcac atacgatcag 25140
ccccacagct cattttggtt cacagacaat aaggagagag gacttaacaa ttactgagtg 25200
ctgagtaccc actatgtggt aggaacattt tgtacataat tacaattatg catcctcata 25260
actttatcac ataagtatca tctctatttt acaagtagac actaagcaac ctcccaagat 25320
tgtacagcta gtaaatctga gtgaggaagt gaactcagat ggtctacaaa acttttagca 25380
tttgtggcca ggcatggtgg cttacacctc taatcccagc actttgggag gctgaggtgg 25440
gaggattgct tgaactcagg agttagagat cagcctgggc tacaatggca aaaccttgtc 25500
tgtacaaaaa atacaaaagt tagctgggcg tggtggcaca cacctgcagt cccagctact 25560
tgggaggctg aggtaggagg atcacttgaa ccaagaggtg gaggttgcag tgagccatga 25620
tcacgccact gcactccagc ctgtgtgaca gaacaaggcc ctgtcttaaa aaacaaacaa 25680
gcaaacaaac aaacaaagaa acttttagca tttgagacct tcagtgtgat caaagcaaat 25740
tagctttgtg aggtggggtt tcctttagct tctacgtcag caaaattgtg aaccaacagg 25800
attaaccttt tgccgtctta gtactgcagg gaactagctt tcagaaaatt gtttgggggc 25860
cgggcatggt ggcttgtgcc tgtaatccca gcactttggg aggccaaggt gagtggattg 25920
cctgaggtca ggagtttgag accagcctgg gcaacatgtc gaaaccctgt ctctactaaa 25980
aatataaaaa ctagccaggc atggtggcga gtgcctgtaa tcccaggtac ttaggaagct 26040
gaggcatgag aatcgcttga acccgagagg cggaggttgc agtggctgag atcatggatc 26100
gtgccactgc actccagcct ggatgataga atgagactct gtctcaaaaa aaaaaaaata 26160
ctgtttgggg acatttgggt cctattttct tccctgatgc tgatgtgatg ctgatgatag 26220
tgagtgtttc aagaaccagg agaggctggt tgttgtaaac attcctataa acgttggctc 26280
agagattgtt aatgtaagtt tttctggagc agtgaggtaa gccccaagta gaaaagggca 26340
gaagtatagt aaaatttggt tgttttgtca actttgtaag acttcagctg cataccttgt 26400
taggaaacta gaagccggag gaaagttgtg ctgagatgtg tagcatttgt tcttgctttg 26460
tgtggccatt ttattcccat taagtactgt atccttcact aaagaaatgg gccattcagc 26520
agcccgcctg aacactgtgg cactgtatat tgttatagag agttatatcc attcagaggg 26580
acacaacaga tcagaggaaa ggaaagaggt aaccatgtat gcagacgtgg aactgtcccc 26640
aacatgtata atagacaagc caaggtgaaa tgtatagcat actaatattc aactttttaa 26700

atttaaaaaa ctatgcatct caaggcatgg tggctcacat ctataatccc agcactttgt 26760
gaggccgagg tgggaggatc gcttgattgc ctgaagccag gagttggaga ccagcttggg 26820
caacatagca agacctggtc tctacaaaaa atacaaaaat tagctgggca tggtggtgtg 26880
tgcctgtagt cctagctact taaaaggccg aggcaaaagg atcacttgag cctgggggtt 26940
caaggctgta gtgagctatg atcactccac tgcactccag cctgggcaac aaagcaacac 27000
cctgtctaaa gaatacaaca gcaaacaaac aaacaaaaaa ccaaaaaccc tcaaacacta 27060
taaatccata gtttttatat atgtgtaaac tatttctaga aaattactca agtaactgat 27120
aattagttat atctgaagga gggtgatggt gcggtggaaa tgagactcac ttttcatcgt 27180
gtggagcagt gattcccagt gtgttctggg attctgggga gtccttgaga ccctttcagg 27240
agatccatag atcaaaatta ttttaataat aatactaagt tgtttttatt ctcattctct 27300
cacgagtgta cggtggagtt ttccagaggc tcagtgacat gtgccattgc agcacattga 27360
atccagaagc agatacgata atccaactgt gtctggtcaa gccaaatatt taagagattt 27420
taaaaaatgt aaaatgatgt cattcttctc atgttgaaga taaagttatt tttataacaa 27480
ttttgaagat aaagttattt ttataacaat tattattcaa caaaattact attatttaat 27540
gagttaatac acaataaatt tcaaattctc agttttaatt tcttaactgg gcaagtatca 27600
gtttaggtat aaacaacatc cctccgagat cctctgggat acatccaatt cctttagtgc 27660
ctggtgtgct tggaaggttt ctgttgcacg gcgctggaca ggagtgccaa agaggaaaga 27720
aatgtgaatg gaaccaaagt ctataataaa atattgcttt ctctttttcc cagcctccat 27780
caagggagac ctacaagttg cctggggttc agtgctctag aaagttccaa ggtttgtggc 27840
ttgaattatt ctaaagaagc tgaaataatt gaagagaagc agaggccagc tgtttttgag 27900
gatcctgctc cacagagaat gctctgcacc cgttgatgta agtaggtcca gtcactgagg 27960
ctctgttacc acatgagatt gggagtggtg tggctcacat tgaatccccc cacttatcct 28020
gcctggtgtc acagggaaag ttgaacactt ggtttctgtg cctagagctg ctatcagatg 28080
agaagacaat gtatttgact atataggaaa tctgttgtgg actgtcaatt aactcatttt 28140
cacttccgtt gatttggttg tgaccagccc tagattagag ggcagtttta gagtgtcctg 28200
gcctagaatg tcttcctcag ttttgagtaa gccagggtac ctggcctctt tctgggactt 28260
ctctgccctt acaggaccca ctggaatctg tagggttcca gcgtcaatct gtgttttcta 28320
atattacaat ttgtttttat tttttatttt taacaattat tttataagtt gttttcttct 28380
gggaataatt tgaggtgatt tagagtaaaa caatacttaa tagaagcact acaaccagga 28440

taatagtaca agagtcctgt aattatattt tgggggaatt aaatgtaaga aaacctagcc 28500
cagggatact gattgcagat tcatacagcg cttagctggg actcaaggca aaaagagaag 28560
catgctatat tgcagaactt ttattaggaa accatttcca catatcaagg tggtctgctg 28620
caggtgaatt ttggtctgaa tgggacttct cagttcatcc tttggcaggc taaaatccca 28680
ctgaggatct agaactgtag caatttctag attgtgtctg agaccttccc agcctggagc 28740
tccatatctc cttcatgcct gtgcctctag catctagcac agggtctgac acatggagga 28800
tgctcagcga gagccctcag attcaactta ggctttactg tattctgcac agcttctgga 28860
atgagtgctg aatgaatgaa tgagtgagta aataatgtga atgattattg ggataagggt 28920
ggggctaaag tggtattttc ttacattccc cagaagattt cttctccctg tcaagatgct 28980
ttttggatta tctcttcctt tctgtttatt atctagggaa tgcctcgctg ctctttagct 29040
tcctggtggt aataattcac ttcatagttt aatgtcttag gtttccttct tcccacagat 29100
tgccttcaaa tttgcctttt cttcctccct ccctttcttc cttcctccct cctttccttc 29160
ctcttttcct tccttatgat agactcttgg tatgggggag ttaagaggtc ttaacttgag 29220
ccttcacagt gtatatttta gaatgaataa ttcattgcta tgcttcacca ccaaccagaa 29280
attattacag ggtttctcta ttacatgaga tttttcaaac attatttatc tatgatttgt 29340
tccgctttac ctttcagaaa ctatcactgt ggtcttttct tcccctgcca ctggagtttt 29400
tttttttttt ttattttttt tgttaacagg gttttgctct gtcacccagg ctggagagta 29460
gcagtgtaat cttgtgcaat cttggctcac tgcaacctct gcctccaggc tcaaattatc 29520
ctcccacctc agcctcccaa gtagctggaa atacaggcac acgccaccac tttgtatttt 29580
ttgtggagat gagggtctca ctatgttgtc caggctggtc ttgaactcct gagctcaagc 29640
aatcctacca ccgtgccctc ctaaagtgct gggattacag gcgtgagcca ccatgcctgg 29700
gcaccaatct taaacaatga tttccataaa ttactattag aatattcaag gcatatgcta 29760
tatatagcct tgtcctgtga agcctgattt tggtgcactc ctccaccccc actgtcatat 29820
tatccctgga actatctcaa acaccttggc tcctccagga agagtccttc tggtcaccaa 29880
ttcaggctca ttctctttct gggaacgatc agactgagtt tttcaatctg tttagagatt 29940
tactaagttt tagtttcttg tgtttcttat taatagggag accaaataat tcagcatcca 30000
atctggagaa ctaatgagga caggagaagt tattaatagg taatctggaa cataaggcat 30060
actctggatg ggtagccacc tgcttttcaa agatcttgcc tctttaatgg gtactgagag 30120

cagaagggta acaacagcct ttggagattg aactcctgaa ttctgttcag ttgtctgggg 30180
ctagggaact ctgacacaga tattgtagca ctgagtattt ctaacaagct gtggggggcc 30240
tgtcccctag tggatgttag agttcccaga gggagaagtc aagaccactg cagcaccgac 30300
ggccccgtgc ttatgagatc cagtggtggt acccctagtc agtcttcctc caccctctcc 30360
ttcctctttt cccttctcct ccccttcttc ccttcccctc cccactcccc agtctgctcc 30420
cttctctttt tgatccttat tctctccctc tcccttccaa cgcccccacc tcggctccct 30480
cttgttttgt tttctctttc catcgccttc tctttctcca ttttcttctc tccctttgcc 30540
attcacagtg tcttcctttc cttttctctt ctaaaatggc tcttccaaaa tcactggagg 30600
gcagctcaaa agagcaactc tgccaatatt aatttctcct tgaaagggaa aagcctgcat 30660
ttccaattat gacttagtct tcattagtta gaaactgtct tttttccaca gcaagtggcc 30720
taggaatggt gtgtttctcg caagtctctg gtcggaggtc ctggttttat gtattctgct 30780
gtgcacgctt tctagggcat gaagacaggg caggctgggc cattggaggt gggggtggca 30840
gtgaggatgg cggtctggct gtgacaggaa cacacctgga aatgtctgct tgtcacagaa 30900
tggattgcag ctgaagtaaa caaaactctg tgatcatgtt gctgcataaa cctcgatgtc 30960
tgaaatttgg gacgatggct gtgccaggat tattttagaa aggcaagctc aggatcagtg 31020
ttcaactaac cagagaatct acagctgctg tggatgtccc tccaccagag agagagagag 31080
cctagcagaa actgaggaga gaagctcttc aggccgctca atggctcacg tctgtagtcc 31140
cagcactttg ggaggccgag gaggaaggat cacttgagcc aggttggaga gcagcttggg 31200
caacgtggca agaccttgtc tgtacaaaaa aaaaaaaaaa aaatgccagg tatgatgggg 31260
cctgcctgtc gttccagcta ctggggaggc tgaggctgaa gaatggtttg agcctgggag 31320
gttgaggcat ggtaagccct gttcacatca ttgcactcca gcctgggtga cagaatgaga 31380
ccctgtctca aaaaataaat aaataaataa ataaaagttc ttcaaagagg aatggagggg 31440
aggaaccaag ggctgacatg gacaattcta ggtaaacagg gctccagaaa cattctgaaa 31500
agcataagct gagagcagac ttcagtaaca tgaagaaaga actggggagg aggggagcgg 31560
tctagagaat aacatgctct tatttgttca tgggattcat aaactgtgga caatattttt 31620
atttaccaaa aaggtaacaa agtttattga gcacctgtga tgtgtaaggg actctggaag 31680
tattttgaag ggatacaaat ataaataaga gataactcca gccttggttt ctcatgggct 31740
aataggaaag gaaaggaatg aaaaagagaa ttacactcaa gggaggtaag catgagtcag 31800
gacctgacaa atggttgaag gaaggaaagg acacttgtag ctgggatgtt caaggaacac 31860

atgctttgaa tatggagggt gtggagcttg acctaagcct tgggggctag gaaggaattt 31920
gatatgcaga aggaaagatg tgggtcatta tccttggagc tgacctaggc agtgaggacc 31980
cagagtgatc tggcgaacac agactgtgct gggcatgtgg agtggagggg aggtaggaaa 32040
cggctgtcta cttggggcag aggattgggt tgaggagtgg tggaagataa tactggaaaa 32100
gaggacactt gccagaatgt ggaaagccct gactgttggc tgggctaaga catctggaca 32160
gtctggacac cattttaggg attcagcacg tgcgttttac gtgctctagc agggaaggat 32220
gactttactg ttagagatgc tcatttctga tgtccctctc gctgagtgtg agtgcagtcc 32280
tgaataagat taaaggtgtc ataactataa accatttgga aaatgtaaaa atatatatgc 32340
atgagacact tggagcaagg atttctaagg gtctcaattt cttgtttttg ctttgattgt 32400
tatagcttat aagagtagat ttaagggtta gccacaaact ctattactgt cagagcagat 32460
ttgggctgct aggtagaatc ttattttcac tgtttcaatt ctcatttctt tgtaattttt 32520
tgtattatac tacatatttt agccccaaat tgtgggctgc aggaacaacg ttaagtattt 32580
cttgctggct gtgttcactg aattgcttcc tgagcctgaa cttgctcaaa cttcctgttt 32640
tttgggcaca gaactcctgg tttctccatt tatgatggat gagaggggtt tggagtctct 32700
cttctagact ttcatagcat gtttcaggat ttctcactga tatctaattg tcttataaga 32760
gacattaaat ttttttcaca ctcaaaaatc catgatcccc tctaggtaag gtctgagtct 32820
ctgtcaaaca ttttcgcttt tgtgtaacat ttagcacagt atttatcagg cattattttg 32880
tttgggggta gaggaggtag gcggcggtgg gcagttggga ccccccgtac ttatgttctg 32940
ctccaatatt gtcttgtaga aaatggtcta aaatagatct ttactatctg aaatatcatc 33000
tagtgagagc attggcatca tcaaagagct tgttaaaaat gcaggatcta ggacctcaga 33060
ctaaatcaga atctgtaatt tgattggcct ttgagacttg ctggtctcca gatgtaatgt 33120
atacacatca cctagggatc atgttaaatg caggttctga cactcaatta cttaggtaat 33180
gatcaaacaa ccagatcttg aagttttcta acatcaaagc agcaacttcc tttctagagt 33240
tgtcaccgga caccttccct cctgagagtg acacttctgc acagaacaga tggtttatga 33300
caggagttgg aaaactgtgg cctgctggtg gcatccagcc tgccacctct ttttgcaaag 33360
aaagttttat tagatcacag tgacgctcat gtatttacat attgtctgtg gcttcttttg 33420
tgctgtactg actgagttga atagttgtaa aggagactgc atgacacaca aagccaaaag 33480
tatttactat ctgggacttt atagaaaaca gtttgctgac cctggcttat gtgagtgact 33540

ctaaatattt ccttaaagaa tcaaatagct atactctctt gccccagtgg caaacttcgc 33600
aagcccatct gaatccatgg ctattcatag agaagctgaa ccataaaccg tcattcccag 33660
gcaacccaca aagggtcttg agcctaaaat tggtctggat aatggtacca cagagcctgt 33720
gactttccaa aatccattta atatgtccct cctcaaagaa atggttataa ttttatataa 33780
ttttattgag ctgactccct cctccacaaa ttgatttcat taattgttca acataagtct 33840
gactttcccc aagaattgga atcaaaagga aattctgagt aaaaatatat ctgggaccaa 33900
ggaaataaaa agacttgtac aagttggtta taatacacat tttactccta aaaaggtagc 33960
gttggttttg gccccactct ggcctattca ctgggcagac tgtatgggct tggagtcctt 34020
cttttaattc ccttggagaa tcccaccacc tgtctcttat ttgggttttt gaattgtgaa 34080
atgatggcac acacaagctc caagaatagc tgcgtgtgta caccaagcat acagagacag 34140
aaggcagaga cttaggccaa ggatcaggaa atacagaaga gaagctggca tggaggcatc 34200
tgctccacaa acacagtgaa gccggaggtt gtaggaggag ggaggtggga cccgtgtaat 34260
cactggagga tgtgggcact ctgctctgct ctgcaagtct tggagatctt ggttttcttt 34320
ccagaaccaa atttcagagg aggtgggtgt aggagaattg agattctttt tcctcatcca 34380
gagttatctg ggccaaaga ggtttagggt gagattctgt gggaatcttt gctgtagctc 34440
cagaagcttt attcttactt agttcctttt ccctttgtct gcatagtcca ggacactaaa 34500
agggtttgtg aagagtccgg gtagacataa gtggagagtc ttctccttaa ttctagagtc 34560
tatcttgact tcaaccaatt aaagattgta ggagtgaatt agagcccctt atgttaaacc 34620
ttgtttcttg gaagccctcc tggaattccc taagatgcag agctgattag cttagctggt 34680
cagaatgtga aggttttaaa gccaaagtta ttatttgggc tgatatttta gacattctct 34740
tcattggaag aagtacaatg tattcattca ttcattcatt cactcattca ttcattcact 34800
cagtgaatat ccattgaaaa cctattgtgt aacaggatgc aggaaaaacc tgaactggag 34860
agtcagacac atctgagttc aaattctagt ttcttgatct gtgagctaac taacacctca 34920
ttcagctttc catccaggtg acataaccac cccagcttcc ttccctgaat ctgcaggtta 34980
ggttggacat ttacttctct gcttttgcag caacttctac accgtctgtt ctcataccac 35040
attatatgga aattatccat ttgcagtctg tctctctctc cagattgtaa agtcattgat 35100
agaaaagagc ttcatttcac atactttttt acccttagtg cctgacatat attagaaatt 35160
agacatatgt ttattgcact gaaaagaacc taattgtgtg gcttaatttc cctaaagcat 35220
gtgtttctca tctgtaaaat gaaggtgata aaaacatgcc ttgtgagttt tttgtgaaga 35280

tcaaatcaga taatgtttga atgagtgttt tataaatatg aagtgcttgc aaatactaat 35340
agttgttcta atatctggat tgattgcgat tcagtagaaa acaagcagaa gagaatcaag 35400
aaagtatgtg gaggtgtcct agtccacttt ttctaaactg aataactgaa cgagggggat 35460
ctggagactt cagcttctga ggcacagttt gggtgtgtgg ggttgacacg ggacagagga 35520
ccctttccag caagctcgat gtctctgcca ttagattata tactcataac agtggggata 35580
tgttttatta atttcattgc ccctttattc accctaagca taactaagaa agtgaatatg 35640
taataattgg atttcaatgg gcattgcaag gccaaagtct cctgcccccc aaatctccca 35700
gtatccctgg cctgtatgta tcttttcttg agaacctgaa taatggaacc attagattga 35760
aaacatagat tggggagaag agagtgagtt gaatttgaaa aatgttgagt gtgacatctg 35820
gcagtcaaat atgcagggct gactcatttc acttttcatg tgtctgtttt agctcacaaa 35880
ctagacatag agctcattgt acagattcca tagtgcttaa tgcactggct accccttaaa 35940
aaacctatag agcttgctgg agggggtccc ctatctcatg tcaactcaat aaataaatat 36000
gtatggattt gttgcaaaat attggatttt cttttccatg taagtaacaa agaactgctg 36060
cagatatttg agcagagttg taaaatgata tgaattttgc tgtgagaaaa cttgatcttg 36120
ctgtaatgta tgaaaaagac tagaagggaa agaagttgag ggtggagagt cttgttagga 36180
gggccaggat aagagcaggt gggcggtgaa ggaggctgcg gcagaaccat gccaagcaga 36240
atggagtgga ggggacttga ctacacaaga ggctggtgac tcagctttct gcctagtatt 36300
agtcctagag aagtctccct gctctgggaa gaccggtgtc tagcctcatg tggcttcgga 36360
acaaaagcca gtgattgcat ccaagggaag ataatgagaa acacacaggc cgtgaaaatc 36420
aggaaagcac cactaatctt tttgttgcaa aactttactt ttcaaagtcc tggggaaggg 36480
tgcagaagcc tcagcacgtg gacttgagcc ctgtactcgg aaatgcaaaa tggaaacccc 36540
ctaatagatc acagaactga aaatatccaa gagtctgaga ggctggggga ggacaggagt 36600
gagaaaagga agggggctgg agtactgatc agaagatagg agaaagcaac agtgcaaaat 36660
gcggggaagg gctgagtaga cattcaattt aatctggact tttattttgc tacaaactaa 36720
ttttatgaag actttttttg cccaaatatt aaatatttat taattaaaga tattaaaaca 36780
ggaaaaactg ctttcattca ataaaaactc tagtaattct tcctaactcc agaattgtat 36840
gtatatgttt tacctattaa ataatcagaa atagaaacaa attagacaag atacgtgaat 36900
aaccttaaaa attttccata atatttgact taggaacttt agttttatga atgtactcta 36960

aggaataccc tgaaaaggac aaaatctata cacaaagatt atacacaaat ttattataga 37020
attatgtact aaaaaaccct aaatgaccag ccttgagtat atggttaaat atataagtta 37080
taagcctttg ggttttgaga gttttagata acacaagaag gtatttatga tattatatta 37140
aatcaagata aaagtcacaa tgtaatataa agatatacaa tcaatgatgt aaaagtacat 37200
agaagaaaac tctaatgaaa atatgccaaa ttgttaacta tgatgacttt taggttacgg 37260
aattatggga ccatttttgt ttccttatgc aattgaaaag tcataaatga gcatgcatca 37320
ctttttcatc agaaaaataa atgagttaca tattttttta aagggaagat attgtaaagg 37380
aatatttgtt gacagaatgc attaaaaaac ctaggtaatt tccttatttc cctgggattc 37440
tgagaatttt tttactcttg ggaatatctt tccttgctct tagttatttt catatgttag 37500
tcttgttcct ggggataaaa aatggtaagt ttgctatttg agctctgctc gaacatatgg 37560
agtagttctc tcttacatat acagaattgg tttgacattc aaggtctttc agagtcaaat 37620
cccaattgat cttcccaatt tcacttccct tagtttccta cgtatatttt ctacacaaat 37680
caaaaacctt ttcagacttc ctatttcttg tacatgtcca taacctcccc tttctgaaac 37740
tctacttgtt cctggggaca gaatgttcac agagcatgcg ttaaacatca taaagtggat 37800
cgggagcctc ttttaacata aggctgtgtg attttgacaa ggcagttagc tcaccctcta 37860
gatctcagat ggaacacctg taaaacaagg gactatgttg tagatggttg ccaacattcc 37920
ttgcagtgct acagctgagt gaaccttgtt ccctcatggc cctaggtgtg ctcattcatc 37980
tgcctgcacc ttgtctcagg ttcccactgc atcttaagtg gcccctgccc accccactgt 38040
caaatttgac ccttcctagc tcatgtacca tttcctttat aatgccgtcc tcagtgattc 38100
tgattaacag ggacttgcat gtatgatttt tttttttttt ttgccactca tacaggctgc 38160
cttttatcaa aactatggca catgtcaatg atataacaga taatatcatt tgaataaaaa 38220
gtcaactcca tggggaggaa aaaaacctac ttaagtgagc tttagagata aaggtgaaca 38280
cttctctaaa tcctttgttt atggtctgtt aatcaataaa gtccaatctg tgatccccag 38340
atacataaga ggagaaggat atgtagacta tttatgatat ttctgaaaat cctaagaaaa 38400
atcacacaca ggcccacagt aagtctatgt ttctttattt tgagtcagag gatttctttt 38460
ttccattttt aaccagtata aattggttta aacttcatct cttgaataac tcctaacatg 38520
accagagccc ttttcttcct tcaaatttcc acaaatgcat tgctcccctt ttttcttttc 38580
accacccata tgtccatgtt ggacaaaaac catgaaggaa ggaggttgag aaactgtgta 38640
ccaaaacgcc aagagcatct ttgccatgtt ccagcacaaa gggcactgtt agaaggaggt 38700

gatcagagtc atcttctgac tcaacctcac aagaggagtc aggttagatg aacagttccc 38760
aactatttac tctcctctct ctctcattct ctctctcgca ttctttttcc ttctgttttt 38820
tattagcagc acaaaagatt ttgaagtgct taagggcaga aagcttgcct tgttcatcta 38880
tgcagctctt gctgggacca agtatggtgc taaacacacg gtagacattc agtaagtatt 38940
tgtggaatga atgaatgagc aaatgaagga caggtgtggc ttcgatggca atgggtcagt 39000
gtgaactccg gaaagagtat gcagagttat agtcaaagtg gatgatggat cattgctctg 39060
acttactagt tatgtaatct tgagtaaacc cctttatcac tgtgggatta atttccccag 39120
ctgttgaaaa aatgtgtgtg ggggaaaggg ggagggttag tgattaaaaa tgtgttccat 39180
aaggagcatg tatgtaaaat catctggcac atggttgcag ctgaatagct ggcatttatg 39240
tagcacttta ttttttctgc attttttaaa aattaaaaac ctgaaaattg ttaagttgta 39300
ctaacctcaa atctcaccac ttaagaatta tataaaataa gaagtaaaat ttctcttccc 39360
agccaacttt tctaattccc cagagataac tacttttctt aacatttaag tttgatgttt 39420
atccttcttg acatttttct aaagtttcat atatatatat tttttataat tatttaatat 39480
atatgtaaac tcatgaatat atatatgcaa actcataaat atatatatat atatctccat 39540
gcaactttgg atggagatat atatacacac atatcatttt caaaatggaa ctattcatat 39600
atgtctatct atctatctat ctatcaatca tctatctatc tccaagtcaa ttatttttga 39660
atgatggcag aatcatttgt tgtttgaata taccatattt tgctgccagt cacccagttc 39720
cagtgttttg tgattacaaa caataatgca agggtcttcc ttgtacatct atatctgtaa 39780
gcttttgtac agtacttact ttagtaataa ggcacttgaa cacttattat tctctttact 39840
cataaccacc acaagaagaa aggacagaaa ttgttattct gtgttacaga tgaagaaaaa 39900
ggagagctaa agggggcaac tggcttggcc agagacccac acagatagac tatggcagac 39960
cccgtgtttc ccagtggctt gaatttttgt ccagagctct ttttgtcaca ctgcaataaa 40020
gcacaagtca acaggaaacc tttacaacca ggcagagaag cttggatcag ggagcaggca 40080
gtcacactaa attgtagaaa agaacaaaca cacacacaca aacaagaagc tcgaggccaa 40140
acacatttaa aggatattat ttggaagaaa tgcactggtg gtgagtgagg gaccagcata 40200
agaagagctg tcctttaact agttaccatg ttttatgcaa tgttctcaat atgatatcca 40260
aatgattagg atggcttaga ttgcataaat gaggcaggag aatgacaaag gacaccccca 40320
caaccccaac aatgacacta cccctctccc tcaagggaag tgtctaggga aggccagtca 40380

gcatctggtt aggcttggcc ttaattgtca tcttcctcaa ttatctgcag gaaatattag 40440
ctgttattaa aatttgcaga tgcagtcaat tgtcagagtc actggagacc aagaattatt 40500
actcagagca ttggagagag gctggaaaga aaaagaacaa ttcagcctgg aaaaatgtaa 40560
actaatggat aaagtgaggt agggggagtg ggggaagaat ttagagggat aaggccagta 40620
gtagggtgag gcagtgaagc actcccctgg agtacaaact ttaaggggat gccaaaaact 40680
cagctatcaa gataaataaa attttaattc aatattgaaa aaaatcaagt tggcaaatga 40740
cagcctgtgg gccagcagcc tgtttgtaaa tacaatttta ttggaaagcc actggaagta 40800
aaggtactgt ggcaggggag atccaggcta attctttgat gtgaaaaccc tggttaggct 40860
tgtgaatgtt agacacatta gaccttagct tgtgattgat gggaaaaata tttctgaaga 40920
aaaggaataa tggatctgac tgcttttatg ggtttcctgg ggttactgta acaagtgatc 40980
acaaactggg tggcttagga caacagatat tgattgtctc acagttctgg aggctagaag 41040
tccaagatca aggtgtcagc agggccatat tccctctgaa ggttctagag aagcatcttt 41100
ctttgacatc taatttttga tggctcttgc aatcactcca atctctgcct tgatgtttac 41160
atactcttca ttgtgtatgt ctctctgtct ttgtgtccgt ctctctgtct ttgtgtccac 41220
atctttttct cctttccctt agatcctagt cattgggtga catcccaatc cagataacct 41280
cattttaact tggtaacatc tgcaaagacc ctagtttcaa ataagattac attcctaaga 41340
ttacctatga atataacctt aactctttgg ttccttattt ggaaataggg tctttcttgg 41400
acacgattca acccaccaca ctgttaaaga tatctttcta ggacacaatt caacccacca 41460
tgctattaaa taggtaggga tgtggagaga tttgacttag gcgcagcaga aacattatcc 41520
attcacagaa atctggcagc ggagtcaggg gcagactcct gacatgattt aaatacctaa 41580
gaaaatattt aaggaaaagt actttagttc ctttggggcg caaatattat aacctttgct 41640
catgtcacat ggtgacaaat acatgcagga tttttaaggg tagttctgat ttcaaacatg 41700
caaacaatag tgactggaaa tatttttact aaattttata ttccttttgt tactatttta 41760
ttcttctcaa tgtaagcaac ttattaaatt attactataa ccgaatataa caacttttag 41820
atatttttca caaaaattca ccaaccgggg aaaaacgttg tttgtaaaat gcagtattct 41880
gatttttaat ttatgaaata tggctgttat gcttctgaag cctgttcaaa ggctatgaaa 41940
caatggagat gatcgaatac cattagaatt ttaagtaagt tgaaatgtaa atgggaaaca 42000
aatctatttt ctcccatttt ccaaacacat cacagtcaag tccaaaaatt tgaatgttac 42060
atggcttttt agtgaacact tttgttataa acatgcgtgt gtaggtatac ttaattgtat 42120

cagcaacaat aacattagtt attttaatta ttgtatcacc aatcctaatc actaatcaat 42180
aaatgtatac tcattaatta ataattcaat aataatggct aatatttatg aagtgcttat 42240
gtgctagaca gaagaattga tccctttatg tgaattattt aatttatgaa tacagtaatt 42300
cttccttaga attggtattc tgtttatttg ggcttgaaat gttctacata ttatttttcc 42360
tgcttttggt caggagtcac cagcccattt aaagagagac agctgtatct ttctgtaact 42420
gggctatttt ctctatgagt gcattaatgt taacagagaa gcatggccag caagggctgc 42480
tagcacaaag tgaagggccc tgctgtgatg aacagagaaa ttgggaactt tatcattgct 42540
gctatagaca ttatcaacta ataacacgtt tttaaaattc ctgaaaagag tttgctttct 42600
aaaggttaaa aaggtttaag ttctccctgt aattttgttt atatagatga tttgctttgt 42660
aaacatttgt tttgtttgtt tgtttgtttt tgagacagag tcttgctctg tcgcccaggc 42720
tggagtgcag tggcacgatc tcagctcact gcgacctctg cctcctgggt tcaagcaatt 42780
ctcctgtctc agcctcccca gtagctggga ttacaggcat gcgccaccat gcctggctaa 42840
tttttgtatt tttagtagag acgcttcacc atgttggcca ggctggtctg gaactcctga 42900
cctcgtgatc tgcctgcctt ggcctcccaa ggtactggga ttacaggcgt gagccattgt 42960
gcctggcaca gctctccatt ttaaggcttt gcaattgatt gaatcaggct cgttcaggtt 43020
acctaggatg atcttcctca cgtaaagtca acccatgatg gattctaatc ggtctacaga 43080
acttcttcac agcaacacct agatcagtgt ttcagtaact ggggtctaca gcctagccaa 43140
gctgacagat cagaagagcc atgacaacaa tgataaaaga aactgttgcc ctctagtttc 43200
ctcctgggca ttaataattt agctaatata aacccaatac tttggatacg attgactgaa 43260
ttcagctttg ctgatgcctc tgatttcagt tgctttaacc aaatgcaaac ccctagtgct 43320
tctgttgtct gttgataccc agaggctcat gtgatctgga agggtaagtg gcttctctct 43380
gatttgccag aagaaagtgg ttatgttagc tgaggagcca gaacactaat ttcagtgatt 43440
tgactctggt acaagtggtg ggaggcccga gggtgggatg ctgctttttc ctttggccct 43500
ggtaaacatt ttatgcttgt tatggtggtt gaaatgctgt atttaacctt ttcttcatgt 43560
gccaggaggt tcacagtaaa tgaggggtgt ggtctgtgaa gggaaaagtg gctgtggctt 43620
ggcctctttg taccctgtca gcacacatac acgcatggtc tgtaacatgt gaacctatca 43680
cgtgggaact cattactgag ctgtgagggc tgcccctgag gagctggggg cagtggtcgg 43740
gagaggagaa ggtcgtgggg cacagtagaa atggcaccgg actgggaatg agttcctact 43800

gccaacttct catcagctca gttcctacag tcctctactt actccttcgt tgcccaggac 43860
atttatttaa tgccaggcta agcacaaggc ttccaggatt cagcagtgaa caagacagat 43920
gctacccttg cccttttgga gctgactgtt gagcaatttt aataaatagc aatgatcata 43980
tcattagcat ttcccgaggg ttaccatgtc ccaaatgtgt gagttatgat agaggaagat 44040
ggggttattc taggtgaagg ccgagaagct tctgggaggc agtgactaat aactactgcc 44100
tttttttactt tggagtcatg tcctttctgt gactcccctt tccttctcct ctgtgttctc 44160
tcccttggcc ttgtcaggct gcccctggct ttggttctct cttcctagtc tcctttctaa 44220
ttagcctttc tagcccaata aattaagctc ttgtctggaa tctctagctc cctgatggac 44280
acctcctcgt gaaagccctc agtatctcaa agacacaaca tctgtgaaga actcaactct 44340
tccattgtat tcttccttcc ctttcctgtc tctgataatg ttatcacaat tcgttttgtc 44400
atttaggtca aaaacatagt catctttgac ctaccttccc tatcatacct accagtcact 44460
cagccctacc ctgtctattc ccatgatgtc tctcatctct ccctccattt gtttgtcact 44520
ctcaatggtc agactgtctt ggtcactttt ctgggcaatt gctatagttt aatgcttgca 44580
gttttccctc aactttagtt catcctcttt agagctacca gagcacatac tcttgataca 44640
tcttcttgcg tcactctgtt gctcaaaagc cttcagtgga ccactgctgg gctctgcagc 44700
caggtgcttg tgttctaaac ctggctctgt cacttaccag cttggtaact ggaaaattat 44760
taaccattct gtgcttctat tatttcatct ataaaacaat tgtgtctgtc tcatagagtt 44820
gtgatgggga ttaaatgagt caacatgtaa gtagtgctta taaaaaaaag tctgacataa 44880
ttttagcatt ccgtccaggt agatgctctg gctcctggtc ctccacattt tccctcttgt 44940
gccttgactt tttgcgtttc taacttttcc ctgttatatt tctgaatagt tttttgcaaa 45000
gtagtgcaca aattagtgcc tggatctaca caaactacat catacatgat gctcttgtct 45060
ttcttcatat tcttttaaag actatatgtt atatatttaa acacatatgc atatttatac 45120
taaactaagt atatatatgt atacacatat atacatatag attcatgtat actttattgt 45180
ttcttcatta attgaccaaa tgttttgatg gtgcctctaa ataagtagta cttaggcaaa 45240
tctccactca tagtattgtg gtaaaagatt aacttttgaa aactgcccag agaacagctt 45300
tctatcacat taatcatgag atacagtttt gtttttttcca atgttctggc ggccattctc 45360
atgaggatgg actgtgctga ctgatctcag tgaaagttac cagtgggcat gttctcgggg 45420
cctctctgtc cccatgaagc atccccactc agagacatgg aaaacctgtg agatgagctc 45480
atacaaatga tttctgggat gaaaatagca atgtgagata gtgccaggaa cttcaggttc 45540

acagagtata attaagattt ttcctgaatc attgcacact gtaatttcat cacatcatta 45600
tgtacgttct ttaattatgg gctctaattg cccattttag ttctgtaatt agacataaag 45660
acattaattt tgtaggtata acaatcagct caaatgtact tccaaattct ttcccctacc 45720
tttcaagaaa aattaataca tcattaaaaa gtttgtgcac agaagagcct cttagagggc 45780
agcttttggg agccagttgg tgttctcaaa ggtggggccc cagttcagaa agggtatggg 45840
aacatcaccc tcttatattc cccataaact gtgttgcttc atgtcagata gaccctactg 45900
ggtccagggc agggaagagc tccattaatt cacatagggc cgatttctct taatcgtaaa 45960
attttaaggt gacatcaatg cttaatgagt catgggagag gaatgaacta atgttagggc 46020
aactctggct ttttatttct cttgtgtcct attaaaagat gagaagatga agcattttag 46080
gctcatgggg tttgtatttt tcatttgtgc atgggtagct gattatcatc tgtcattatg 46140
tgcgtgtgtg tgtgtgtatg tgtgtgtgtg tgagagagag agagagagag agagagagag 46200
agagagagac agcaagagag agccagaatc tgttttttaa gaactctcaa tgattcagat 46260
ctccattttt tcccttcacc tacagcactt tagttaaaac aaaacaaaac aaaacaaaac 46320
aaaaaaaccg acgagttcca gcatttcccc ccatggacca tgctatgtcc atttcagccg 46380
acacatttta catgtgtatc tatttgcctc cacttacaaa tccaaggaaa aaaaggcagt 46440
gtggtagagt agaaagagcc ttagtaaggg tcctggcccc tttttcttcc ctttttacac 46500
atactctctc tctaggtaat ctctctattc tcgtccttaa aggccgttta tatgcttttg 46560
tccctccaat ttatatctct agctcaggcc tcatctctga gctccagatt agataacaat 46620
cagaatgctt atttctactt ggatgattta cagataattt aagcttgctg tgttcaaagc 46680
tgggtttact ttcctccaaa tgtattgctt ttctattctt cccaaactca ataaatggca 46740
aaaccatcca ttcaagtact caagctgagt catccttgat tattctttcg acatatgagc 46800
catcagcaag gcttgtttca ccctccagaa cctatcttca atctgtctgt ttccctctcc 46860
ctcaattcag gctgcaatcc caatgcagag tgttcagtaa agcaaaaggg gtggtctgct 46920
ctcaccaaaa gcaatttgca tgctcttaat tcacggatgg attacctagc acttaacaca 46980
gacccagata tatttggtac tcagtaatat acactgaaga atgaatgagt gagttaatga 47040
attaatgaat gaataacaga aactctacct cctaggactt aataatacag agcaggatta 47100
acctgctttc ataagtaatc agtgaacctg gtaaagtgtg acatttatct tttggtgaaa 47160
gcagctcacc ccttttgctt tactgtttca ccagcaatct ctgttttatt tttacctgtt 47220

cttttggaca cacaatttag tcagaaattt gcacctgagt cttacttctg ccacctgcta 47280
gtataaagat caactggtgc agagcactat acagagatta tttattgttt gctacaatgt 47340
tggatataga gtttcctctc taggacctct ttaactggga gggataaaaa tggcacaact 47400
aaattttcat gatttggata agaagagaaa cattgggtgg gcatggtggc tcatgcctgt 47460
aatcccagca ctttgagggg ctgaggcggg tggatcacct gaggtcagga atttgagacc 47520
agcctggcca acatagggaa accctgtctc tactaaaagt acaaaaaaaa actagccggg 47580
catggtggtg ggcacctgta atttcaacta ctagggagcc tgaggcagga gaatgcttgg 47640
actcgggagg tggaggttgc agtgagtcaa gatcatgcta ttgcgttcca gcctggccaa 47700
caagagtgaa actccatctc aaaaaaaaaa agtagaggag aaacattgct tcattcagaa 47760
agtgcttcct aagagattcc catggtccag ggatgatgct ggatggtgga gatgtagaat 47820
aatgtagaga ttacaggtta gcagggatgg ggatgcactt gtaagccaat gagtagaaga 47880
aaggggttct ggcggtggca cagcagcaac cacaggacga aagcaactgc agaggacatg 47940
tgctcaattc tgtgagggga cgatggcggg acatggaagg gtcctgatca tgtcgatgtt 48000
gatggcaagt ctaccagtca aaaggaacag gtgagcaagg gcctgtagcc tggaggtggc 48060
ttggggcttt caaggaacca ctttgttaga cctgctaaag catgtgtttc aaagagaatg 48120
tacccagctc gaagacttgc acattgtagg cacttagcta atatctgttt aataaatgga 48180
taagtgaata taagtggcag aaagacagct gagttgctgg gacaggtgag atcatggaag 48240
gccttgtgta tcatgctagg atcagtaatg ggaagtagag aagtggtcaa tgaagtttta 48300
tttaagcaga aaagagaccc tattgggttg agttttagaa aaatccctgt ggctgcagaa 48360
aagaggatgg tttggtggga atgagactga agtcatgagc acagacagga agggtaactg 48420
aacattttga gagagctgaa agtgtagaca ggaagggtgt tcaaagattg ttggagttca 48480
gagagaagtc ctcggcctgg cctagcagtg tagacagcca aagacagaca agtagtggaa 48540
tgtcacattc tagtaactga ttgaaatgtg tgtgtgttta taggcaagtg agttgaaaac 48600
tgtgtgtgtg cttaagttgg aatgtacatg tgcatatatg aaggtgattt gaagtgtgtg 48660
tgtgtgtgaa tgcagtgtgt ggtagtgcat tggaacgtgt gtgtgtttac atgagtgcaa 48720
gtggacagtc aagaatggtg tctaacactt acctatttgg tctaaaggct cataaaactt 48780
ctgccttact cctggtcaca tgctccaagt cacaagcaga aatgggcaga acagctcggc 48840
cttgtaattc cagcctgact cacactaagc tgtttccaac caagtgaatt aatttctccc 48900
tccttatgcc cacacagcac tttcctctac aacaattgtt acaccactgt tgttattagt 48960

ttatataata ctttaccccc tgctaggctg tgaaatcctg aaggaggaga aaatatgtta 49020
tgtatcattt atctccagag tctagctcat tcaggcacat gctaagttct cagctaatgt 49080
ctgttgaacg aactaaaaga ataggcacac ttttatagct tattaagctc aaacatagac 49140
tccaaagcaa aatctcatca ttgcctcttg cacattttgg aaacaacagc aataatagtt 49200
accatttctt gagaacctac tgtgtgcaag gcacagtgcc aaatactttg ggggagccag 49260
tgattttatg tgcaatgttt ttaaaagtcc ctggagttaa aaaattccta ggaggtaaat 49320
attatccacc ccttccacag atgacggaag tgaggctcac taaggataag taacctgcca 49380
agttgcacag ctagaaagta gagagctgaa acaggaactc aaagcctggg ctccgaacaa 49440
gcacgccctg cagacttccc ttccctgtga tgccccatgc tcagttttct attttcttta 49500
tagtggaaaa gtcaatgccc ttcctatgag aaataaaagg gtttaaagtg gtcaaaagga 49560
aacagtgttt cctctgtgct cacggggttt tggccaatca cagagggtga accaaaaaca 49620
atattatgcg agatgtggcc agtttgggtt gattccagct ttggatgctg tgagctggat 49680
cccaggagca cacgaggaaa aacgataata gcatacttga gatccaagcc catgttgggg 49740
tgaggataga ggttttgctt tcctcaaact attgcatttt tcttttcctc cctaagggtc 49800
cgggcaggat gcttcctacc ccagggggcc acttccaaat gctgctctgt cacttggtgg 49860
aaaacttcaa atttactgag ggtttactgt ggaagactta gtcaagaggg caagtaccct 49920
atatctccag attgctttgc acatgttggc attcaattaa tattttattt gtcaatggat 49980
ttaatagctg attacagttt gcaatgctct actcatggat aaatcagttg atcttctcag 50040
gcttcttatg ccttttccca gtggattttt cattgtcgtg gataacccca ggaaaaagag 50100
gaggcacaga ggaacctctc ttacatgaat aggtgggggg atgcaaggga ggacttctag 50160
cttttcttct cctgactttt ctacattccc caggttgagt gactcgtctc catcttttaa 50220
tttagcatct tggaactgaa cagcacctcc caagcaaccc tcacctggga accaggcaga 50280
aacaagtgaa aatgtggagg aggtgtttat gaatttttgg gcagtcagtg atttcaggga 50340
tccaggaagt tattctactt tctaaaccac ttccccacct gctggatatg aatgtaactc 50400
cctcagcttt cagctttcac atctacttta tatacaattc tatactgcct ctcccatttt 50460
atattattta tacctatgtg ctgggacttg agcaaaggtt tttttatttt ttattttttt 50520
ttatttttta aactttgtac aagggaagcc ataaagaatt tgggcaactt gtagggaaga 50580
gggagagtga atttggtgaa accgaaagtc agtaaaggtc acatgggaga tgctgagctc 50640

tgcagacaga tctttcaacg tgaagcagca gtgaagaaca tggctctgga gtcaaactgg 50700
ctgagctaaa gagttgactt tgttacctac tagttaccta ttggtagtta cctactagtt 50760
atctactagt tacctacctc tgttacctac tagtgtgacc ttgggcaggt taagtaactt 50820
tttgaactta gtttactgat acataaatta gtgataataa taattcctgt ctcataaagt 50880
taccgtgaag attaagtgag gcaatgtatg tggagtgtta aaacagtgca tgacgcagag 50940
taaacacgtt aaaatgagct gttattaata tttggttgct aaaggtctgt cctacctgat 51000
gtgtctatct ctttcaaata catcctctgg gatttagcac tagaaacagt cttgcagaaa 51060
gttagagcag ggaggaaccc tcaagatcat ctaatccaaa gtcctctttt gtaaatgagg 51120
aatcactggc ccaaggtcac ggagtaagtc actgagtaat ccctctggtc tgttgcccag 51180
aatgcattcc ttcccacaaa tctggccatg cttgtcctgg agggcagata gactagcctt 51240
ccccaggtaa ccttgtatct tcccgtaaaa ccgtctgtcc tcacaaagtg gcacaatttc 51300
taaggctccc tcgggcaaca aagtaggttg tgttcagaga ccagtgaggt ggagaaactg 51360
cccagccact gaactccagg ctggacttgg tgagtggcac tcagtggagt cagagtcctg 51420
gagacctgca catcagggag agggtcctga agaggtcttg aaataatttg acatggaaag 51480
agaaaataca aattttcaga gacacagata agcatcatat ttttaagcca agaactcagt 51540
tatatttttg acagtgtggt gtctacagta ctaaggcatg ttcttacaag agtgacactc 51600
actagctctt tttttttttt aactttcatt ttagtttcag tggtatctgt gcaggtttgt 51660
catatagtaa tggggatttg gtgtacaggt aattttgtca cctgggtaat aagcacagta 51720
cccaataggt tttttttttc tgattctctc cctcctccca ctgaccatcc tcaagtaggc 51780
cccagagtct gttgttccac tcctagcatc cgtgtgtttt ccttgtttag ctcctacttg 51840
taagtgagaa catgcgatat ttggttttct gttcctgtgt taatttgctt aggataatga 51900
gatcatgttc tctgacatgg aaagagaaac acacaaattt tcagagacac agatgaccat 51960
catattttta agtcaagaac tcagttatat ttttgacagt atggagcctc cagctccatc 52020
catgttgctg caaaggacat gatctcattc ttttttatgg ctgcatagta ttccatggtg 52080
tgtatgtact atgttttctt tattcagtct accattaatg ggcatttagt ttgattccat 52140
gtctttgcta ttgtggatag tgctgcaatg aacatgtgtc tttatggtag aacaatttat 52200
agccctttgg gtatataccc aataataaga ttgctgggtc aaacggtaat tctcctttta 52260
gttctttgag gaatcaccac actgccttcc acaatcgctg aactaatttg cactcccacc 52320
agcagattat gtgttttttt ttcaaccttg ccaacatctg ttttgttttg ttttttaata 52380

atagctattc tgatgagtat gagatggtat cttattatgg ttttgatttg cagtgctcta 52440
atgattagtg atgtcaagta tttttttcgt atgcctgttg gccgcatgta cgtctttttt 52500
tgaaaatgtc tgttcatgtt ctttgcccac tttttaatgg ggttgtttgg tttttccttg 52560
taaatttgtt caagttcctt atagattctg gacattaagt ctttgtctgt tgttcccctc 52620
ctagtttgca aatattttct cccattctgt aggttattta ctgttaatag tatcttttgc 52680
tgtgcaaaat ctttttaatt agttctcatt ggtaaatttc tgtttttgtt gcaattgctt 52740
ttggtgtctt catcacgaaa tctttgccaa gtcctatgtc cagaatggta tttcctaggt 52800
tatcttccaa agtttctatg gttttaggtt ttacatttaa gtctttaatc catcttgagt 52860
tgattttcgt atgtggtata taagataggg gtccagtttc aatcttctgc atatggctag 52920
ccagctatct caatatcatt tattgaatgg agagtccttt tcctagtgct tggttttgtt 52980
gattttgttg aagatcagat ggttgtaagt atgcagcatt atttctgggc tctctattca 53040
attacattgg tctatatgtc tgtttctgta tcagcaccat gctgttttgg ttactacagc 53100
cctgcagtat actttgaagt taagtcgagt aacatgatac ctcctggttt gttcagctca 53160
tttctttctt gttgaagact tcagttcaat ggccttagtc atctttggaa tggcatttag 53220
gattcagttc aatgacctta gtcatcttta gaatggcatt taggaaaatg gactccttta 53280
ccaaaaaggt gaaaaagaaa ataatataac ccaaccttaa agactatttt tttaagggga 53340
gagggagagg actttattca taatcctatc agctgaacac attccagtct tcttctataa 53400
aaacacaatt ttttataaag ttgaaattat ggagctaaga catttctaag aagacttaat 53460
ttcagttagg gacttgctga ttatctttgg gtaagtcagt ctggcttctt ttctgggagg 53520
agtgttgtgt ccatagagac gtgaacatcg acgtctgctt gtttttgtga ggaagcatgg 53580
tagaatgaag agaacgctgt ctggagccaa aagatccaga ttgaaaccag actttaagcc 53640
tgggctctgc ttcccctttt gctggatgac cttgagacag tgtcttaacc ttcctggatc 53700
tgttttttag cttttataaa atagatcact ggcatatcag cttgttaacg tgcactgtac 53760
tcagccttga cacacgggag aaaggttttc agggacaggg tgaagatttc atctatatga 53820
cctttgactg cagtttggtt ggttgggtta cacaatctac aattgtagat tgattgagag 53880
tcttttgaaa tatgtttttc tttatgctag ttacccaggt cctctcctct ctggtgacag 53940
tggtaattta ccagggcatt gtgtcctctt ttcattaatt aggccactca ggcctacccg 54000
aatcccaaga gaccatttat ggatgatgaa ggtggtaaga tagggttgta gtatgttgga 54060

cctgctgtca caaactacca tatgcagggt gacttataat taagagaaat ttatttttca 54120
caattctgga ggctgggaaa tcctagatca aagtgccagc agatttggtg tctggttagg 54180
cctgcttgct ggttcagaga taactgtttt cttgctgtgt tgtcacatgg cagaaggggg 54240
aagagaactc tctggggtcc ttagtataag ggtactagtc ttatccacaa gagctctgtc 54300
ctcatgatct aatcacccca caaaggcttc aactccaaat gccatgactt tgggggtcag 54360
atttaaacat aggaatttta aggggacaca aacattcagt ttatagcaga gagtgtatgt 54420
tcacagcaca ggaaggttca cagcagaagg gagccattaa ttgtgtatag actttcatgt 54480
ctctgctctg attcattgta taatctgctt cattcaaagg aagaaggtaa attttgggtc 54540
cctcttggaa catttaggtg actcccacca aatacaggat agctttgagt tataacctca 54600
tgaagtccct ggtcatgtct gtgtgtttgc tttggaccag acccaagggc ctgtcctgtg 54660
tcctgcccgg gctcctccca cctctttcct ggagaaaccc acagtgagag tggagggtgg 54720
gggtccatgt ttatcttggt gggcctcact ggagctggac actgctgctc tgtttgccca 54780
ggcttaagta tgcatctcct tttttaactg gccagggact ttcccccagt cacttggcct 54840
gccattgatt tgggactttt ctgcctaaaa cttctaggat gataaggaga gcactattac 54900
ccactgcctg cccaaaccta gtagcaacag cagctagtca tgaccagcat tcatggctgc 54960
aagtactgtc aggccagggg cctgaccagt gaactggaac tttacttctc tcccaagtgg 55020
gattagagtt cactgagtag gtggccctat taaaatatgt atagcctgtg ctaaagttaa 55080
ttctaggggt gagaatgagg cagctctgct tgctggctag cccaggctgg caaagaataa 55140
tgggtgcaag caagaatacc agagccagat tgggttcaaa ttccaattct attgcttcct 55200
aggtatactg acttaggcaa gtacttctat ttctctgtgt ctcagtttct tcacttgtga 55260
aattttcata ttttaaggat tttgaagata tcattttgag gcttaaaaca atgtatttac 55320
ttatgtttaa cacatgtaat tactgatgtt taactttttt ggacttataa aaatgacaat 55380
gtttagcaga gtgctttaaa aacatgaccc aactatgtgc tttctataag aagccccttt 55440
gaatataatg atatagccat gttcaaagaa aaaggatgaa gaaagctata ttatataaac 55500
attaattgaa tgaaagcagg agtagctata ttaatacatg tgaaaatcct ctacaagata 55560
ttagcaaatt gaatacaacc atgtacaaaa agaattacac atcatatcaa attagattta 55620
ttctaagtat gcaagactgg ttcaatattt taaaatcatg tgattatatt aattgatgca 55680
gaaaattatt tgacaaaatt caatattcat tcatgactta aaaaaaactc tcggcaagtt 55740
aggaatagaa taattacctc aattaaaaag tttacagcta acatcatact ttgtgaagaa 55800

ggacttaatg ttttctccct aagacaggga tcaaggcaag aatatctgct cctgccactc 55860
ctattcaaca tagcactgga aattctagcc attatagaaa agaaattaaa ggcatacaga 55920
ttggaaagga ggaaataaaa ctatatttga agatgacatg attgcctaca gaaaaaaata 55980
ccaacacatt tacaaaaaac tctcctagaa ctaataaatt ctcctagaac taataagaga 56040
ccacaggata caggctcaac aagctcaccg gatacaaaaa tcaattgtac gtctacatac 56100
ctataatgaa catgctgaca ccagaataag aaatatagtg ctaattttaa tcactcaaaa 56160
ataataagtg taagtacatt taaatatatt atatttgtaa tgtatattat gtaagtttaa 56220
atataataaa acatacatag gacttgtatg ttgaaggcta caaagactga tgaaagaaat 56280
aaaaagatct aaataaacag agagacatac tatgttcatg gaagtacttg acatagtaaa 56340
gacacaatat actaaagatg gcaattctcc ccaaatcaat atatacattt aatataattc 56400
ctacccaaat ctcagcaacg ttgttttttt taaaatagat agaaagagga ttattctaaa 56460
attttgacta aaggctgagg aaccataata gctagaacaa tttttaaaac aaactaagtg 56520
ggaggaatca gtctacccta tttgaagact tattatatag ctgtagtaat caaggctgtg 56580
tggtattgac agaagtacag acatagatca aaggaatgca ataaagaatt cagaaataga 56640
cccacacaaa tacacctctg tgttgcctta tttttcagaa aagctctaga ctcagagtca 56700
ggaagcccag gtttaggtcc ttggccttgc acaagtgagt tcttccagtt ataaaacttt 56760
gattttctgg gtctaaatca cttaaacttt tctatctcag ttttcttttc tgtttaaggg 56820
aacatccttc cttcttcctt ccaagcctct atttgacaag gatgataaaa tatctttctt 56880
ttttaagcat aaggaattct acaaaccaaa agaattatta cattggttga tgtgaataac 56940
acatctcaac accatgaggc cctgactccg caaacctgaa gtcaaatgcc aagaactttc 57000
atgaaattta ttaatttatt ttttcttccc aactaacacc actaattctt tatttggact 57060
ccttaaggag catgaagaat gggactcatt cttgcagaac tgtttttaga gtttgctact 57120
gctcaaagct cagagtttgg ccatgtgaac ttctgagaag tctgtgaata agagatttta 57180
cagcaattta acagtggttc aaagatagat gctctgtctc ccagtagaat acttgtgctt 57240
gtgtgggcag aaaattctta aaatgattca aaccaaccac ccaaagagga ttatatgtaa 57300
attcatattg tgtgggtcct gggagctttg aaaaccacat acacagagca cagaaacaca 57360
caaacacacc caccaacaca cacacacaca cacacacaca cacacacaca cacacacata 57420
catgaatagc acccttagca ccattgctac cttatgtgat tcctaccatg aaaatctgca 57480

ctttaaaaag caggaaaatt tgaaatctac agatgtggat aacactcaaa cttgaattgg 57540
gacacaagaa cttttccctc tcgggctagt gagatagtac ggcctagtgg ctacaagggt 57600
gaattctgga gataaatttt ctgggtttaa atcacaattt tacctttcat taactgtgat 57660
ttggggcatg ttttttaact attgggtgcc ttggtttgct cttcagtatg attggaataa 57720
ttatatacaa aatatacaat atctatctca cagtattgct gtgaggattt gaagaaatca 57780
tttatactaa gcttttatgc atgaaatata tttagaacag agtgaagcag aagtatctgt 57840
gttctttcat agaatgtatg aatgaaatgg gacagtgggt ggtcactgcc ttaaaatact 57900
ataccatata ttttgtcatg gtgctattgc aacaatactc ctaacattat gtatttcttt 57960
atttgagtca ctgaaataca aatatgttct gagaagagtc agcagagtga tttacaaact 58020
agctttttag actgggaggc acactggaga tccaggttgg gggcctgggt ctaatctcag 58080
ggctctactt ttttaacaag gtatataatt gtggacaagt caactggtgt tcctgaacct 58140
cattcttacc tgcacactga ggaggctgaa ctgggtgatc tcggaggtcc tttctggctg 58200
taatatccta agagactgga tgctccctag gcaaggaaac tgttttatcg acgtacatac 58260
tgtggaagct tactaatgca agtctttatt aagtagtttt tctgtagcag ccgcttttct 58320
aagccctttg acctatttaa cccttgcagc aatcctatga agaaggattt atcattatcc 58380
ccattttaca gataaggaaa ctgaagcaga cagaggctaa gtgatttgcc caaagaccat 58440
atggctatta agtggcaggg ctgagattca taatccagta agttgaggta cgcacaccag 58500
gcattctatt cagcatcaga cagtgctagg caaagaataa ccgaagaaga gggactgtgt 58560
tctattcact ttgttttccc cacagatctg taagagtgcc tggagctttt tataggttct 58620
gaatagatat ttatcattct tgcagtggct tgatcttggc tcactgcaac ctccgcctct 58680
tgggttcaag cgattctcct actttatcct ctggagtagc tgggattaca ggtgcccggc 58740
accatgccca gctaattttt gtattttttt tttaaatttt attttaagtt ctgggataca 58800
tgtgcagaac gtgcaggttt gttgcatagg catgcatgtg ccatagtggt ttgctgcact 58860
tatcaaccca ccatctaggt tttaagccct gcatgcatta ggtatttgtc ctaatgctct 58920
ccttcccctt gccccccacc ccgtgacagg ccctggtgtg tgatatccca ctccctgtgt 58980
ccatgtgtcc tcattgttca actcccactt atgagtgaga atatgcggtg tttggttttg 59040
tgttcctgtg ttagtttgct gagaatgatg gcttccagct tcatccatgc ccctgcaaag 59100
gatatgaact cattgttttt tatggctgcg tagtattcca tggtatatat gtgccacatt 59160
ttctttatcc agtctatcat tgatggacat ttgggttgat tccaagtttt tgctattgta 59220

aatagtgctg cagtaaacat acgtgtgcat gtgcctttat agtagaatga tttataatcc 59280
tttcgatata tacccagtaa tgggaatttt ttgtattttt agtagaaatg gggttttgcc 59340
atattggcca ggctggtctt gaactcctaa cctcaggtta tctgcccgcc ttggcctccc 59400
aaagtactgg gattacaggc atgagccacc acgcctggcc tatcattctt gtttttgttg 59460
acgacaaaaa gttgttggat taaattgctt tcagccttct gggttgctaa gtgttgcaga 59520
gttggctgtg ttaaatttca ttttcttttg ttaatgttac ccacacaagg tgaaggtttt 59580
ggttggggct caggagggct cctgactggg gagaaagtgg gaagatccag agcaaggaga 59640
agacaatgag gctgccaggc tttgggctgc tctggcctct ctgctgcctg ccactgtctt 59700
catgtccagc ctcgctgtgt ttctataaag gtggagttgt acctctctga tttcccctaa 59760
aggtggcctg gatgtggtag aagaaagagg aggcaaggca ttacctgcac ctggaatact 59820
gatgagtggt ttgctctttc ttgtatgccc agtggcctgg tcttaagcct aggaataaat 59880
gtagtcacca tggccataaa acggccccac tgctgggctt gctttccctg gcctctgtga 59940
gttccatgct cattatatgg actccccata gcaacgttga gagcttgctt atcatttcca 60000
gaagtgacca tggcagatta ggaatatatg tttattttga agtgggtttt tttcttttta 60060
aattttacac attgttggat gaacataaca tcaagggctg ctttcccaac tgtcttagga 60120
aaagagctct aatgttggag ccagtagccc tgcgtgttct catcccagtt ttgattataa 60180
ctgactataa tactgtaatg acactctgac ctgtgttctt ttatgactga gatttccggt 60240
ggggatggag gaggtgtctg atgtacagaa ccagggggat gctgcctgag ccaggttggg 60300
gacggagaga agaggctgaa tttcaagaca ctgacatcat cagtactggc cctagtgcag 60360
ttacatacct ggtcctgcaa caaataatga gtatttaggg cacatatgcc aagcattaat 60420
ggggtcacga ggtggatcgt tcttttgggt gcccttttat atgccgctgc aatcagagaa 60480
gcaggacacc atgtaggtga gcatctgctg ttttcacagc tatttgaagc tgcaacccag 60540
tgtactaaaa gtttccaggc tgactcagtc caaaggtgac tgttttggaa actttgaatg 60600
agattggttc ggcaacttca gtgttatgta agtaaaagga tgagagcagt tgaaagagcc 60660
actgctctgg acatgttcta agtggcttct accaatggga atacgtgagc gtgcattaag 60720
aatgtagcaa gtgagactga catgtctcca gtacaagcac agcacgtaca aggacacaac 60780
ttcatgtttg gttcaatgtg agacttctct gtggctcctt tctcttcatt tgcacagata 60840
tctgtaccac ccagtataag tggtatacta atcgtagcta gcctgtgctg catgcctgct 60900

attttctggg taattcccat gtgttttaca caggttaact ctacttcctc tcatggaaac 60960
tacttgttgt cgaaataatg gttatatttt accaatatgg caactgaggc acagagatgt 61020
taagtgtttc agcccaggta aataagtggt tcaatcagga tttgaactca agcagcacct 61080
accttggagc ctaagctctt aaccacagca cttcactgaa cctataaaat acatgtttat 61140
tggttttaag ctctgctagt tgaagaacat ttatacatgt ctctattaaa aattcacttg 61200
tgtgtgggtt atgaattcac ccataaagaa gactgccatg acgcacggag gagggatgtc 61260
tgtggagctt caggagacgt gataatccac aaactgtcct gggttgaact tagctattat 61320
tctgccagga aactgtctgg agaaggtcca ggaaagtacc aagaagtaaa gaagtacctg 61380
agagtgctca gctcccagtg tgtcccttgg cacctgccag tcacagcttg gttggttctg 61440
gctgtggggc cggccttcct gagacccttg tcagggggct cataccctgg tgctctgttt 61500
tcactgatga tgaagcctca gagcccttgg tcccctcatg atgttctgcg ggaaaattgt 61560
gcacactttt gttgtgtgac ttaggttctt taactcaggc tggttgacat ggagcttggt 61620
tccagtgatc agtgctggct ttagatatga tttgcgcatc tctgcaggct agtgtcacgc 61680
tgcactgtgg atctggactg tggctcgggt gccagttgca acatagtctt caaggatagc 61740
caatggcgag acaacagcac tctgtcatgc agctgaagtt atggaaacac atgatgtcac 61800
atgaatgtct attgtgtgag gtgatggtta atacctgtgt ttccaagaaa gtgccaggta 61860
ctggctagtc ctcaccacaa ccctatatgt ggtaagggca gttattaagc ttattttata 61920
actgaagaag cattagcacc aacagagaag ataacttgcc caaggtcata catgaaaacc 61980
aatggagaaa ttaaggtttc aacctaggca gttaaggcct aaagtctgtg ctcgtgacca 62040
ccaagctatc tcaccggtcc cctgtcaggc tttttagggg ataaagtaca gagtaaagta 62100
gaactttcct caaagactgt cttattggtc tcacttattt tttttttttg ttagtctttg 62160
ggtttacttt aaattgatgg gctgctggaa aaaatgtaaa ttgtcattta aaaagaaggc 62220
atcaaggttt tgtcttaggt taatttttca aagggcttac caagaacaca gaagacagaa 62280
tgccagctgg gtgccgtggc tcacgcctgt aatcccagca ctttgggagg ccaaggcagg 62340
cagatcactt gaggccagga gtttgagacc agcctggcca acatggtgaa accccatctc 62400
tacttaaaaa aaaaaaaaaa aaaaaaaaaa tagccaggtg tggtggtgca tgcctgtaac 62460
cctagctgct tgggaggctg agttgggagg atcccttgaa cctgggaggc agagattgca 62520
gtaagctgag atcatgacac tgcactatag cctgggtgac aaggcaagac aatgtcaaaa 62580
aaaaaaaaaa agacagaatg ctgagcaatt aagaactact ttatttagtc ctgtcttatc 62640

atttgtggcc tttgtgtatt gagcacgtcc tgagagctgg gcactttcta aatactttac 62700
atgtgtttcc tcactttatg cttgtaactg ctttctggaa gcaggactgt gattattccc 62760
attttatgga agaaggaaat gaggttcacg aatattacat aatctgctca tagtcacaaa 62820
gctagcaggt ggtggggctg gcgtagaaac ccaggcagtc tggctccaga gtccatgttc 62880
ttaattaacc actcctctag ggtggaaagc tcactggttt tggggttagg acactcatgc 62940
cagctctgcc aaaatgtcaa catgtgtgac actggccaag tcatatctcc tcttgggttc 63000
tcattcattt atttactgat tcattcagca gctctttcca gcatacctac catgtgctag 63060
gcattgttag tactagatac ataacgggtg ataagataga tttcctcgat gcttctgtag 63120
agtttacaga tggattttca gtttcctcat ctgtaaaaga tgagaattaa aatatatgaa 63180
tgtttctcat cctttacaaa acccacacat cttttataa acataaaaag ttatgccttc 63240
cctaatgtta tttgaaattt ctgagtaaat aaccatgact aaaaataaat taaagcaatt 63300
ataatagtga gtaatttttg tttgaatcac acaagccctc atgcctgttc ctgaggagat 63360
tctcctgcgc tggccctctg agccctgggc attctttcgg ttaagaatca tgggaatagt 63420
attcttgctc taaaaacgga cctacagtta tattttttaa ttgctttgaa atgctggagt 63480
tgtgtctatt taatttgtat tcattctttc accaagtttt ggctcttggg tttgtatggc 63540
agataaaact ttactcattc cttttactga aaacctcttt cccccccagg gtatagtcgt 63600
gagtcacgag ctctctcacc aactgacatt tccccatcct gtttttggag ggaacaacaa 63660
aggacatgcc tggtgcctga cacagcgggc ccacaggctg ctcctttagt ggccttgtga 63720
gttcgatgag cttttggctc taggaagtgc ctgggctgct attactctgt gaatctcatg 63780
agattcacag acctcttagg agcaccagcc aggttgtaaa gatctcttga ggactgggct 63840
ctggaaggac tggccactcc aacaccctgc cttgccctta attgtgcatt gcctggtgtt 63900
ctttaattgc ctcacaaatg cccttgaaaa ctctcaggga tggcctcact gtgcatgaca 63960
aaggaatgca acctactgtc tggttgtact gaaggagcag agagcaggat tctcataatg 64020
attgttcaaa gaatttattt atgtggagtc ctaaaaggag tgataggtgt gaaaaaatgc 64080
aatttaattc aacaaacatt gatgcaaacg ctgtgcttgg tgacagcaat atagagatga 64140
ataagaccag tgtctagtga cgtgctggca agtacaactg gctctgtggg gaggcggtac 64200
cctgatttgt accttttgct gatttccatt gagtaaatac tcttgccatg tttgatttca 64260
agctaccaac atggtgtcaa ttggctcaca gaagccctga aaatttaata atcagctctt 64320

atgagcctgt acaaactgaa cttgcattct gctaccaata tctacttttg aagttgtcag 64380
tttagattgg aagatacata aattgccatg cagtctgata aagacagaaa tagaagtatg 64440
tagaaggtag aaaggtatca caagggacac tggttcaatt ctgcggtggg catatccaca 64500
tggtaggctg caaacaaaga caggcaaggc ctgtaggtgg gtggagacag agaccttcag 64560
agcatcctca gtgccttcag gtcatgggga aatgccttgg cctgccttaa ggtcccgtgc 64620
agtctagcct caccttacct ggctgaacat tctcccacat ttactgacac aaaccttctg 64680
ctctagtgag tccagcccag tgttttcagt cttgagagca catccctgcc ttggctgaca 64740
cccacctcct cccaccaccg tttagaaagt gccttccctc cacctccact tccctgctct 64800
actcatcctt ctttatgaat gcttcctaca acagctccaa gccacagaaa gcactgacag 64860
aaagggaaag attgatcacg tgaattccat taaagtgaag aatttatgtt tatcaaaagt 64920
cactgttaag aaaggaaaag gcaaacaatg gagtcagagg aactgtttgg aacacttatg 64980
accaatcgag gatttgtatt caggacatgt aaggacctct cacaaatcaa taagaaaaag 65040
acaagaaaag ggaaaagaga ccagaacagg aacttcacaa gaatggatat ccaaatgaca 65100
tgaaaagaag cttgccttca ctccaccttt acctcagtca ctagggaaat gtgatgccac 65160
tacataccca ctaaagtggc tgaaaccaaa tagacttgta ttaccaagca ttgttgaaga 65220
cttgaaagag tgggaaataa tgtatactgc tggtaagaat cagacatggt ataacctatt 65280
tgaaaaacag tttggcaata tttattaaaa tggaacattt gcatactcta tgacccagca 65340
tataatctga ccatactcta tgacccagca tatgatatga cctagctata atccaatgaa 65400
aatgtatatc catatgttta cctaaaagta tgtactggaa tgttgataca gcatcattag 65460
ttatggctcc aaattggaaa tacccagaca ttcatcaata gtaaaaaata aattataggc 65520
tatttgtgaa gtggaatctt tttaaactat gaaaaagcat gacatactgc tccatagaac 65580
tacatggatg aacctcacaa acctaatttt gagcgaaagg ggccaaacaa aaaagaaaac 65640
aaatggagtt attatattta tataaagttc aaaaacagac aaaactatat gaggttagac 65700
atcagtttac ctgatacctt tggggaagag caagaggtta gtgactggga gggctgtgtt 65760
gggggcttct gagttgctga ggttgttata tttctggccc tgagtggtga taacacaggt 65820
atgttcactt tgtcattgag ctttacgtgt gcaatttgtg tacttttctg tatatcttac 65880
atttttatta gctaaaaagc aaaggagctt ctgcatgaaa gtattagaac tagtgctgga 65940
aagtgaggga gtgtttctga accgcctttg tgtttttctt ttgacgtttt ctatctttat 66000
gagtttattc atcaaacaaa gacattttga gagtgacaat gtgccgggaa ctattttagg 66060

tactgagagt gaaacaaaat agaaacaact gctgctttca tggagcttac attctcaagg 66120
ggacagcaag gtccttgaac agacaaatgt ataacaagtc aggggataag aaatactctg 66180
aagtggtatt aggggataag aggataaaga ataatggagc tgggctgagg gtttgggcac 66240
tgactaggtg ttgttttata tggggtggtc gaagaggacc tctgaccagg cagccttgga 66300
gccagagcct ggatgaagtg agtaagggag gctgacaggt atgtgaagaa cattccaggc 66360
agagagggca gtaagcgcaa aggccctgca ttgggatcct tggcctccga agcaggaaac 66420
tcagcatcat tcttggcttc tctctctctt cttcaccttc atatttgaat caaatcatga 66480
tccatttgat ttcatagcta aatctctcat agtttgcaac ctctgcctcc caggctcaag 66540
cgcttcttct gcctcagcct ctcgagtagc tgggattaca ggaacatgcc agcacaccca 66600
gctaattttt gtatttttag tagagatggg gtttcaccat gttggccagg gtggtctcaa 66660
actcctggtt tgtctccatc ccctccattg ttgcctattt gggtaatagt ttcttaactc 66720
tgccttcccg tttccatttt tttctcttct gattttaagt cactgactgg atatgccact 66780
cctctgccta aaagccctcc agtggctctg ctttgctctc agagtaaatg ccctacaagg 66840
ttttggtttt cctcactggt ctcatatctt tctgctttgc cgtacactcc gtgcttgagc 66900
cacaccaagg cgattccatg tttcccacat tccctctgag gaaaggccct ttagtctctg 66960
ggtctttctt tatgagggtg catctacctg tggagttctt tcccccacct cctcatttta 67020
tgttttgcac atcccttaga tctcagctta gatgttgttt agtctgggaa gctgttcctg 67080
tcccttaaac cttggccaag tccctgtgtt ctcctatcgt tgccccgtca tatcattttg 67140
tcattgattt ttcttttttc cagcctccca tgaaactcta agacacatgt gggaagtaac 67200
tatgttactt attcatagtg cttagcaaag tttctgatgt ttagaaattt ctcaataaat 67260
attaaaaaaa gaatgcatgt tgagctacat ttctgtgata tgtcaaaaat ctagaagtat 67320
ttttattagt aactgataga aatatctttc agtcaaaaca ttattcatca actcacaaaa 67380
aagcagacta gctttgtgtt tgagttttgt ttttgttttt cctggtaatt ttgtgttcca 67440
taatttctca gacacagaaa cattatcttt caaattttaa taccacttta tttgactcaa 67500
tcacatgtaa aatttttgtt cagagaattt tgaatgtatc cgtgaaaaat attcaaatgt 67560
cgaaatccaa atgtcttgct ttggctgtgg agcaatatca ttactgtgac ttagttctag 67620
aatttagact taattgctaa aaaaaaaatt ttgaattcaa ctttattaat tttgtcaagt 67680
ttttgatgtg ctaggtggac attctttcca atttattaca gtcccttcat atagacacaa 67740

aagcttttaa cacgatggga aagctggtga gacaagtcag aaaataaagc agctcttttt 67800
tctttttttg ttgagacaga gactagttct gttgcccagg ctagagtgca gtggtgcgat 67860
cttggctcac tgtagcctcc acctcccagg ttcaagcaat tcttgtgtct caggcaccga 67920
agtagctggg attacaggca gtagccacca cgccggctaa tttttgtatt tttagtagag 67980
atggggtttc accatgttgg ccaggctggt ctcaaacttc tgagctcaag ttatccacct 68040
gcctcggcct cccaaagtgc tgggattaca ggcatgagcc accgtcccca gccagaaagc 68100
cgtgattttt aagctgatgc tttgtaccat cagtgtgagg agtgatgcta ttgatccctg 68160
ctgggcatga gcattagaag aaagttgagc agggacttaa aatggggatg tggagcactg 68220
tgccaatgtc tttcaaatgg gcttttaaaa attattttaa caggattatt tggagaatca 68280
taaatgtttg agcctcagat acactcatag ctaccattta ctgagtactt gctatatgcc 68340
aggcactata aaaactgctt taaatagaca atcttgttta attgtaataa cagtcatatg 68400
ggattgttac catcattccc atttaacaga tgagaaaatt aaggctcaga gaggttgagt 68460
ggtttgccct aggtcatata gcaggtcaaa gctaatgctg aactatgaac acagttcctc 68520
tgactgaaaa tgtgcaaggc tactaacgac taggtcctac tggcttatgt attgaaaagt 68580
agatcaatga actcattctg gagctaaatg ttgtctatta ctgtggatta tccatgacat 68640
aacccactca ttatattcca gtattggtta aaaggtagga gagaagtgat agtaaatttc 68700
atgggttcgg catatcttct gaccgacaga agacatggaa atgttttaaa tgtttctttt 68760
cctttttttt tttttttaaa aaaaaaacaa aaacaaataa cactaaaagt ttcaccaaac 68820
aagggttcat aaaaggatcg tctctaaatt gtgagtgtgg tgagtgtgca tgctctgagc 68880
aaaaagagcc tcccctttct gagggaattg tgaccctgga aaggaggaga gcaggaacag 68940
ctggccatct ccccctgaaa ggccagggcc cccctctcag gcctctgggc tccagctctg 69000
gctgagggtg tgtttgtttg gctgtgactc tttcccttct gtttccctct cggtgtcata 69060
tttcatgagc tccaggctcc tgcctgtttc agcttgagcc tctggctttc atcgctgcct 69120
ccttggccaa agctgagtga gggcttgaaa aaaacactga ttggatgcac atttagtgga 69180
ggcgattgct ggaccagcac ctcaccaatc cccagcccca aatgcaactg acagctacac 69240
cctcctggct ggagcctaaa ccagaggccc ctttcctccc tcctctccac ccataccctt 69300
gctgtttgcc caactcttcc tgctgaaatc agaggcagct tgtgcctccc agccttcttc 69360
cctgcccccg ctgtctgccc agccaccagc ttcccctggg ctggaaagac ccccgcttgt 69420
ctgactgtgc taatcctctg tttgaggttc cagaacttcc caattgttgg gcctgggttc 69480

ctgtacttag ctgcccagag tccacttctc tgctgagatt tctattatac aagagattca 69540
accagaaccc tctccgtggg cctccatgcc tctttttgct gaattcccca ttctcattct 69600
gcgtaggggg tgggagttgg tgggggtaca tgcaccttga gtctaatcat gatcttactg 69660
gggagagctc agtaggaaag taataacata ttcatttaaa caactttttc tctgttaatc 69720
tttttattga acttcataga attaatggat aaagactatt ggttagagtg cgtctttctt 69780
agacgagatg gagttagaga cacttaaaaa aaaataaggc ttgccactgc aggagtgcac 69840
agagcacctc aactattgga gcccaggtga tctcttcagt gggtcataaa atctgagtca 69900
taagaacctc atggcaaaga aaacttgaaa ctgcaaagtc tcacatagtc atttgctgaa 69960
aactgaagtg tcccaacaaa tgtcgggtta gacctacttt gagaaacaca ttccaatatg 70020
agatcgaacc tgacaatttt gcatgctaac ctggactgtt tcttattaga gcaaagggga 70080
tgggcttttt gtttgctttg ggtgtatttg tttttgctgc caaagtgttg taatgtcatg 70140
attatttaat atgtatgagg cctagttgtg tactagggag agtcaaaata catttactac 70200
gaaatgagac cagctccagt tgtttaaaca gctccagttg taataataag agctaagact 70260
tgatgccaag cactgtttct aggtattaac ttattgagtc ctcatgatta ctcctcacga 70320
tggtcctctt ggaaaggatc ctatttttct atgagaaaac tgaggcttga gtgttaagga 70380
acttgcttaa actcacacag ttagtaaagg tcagctttta gatttaaagc caggcaccct 70440
cattctagag cttgtggctc aaccactgag ctatactgct tgttagggtt gttggtggag 70500
tgatagtaga agtagatggg ctagagtttt tcatttctaa ccctaaaaat ctatagctat 70560
taacactgaa gacagcaact gccaattaaa taaatcctca acaagactga gaagagaatc 70620
tgctaatctg ttggtcacta atgtctgcag tttatctgct tatctatctg cagcaaaact 70680
gtcttagaac actttgctga atgaaattga acatggaaag ctgactgaag ggaaaacaga 70740
gaaggtggga tagttgagaa gggagtaaga tcagacaaat ggcaatgctc tgtgggatac 70800
tgcgtccaag cagaacagtc aagggattca gttctcagaa gctgttcagg tagacgccct 70860
ccttaccaga gtcggttagc ctcttgctgt atgcacactt gaggctgctc aaagcgcgag 70920
ggaggcatgg tctgtgctca ttccaggcat ccaaagcacc agggaggcat gatctgtgtt 70980
cattccaggt gtcttatctc gctgccactt ccaggcctgc acattatagg caatatattt 71040
actacactct cccttctcct ttatttttta cttctggatg tgccatggtg aatatttttc 71100
aggcaaatgg ggggaatcgt ttccatttca agctgggttg aaatctagca ctatgattag 71160

ggttgtgcta acctctgcct gcctctcccc taactcctgc tgtgtatagg tgggcatggc 71220
tcaagcatcc tcagggctgg gagaaggttg catcaggaat gtttctacca acagaagctg 71280
gccagtggga ggcttgtagg cactttgtaa tttaccattc tcttcttgaa ctatgcagca 71340
gtctgagggg ctgtacagag gattcttccc agtcattccc attgctagct ttggaggcct 71400
tctcccacaa ttcatagcct cctagtaatg tgctttattc ctaaagccca gtccttgggg 71460
tttttgaaga aaaggaaggc agatcagcat gccttcccta gtctgaaatt accctgtttg 71520
actcatatag tgtggagctt tataatctgt aatccaaaat tgggattgtc tatactataa 71580
ggatattctc tgggacttac gcttatttaa ccaaggttgt ctgcaatggg aagctcccat 71640
gacgggagat tcaggagaag gctggcatag gcttctaact tacaccagtg ctccctgatg 71700
cctgtgaatg tggggagagg gcaaggagat tttgagaaca gataagagac atttgaattt 71760
ctcaatgcct tctgaaaacc tgtgtgcaga gttgaattga aacttacctt gttagcatta 71820
taaatctgaa cactttcctt gctaggccac aatgacttaa taatatcgtc taaaggatct 71880
atgttcactc tgcttgcact gatttacatc tcctttttcc aattctgcgt agtctctgga 71940
cacttctact tccaaatatc acctgtttaa ggaatagcac ttaccttgtc ttgtactgtg 72000
aacccaaatt tctgtgaaaa atggactgta gcagttgttc cagtttgaga ttgctgagtg 72060
tcatgaacat tggttttgaa gagtatggag tgttgttgga ggacagctcg aggctaaatg 72120
tggttgtgca atatggtttc tacacactgt ccagcaggtg gttggataca gttatgccat 72180
tctctgcttc ttgtttgttg tcttcaaaac atgaaaaaat aaaagcaaaa tatagaatat 72240
cttcgatgaa ttcagcttct cttaatctca cttttatctc ctttctgtaa gacttagtct 72300
attatttccg aatatgccat caattttatc caagtgaaaa ttcagtttta gttttaaaac 72360
acacagatat aaaaataaga tgcaaagttc tacagtaaaa ttcttagaca taaacactga 72420
gatttttgga ttcacaaata aatagtttag ataacactca gaaagatctt ttcctcctaa 72480
ttagcttcta ttgtcacctc ttcctaagtt gatatgtagc tcaatttgtt aggaaatgga 72540
tttcccatgc atgccctaac tataatcata agcatctgca actctgtttt caaacaaagc 72600
atctgcaact ctattttcaa acaaaggtag ttactcatga gagagcttct ttaaactgca 72660
atggaccaca cagaggtgga tgtctagcac atttacagtg accatgatcc tatctctcac 72720
catttcttct tttcaatatt gcaggattct aaccatatga ctttcccacc cctcacctga 72780
tgtttggcga caatatcctg gcttctactg gcaacaccat cttttgctat ttgggtaaat 72840
gtgctcctta tcagccttgg atcctaccca ggctcagggc cttgtgagtg caatgtctat 72900

tgggctattg tggaccccat gactatgacc cagacactca gttttgtatg ccacatccct 72960
tttcagggag ctaggtcaag gacatcatgc aactcatttg catgccatat tgaagattca 73020
cacttggggg aatattttat tctcaggcag ctaagacctt gaaactccag catagtatgg 73080
agagtgctga gccttcagca tttttagcac atgatttcag ccttggaaat ctgatgtact 73140
tgcagacctc tacacaagag gaggtatttg catagaaaca tgagcatgct ggggctgggt 73200
catcctttta ctacacatgt taaagcatac attgtaacca aaatttggga agcaatgaga 73260
atctcttact ctactattgg gatttggggt cacagcaaga ctttggaaga ggtattactg 73320
agaagatgcc tctatgaggt gttactcaaa gtcattaatt acttacaggg ctctacgaga 73380
atggcatagt tcatgtctta aaaccgctta tagctactgg ttgagacaga aaaggaaact 73440
atgtgctgtg tgattaaacc ggtgtttctt tttttgcatt ctgtcttcta catgtgttgt 73500
ggtgaaatga aactttacca cattcagcag tgctcaggag aaacattgag cagtgttggt 73560
atatatttca atggcattca aaaccctgat aaataattta ggacttgggg agatctgggt 73620
atgggaagaa gaactgaaaa aaaaaaaaag cagcggtaat ttactaatat atgtttgtag 73680
gttaaaacac tttttatagc tgaccgtagt agatagaatc attctttaca actattaagt 73740
ggtattaaat aggttgaccc aactcccttg gaaaagtgat taattttgtc ttccagacct 73800
gacttctgaa gctcaaagtt ggtttaaaca gttcagctgt agtagaggat agcctgatat 73860
cctagttttg ccacagtatc tgggccagat ggcatttgca gtaaatggaa ggtaacaatg 73920
atcagctctg gtatttacgt gtgtaggtga tatggtcatg tgggagtgtt cataggctta 73980
caagccatat ggctcttggc cataaactat tgctaggtaa ggctttgact ctgtatcagt 74040
atgaataaag tagctctttc agctcatgag tatccatttg ctttgtgtca ggctgatgtc 74100
aagttaacct gcttttgcac tctggtgaac agacacatag attattaaaa ctgaaagtga 74160
tctaaaaagt tatctaaacc aatatcctac atgaagaagg atcctcccta cagcctcaca 74220
agtaagatga ttagtcagcc tctacttgaa tatttccagt agtggggagc tcataacctc 74280
acaaagaagc acagcctgtt tttggatggc tctaattttt agggtgttcc ctcttatttt 74340
tgatgaaaat ctgcctctct gtaacttcct tagtcatgac tggagttctg ttgtatgagc 74400
tgaatccttt tatttcagaa tagggcatca catatttgaa gaccaacatt aagtcccagt 74460
gtagttttct tttttctgca tgaaacattc gaaggtcttt cagtaaatcc ccttgttttc 74520
ttggtaggga gtcactcatc accacttttc tcttcccact ggagctgtat taagctcctt 74580

gaaactttgc atcctggaga tacagctttc tagtcacaaa gcccaattta ccttctctga 74640
gaagagtcta gttcttttta ttagttttag ttgtacagtt tgggattctc ctttttctcc 74700
tctttgtctt atttttcttc ccagcttcct cctcacgtgg cgttgttgtg cataaagttc 74760
tgaatcatat ttttcatctt tgttcgttgc aattattgtc actctgtctt ccttcttgga 74820
ttatttcttt cattctcctt agaaaatttt tgaattctcc catcagctgg agaaaaggta 74880
tcttttacct tctcctccat cttatactga ctatgcaagc agcttatact taggtcccat 74940
gcaagcagtg acttcagaaa aaacaagaat accaacaatg tatcatctcc tattgttggc 75000
tcttggtttt gaatagatat tccctaccca actagattca tgtgtgcgac tctgagtata 75060
ggtcattcat cgaatgtctg ttactgctag agtctttgag tatgtagtct gttgatgtga 75120
acctaaatgt aattggtgtt acatgcagtt taaatgtcac cctatttcaa cctcatgatt 75180
ctcaatcttt atgccaatag ctacagtata tttattgtct accataggat ctaattacat 75240
aagctttggt actgctcgtt gtccagaagg atcataatgt tagagaggac atgtcttcct 75300
cacattatga aggactaaga aacagaggct cagggagaat atttggctca tccaagatta 75360
caccattagt tcagcagctg gtcagatgct gtttccgata agccactcag ctagctaact 75420
cccttggctt caatagagaa agtgacttaa actgtggcat ttctggtctt ttatactctc 75480
tctctctctc tgtgtgtgtg tatatacatt ttttcttctt tatcacctag catatttctt 75540
gatatgtaat aagttctcaa taaatgctgg ttaatgaata tttcaagcca tcctttcctt 75600
ttggaagttg ggaacatgtg taaaggaaga agactagatt gagtgataaa gcataaaata 75660
taactaggaa agtcatagct tgggatagag agtatatgca gtttgttcag aagtatataa 75720
ggcattgtgc tatgtgccac gatgcctata cagaacgtat agcctagttc ctgatatcag 75780
tgagtttata atgagataaa cgtattatat ttggcattgt gaaaatgaga taaaaataga 75840
gataagactg aatcatatgg aaaaaattag tttccagttc aagagaatgt tatcaacatc 75900
aatgaggagt tcagagaagg gaaggtaatg aagagcctgc tattatgcta ttgttctcat 75960
cagaagaggg gtgagtgtga gggaggaaga gaggatagat ggaagatgag aagagggtgt 76020
gtgccagagc actgttgtcc atttagtgcc agccctgtgt ggcaaatagg acacaaacct 76080
ccaattggct taagttcctg aggtagaaga gattaagaat gttaaaaagg gaacaaaatt 76140
tattgagcac ctactatagg tgaattcatg aatatttctt gatcacctgt ctgtggaagg 76200
cactgtgtta ggtgttagag ataaagagag atactatctt caccttcaag ggcaggccaa 76260
gcagtgtgct aggaacctca caaaaggagg tgctgaagtc acataagctc cccttatgta 76320

gttattccag ttcctggttt tcacgtctaa tggatttttc cccatagctt ctccccctcc 76380
acccaacccc agcctttcca gagtgtgtct gtcttcccaa aatgagtttg atactttgtg 76440
cgcatgccgg gatccccatg ggattgatga catcatacgc cattggctcc tattgcacac 76500
aagagaggag acagatctgc acattcctgc agttaatgct gagtagaatc ttggcagagg 76560
tggtggcatc cttcatttga ctttagacct tcagcctccc ttttaaagcc ctggctcaca 76620
cagttattga gaagccttat cctgcaagga acctacagct taggaaagag ttgcccatgt 76680
gtctttgcta tcaaacaaaa gatattggca aatcttttat cgatttctta ttctggcaga 76740
ggtaccattt attatggggt agatgggata tagataaagc atctccctgc ccctaatccc 76800
tgccttgctg agataaatgc ctgttccatt ccccaatcac cagaaaatgc cttggagcaa 76860
tggagtctaa cagcctgaga gttgtaaagg ggcagcaaag ccacttctgc aggaagagag 76920
tgtgcatgaa ccttattcat tgtccatgtg tggagttgct tcatgttcat gctcttcacc 76980
ctgatttgac acataccaac atcaaataac tatctactct accccatttg gatactataa 77040
taaaaaaaag atagtccagt taccttataa acaacagaaa tttatttctc attgtcctgg 77100
aggctaggac gtccaagatc aaggtgctgg cagattcagg gtctggttag ggcccactgc 77160
ctggtttatg gatggcatgt tctcactgtg tcctcacatg gtagaagcag tgaggcatct 77220
ctgcggggtc tcttttaaaa ggaccctgaa agagggtgcc accctcatga cctaatcact 77280
tctcaaaggc cctacttcct aatactatca cattgagggt tagaatttca atataggaat 77340
tttgggggaa cacacacatc cagtctatag cactatctga taaggcttct gaatagtcct 77400
gggccatttt acataacggg ggtttactaa ggactctcaa cagcatagct cttgcttcaa 77460
ggggttttcc atgcggggcc tgccagaatc ccctgaagat ttgccctttt gcttttggga 77520
gaatcattga gagaaaaagg acctggctcc tgtgcttttc gcatgctcac ccactccggc 77580
ccttctgatt aatgtttgct gttctgcttt gcagatgcct cccaggaccc agagggagac 77640
tgtagcctca tttctgtgga gacctttggc tggactctcc tggctctccc agaggtgaga 77700
gaccgtggtt cctgtagcct caggcttggc accagctcat ctctggtgga tccaggattc 77760
cagatatcca atctcactac agccagggcc tctttgtccc agtgtgacct tgtgggctgt 77820
ccccctgact gcgtcttaac agcccctgcc attggtgatt ttcattccag ctacgagcca 77880
atggatccct gaaagcgact gtgtgggctt ctctcaccat gtcgctctgg cctcagcctg 77940
gcggtgtgct cctctgtggt ctggatgggg atgatgaact cgggctgccc gttgtttttg 78000

ttttgtgata tttaaagcag ccgaagctct cattactgta atacagtatg tgcctgggct 78060
aaagggggag cagcattcct tctggatcag ggggcaactc tattctgttg tggtacaccc 78120
gctggagtgt ttagaaccac ggctggtaga tttacagtgc cagggctgac aggggccttg 78180
gaactcctcc attttcattc attccagcct cgttgttgga atgaagaggg agctgagccc 78240
attctgggta tgggacgatt gcttaggccc tggggtgatg agtggcagat ctgggactag 78300
aactcaggcc cagaggccag cttactgtca tctggctaag tttctgcttc tgttctcttc 78360
acttgttcaa gatccttctg aggccctgaa ccaaatgagg aaaattcatg taaatcttag 78420
cacactgcct gatacataat aagtgctcca taaacgttta tcagtgctct cataagccct 78480
gagacatgca aaatattctt gcccttcagg gacttacagt cttgaggagg agataaaaat 78540
ttcacacata cacactaaca caattaagga gcaatttaag tcactaaatg gttatctgct 78600
aagttttggg gaataggtgc agtggatcac gcaagagagg agacagatta ttgtggccta 78660
gaagaagtgc catggtgaag ataagagcat aatgtgtatg acatctgtgt atgacatctt 78720
ttcaactcct ggcagtgaga tgaacaggtc agcagccatt cctggacact gcagagccct 78780
caggccatcc actcatctac ccagcacatt cactgattat ctcccaactc tgtgctctgc 78840
ataagaatgt aaggaagacc atgacaagac cctttcctca agcctagcca gggagacaga 78900
catgcataaa gatactacca ctgaaaaatt ctacattcat atgtgttcat atcctcaaag 78960
catcgtctga gcccacaggt gggtaggcaa gtcagatagg agcaagagtt tgaaggaaag 79020
catacattta cctggtgaaa aggggcaact cacaagccat aagctcttgt gcaaaggggc 79080
tggtaaatgt gagtcttctg caggagaggt ggagttgctg ggaggttaga cccaagcaag 79140
ggcattctga gaatgagatt gagaagcctc tgaggccagc atggggcaag cccttggtgt 79200
cacacttggc cctggcaatg tggtattttt aacctgggcc agaaagtgga gaggagcggg 79260
cctggtagga tagagaggag ggctctttac ttatcaagag taggtaccag atgctggtga 79320
ctcaagggga agcttcctga gtggagatgg agcctatagg tgtttaatcg cctggaacaa 79380
acatattttc tgcactgctt ggttcagagg ctgcaagtta aagtaacaca gaagagaatg 79440
tccggccagc aatgtgaagg aagcatgaaa tggaaagggt tagaagctga acttctcttg 79500
atcaatgact ttattatctt ggcaaggatt gcagaaactg taggtgctga gggactgact 79560
tgtttggagg aaggggtagg gaccaggaaa agaatcccag acacttggtg ctaatccaac 79620
tgctgaatga ggctttcagg ctctgggatt caggcaggaa ctcctgggcc atattcttcc 79680
taggtatggg gctagagagc cttcaatctt agtgtctgta tgtccctggg ccaatctgct 79740

ttgttctacc atgcctaaca tcagaaaaaa aggggtagat taagtttaga gccactgctc 79800
tgtgtctcca tcctgcttct ttttcctaag gcaattgagc cttacaaggc cttctaagtc 79860
cctatgaggt ctacagctgt cagggattgg tgaggatgga gaacaaacca gtctgttctg 79920
aattcaactc ttgctttctt tctccctctc acaattgaag gtcttcctga gcctttgcct 79980
ctttcaacag ctggatgttg agactgcaat tttcatgat tgcttgtttt gtatttttc 80040
ttcatggtct ttcctaaagt ctgttattca tccaggtcca cttatacatc ttctaatcta 80100
ttgcaccaac cctgcagcca tggcctggca tgaagctgct tgctcattgt cttgttggtt 80160
tttccaaatg ctcacaggcg ctagtcatgt aaatctcctc ctggcaagaa aaaaagactg 80220
actttttgtt gttgttgtgt tgccccaggt gattcagcca gtcctgggct cacaatagat 80280
gctaaacata cgattgccac ctcgactgct gatctttttc ttcctataca ccattctatt 80340
cataccgcta ttcaaaaatt gatatttata agcacttact atatcccagg aatggtactg 80400
taagttcttt atatgcatta ttccatttaa ttttaatagt gataccattt tctactttta 80460
accagcttat actttctaca ctgatagaaa agtgtcccta aacccacaga ctagttagta 80520
cctggctgtt ttagaagttc ctcactcatg acatttctga agttatcact acaggatatg 80580
gttgcaaagg cctgggagga gaaatccttt ctgtctctga tggtttcaga gaaggtgaga 80640
ggtctctgtc tctttgtggt aaaaacacca gggtaggcct tgatctcctg agtcttgagt 80700
atgttaaggg aaagacacta agattgctct gccaaaaggg cagacagacc ctgcctggtg 80760
tctgggaagt tgagcccaag tttctctcca aggagggtct cctgcaggga tcaggaagga 80820
gggtctattg gcatcacaca tgatctcttc tgggttcagg aggggttcct atagctagat 80880
gagatagaat tgattgatgt ctcagtgatg cttcaggcca tgttgattgg gcctgcacta 80940
aaagtaagca gcccttcaga gccatgtgct ctcaagaggc agcacagcca taaccgcaag 81000
agtaggatgg tcctggtgga aatggcacaa tcaaaacaag ctcctgggcc ttgtggcttt 81060
gagtggctga actggaaggt tcttgaggta tgagcactct cactcacggt attgatcaag 81120
cagcgtaatc acatcactgc ccccacagaa gtggctgcca ctcagttctc ctcagcccag 81180
gctgatttct gctctgctgg ttcataggaa actgcttggg tctttcatga ctgctgaagc 81240
ccaaggcact ttccccagag gcagcatcac tcacaggatg gaccaaatga aatgtcctgg 81300
tcaaaggcag gaagtttgta ttgttgttct tgcctatttt actatcatgt aaatacctgc 81360
caaccaacct acacatattc tctgagtact tactgagtac ttactatata ggtgttaatg 81420

gatacagaaa taagagtatg gctaggggtg gcatgctttt tgcccctaac ttgcaaattt 81480
attgagaaca tgatgccagg cacagtgaaa accaaagaga aatacaaata aatggtaaca 81540
tttatgagac aggtactagg ttctagacac ttcttacatg ttaactcatt tcatccttac 81600
aacattcctg tgggcaggtg ccattattac tcccattttt acaggtgaga agacagaggt 81660
gccaaggttc aagtgtgtgc ccaggtgcat gacactggtg agaggtggga gacaggactc 81720
aaacccagca agcctggtct cagacaaggt gactcataag gagcagcaag ggaggtagga 81780
ggcagaacat tgagtgcagg ggctcagagg gtggagggaa gtaacttcaa tctggtttaa 81840
agaaaccagt tatcagagac agacagggcc taaagaaggt atgggtggct cagagtttta 81900
ggaaggaaaa acaggaagtt gggctaatag ggatagcgtg ataagagaac aagtgctagg 81960
ctggaatacc aggggcctgg ccttgcctgt gagcagctgc tgcccctggg gaggtcactt 82020
tgctttttgg gctgccttat ttttatcacc tccaattgga gagaattgag aaagatgagc 82080
tttcaggtcc cttcaggttt ttaccgtatg gttctatgat tttgagggct ggcatgtttc 82140
cttggttgct tccaaaagcc tctgaccaca gtccctcagg ctgaccctct tgtgcccagt 82200
aatgtggaaa gatgaccttc tgctcaccag cacatgcttc caagggctgc ggagcctggg 82260
acagaaggta cggtgggaaa gaaaatgaga ggagctggtt gggaaagata ctcacatgca 82320
gaaaagtttc cagcaagtca gaggtgagag taaaagatcc aaatgaaaag ctagagattg 82380
ctagggctca ccaaggttga ataacttgtc cagggtctta gcagactgag ggatactgga 82440
tgataacact tctaaaatac tgttttgtat ctggaaaatg ctctgccttc agtttgcatt 82500
ttctctcctt cttcctccca gtaatcaaat gaagacgtta gatttgacat ttccagtgtg 82560
tacagtacag acaggaaact aggtgaatga agtaatgcag tcaatgttct gtgactccat 82620
tctgcccctg gaagagggat gttttctata atagtcatgt gactcaacat cttcttgtct 82680
tcttagaaag gagtgagaga ataagatagc catctagcac tttcccccct cacctctcct 82740
acaaactcta gcatctcatt ttacagaagg aaaggctgag atggagggga gcaaagtagt 82800
cttataacaa aggcagaagt ggagcttgct atttcatcca gtgtgggtca cctcaggtca 82860
cctggggcac atcctatgtg cccagatact tacggaaggg gtcattctgg gcctttggct 82920
ccacactgag ggctgcattt ttgagaatca cgaggttggg actcccagag gaaagttgag 82980
agttaggaaa acttcaacaa atcctcagga atagaaacag aacatgagat gagaatgttt 83040
gggacagctc tgctgtttgt ttctgtgtat gtgtttgagg gcccatctct tgccaaaacc 83100
aagctcattt ctgcatattt gtttgcttaa tatgaaatac tccaaaactg ctcttcatat 83160

ttgtttaatc tgaaatgaaa atgactcagc agagaggaaa aacaagattt aaatgccctg 83220
aatggcccct gaattaaaat acatagccct aaatcatggg ggtgttcttc tccccaccaa 83280
atcttaatct tcaacacaca caaatgcaag tgccacacac agtcatgcac acacacacac 83340
acacacacac aaacattgta tttgcctgcc ccaacttgac ctcagtccct ttatctcttt 83400
tccccttcat actccagcct ttccagggtc ctacctttcc tgcaggggag gtgcatttga 83460
gcttaggtgg agaggcctgg cctgggctac aggccacaga tgattttcct ggagggccag 83520
cacttcctct ccagtccaga tcacaggctc agtgtcaaga tccctggggg aaatctgaat 83580
tttcattctt cagcaaagtc gaggccacct cagatagtcc ctagcttttg gatctggaaa 83640
aatggactcc gtggagagaa tggctgtcta ccaaatcaga catgtcttgc tttttacaca 83700
agctgtattc ctgaaaagtt gggtataaac tggatcttgg tatgttgcat caattttgaa 83760
tgtactgaaa agatttgcta ttcaaaggaa cccagcactg gattcttttg tcaagggatg 83820
aattactttg taatggctac tcatctagtg atttatgagt ttggtaagta gatggaatat 83880
ttttatatac tgaatgttat tttaaatgta ctgtgtggga ttttttattc aaagttaccc 83940
ggcattgaat ccttttgcga agaactattc tgtagtgtag ataaacattt catgatttat 84000
gagtttggta actttaggca tttcttaaag tgggatatac ccctagttct gcttcagaga 84060
aaaagttgtt ttttgcaatg atcagacttg gagaaaaaag agttctacca tatttgagtt 84120
atggtcactc ctgaggggct taggaagaat ggaggtactc aaactttcca ttttgttgga 84180
gcaaaagatc caggagttgc ctgctcttct tactttccat attatcactg gggtggaaat 84240
gatggaggta tttttccaaa gaaatgtagt tcactattat agctacattt caaaggtgtt 84300
tctcttcata tctatatcat ttgaatatat atttatatat atatacacac acatacatat 84360
atacacacac atgcacatat gtttatacac acacatgcac acatgtacgt atatacatat 84420
ataatctatc atgtgtattt agatctaaag caaagacatt gcagtagggc ttgtgttttg 84480
aaccactgaa gggctgctgt ttccaccagt ccagagtgct ggggcttgct ctgagatcac 84540
caggggtata aaccaggcaa gaggagaact taacttcctg ctcactcttt ctcacttggc 84600
aggtcaaaaa tcatttcagc acttatctcc caaggttcct agctgtgtcc ccataccatc 84660
ctatctgagc actagtgtga agcttgtttt gggaaatgat ctgattgcta aggcagttgg 84720
aaaagagaaa tatggtgtca tgtgtgtaca accaagagct gatctaggat gcccacatat 84780
tcctggctca tccatgagcc acatattcat tgaagttctt ataggtagga gcaaaccctg 84840

cctataagaa ccctacatgg actggaactg tgtcaggcag aatgttcaga taacttaccc 84900
tttcaccatg atttggccct gaatgtgtct cagaaaaagg cttttagaag ctcacccaag 84960
tacagagata atttgtgaga catttaacct ctcagactct tgcttttttc tttcctggag 85020
agctgctaat ttctagttgt ggctgccaca ctcctgtctg cctgatcttc cattttactt 85080
cctggtttcc tggtggcttt gacacatggc ctgtccagat cccagtgggg gcagtcctga 85140
gatggagagc tggtggtctt ttgtcagatc tgggaagtcc tgagccatga cttcctttga 85200
aatcaatttg gaatgtccat ccagcttggc tagggaagag gattttgact gtgaagaggg 85260
aagtgggttc tttactacaa aggacatatt ctctctgagc cacatgctcc ttgatgttct 85320
ttggtaaata ttgggctgtg gtcctagaaa cactgacctt ggagtcaggc gaactgggat 85380
ccagttctta ctctgtggtc ttctaactta gtgacccaag caagtcaaaa ttaatctcat 85440
ttcgagatag agatatgcaa gatccttgac cttcaatgac tcatggtcag gcttgagaga 85500
aagtcaggta aagaagccaa ctctaattca gcacaatgaa tgggaagcac cagccagacc 85560
aggtaaaaca gcctcaattg agggtgaagt gacttagggc cgtacttttc tattgtcaaa 85620
aggagatgat aatttctctc cacaggattg tggtgaggct caatgatgta atgcatctat 85680
aagtaactga taaagttcag gaattctaaa atagaaacag gcagtaattc tcaactgttt 85740
tcttcttcca ctctattcat gtgcaagatg tgctaagaca tttcccaata caacaagtga 85800
ttcttgaagt ataggacgtg ttccccccac acctccaact ctcggaaatg cacagaaagt 85860
gtccttgaga ttctgatagc ctgcttccag gaaaaatgtt ttgtttggtt ttgtcagaaa 85920
agctaatata aatgacatta acaaggaaca cattttcctt gttgaaaatc aaaacattac 85980
aaataaggct aaagtctccc tttgatcacc attcacaatc tcctctgaca aatacagcag 86040
tccatctcct aactctgtct tggagggaac cactgctgtc agcatggtgt ttaatcttcc 86100
agatcttata tatatataaa tgtacatgag tgtgtgtcta tcataaaaat atatagtgtg 86160
ctttacataa actttgcaac cttcttgttc acctaaaaac atgtcataga cttcaaccgt 86220
tgtaactgtg agtagctcta atgcattcat tgttactatt gcatagcatt tcacagtagg 86280
aatattctat agttaataac attatcctac tggtagacat ttttctaagt ttttgttgtt 86340
aaaaaatata ttgcaaagca cttttttgtg catgcctcat tgtgtacaca cacaagtctt 86400
tctttagggt aaataagaat aaatgggata tagaaattat ggtatatgta tttttattga 86460
aattatcttc taatttatat atccagcagc agagtatgag agtatgcatt tgccatatta 86520
ttgccaaagt ttatattatc acactttaaa attttgccag tctgataggt aaaattggca 86580

ttcaactact gctttaatgt ttgtctttgt gactacttga gaggttgaat acttttagat 86640
gtttacagca ttatattgtc tcccctgttg aatgcttttt atttacttgg cccatttttc 86700
tagtggcttt taaaaaatga tttatagaga ttattaatac tagaagttat ttcattatac 86760
aatttcaaat atttatctta gccagttgtt tacatgttat gtttatgatg tcttttggtc 86820
ttaaagaagt tttgaatttt ggtggaaatt tttattattc ttttcatttc tggattttgc 86880
tttccatgtc tcatttgtgc aggccttcct tatctttagc tcatagcaaa tttttctaga 86940
ttttccttga aatattctta tggttccatc tttacatttt gggctcattt ttgtgaggtg 87000
tagatatacc ttcatttgct tttgaaaagg ctactaaagc atctgaaact tcttcaccaa 87060
gtttaaatac ccaccttctc atgtagtaca tttccattta ttcatgagtc ttttctgggt 87120
tgttcattct cttcagtgtt ctgcttatct atttctatac caattgcata ctcagagagg 87180
cctttgctgg ccaccttacc tagaacagct ctcattccct ttctgttaca ttactctatt 87240
ttattttctt ccctgtacat atgtttcttt aaaattcttc ttcatttact tgttttctta 87300
tgtactataa gttccatgtt ggcaggaacc ttctttgcct tttttactga tgtgcttatc 87360
acctgtaata gcacctaata gatatttaca aaatagttgc tgaatgaatg aatgaaggcc 87420
aatggcttga ccaggggtgt ttgcattttg agttttattt cttgaggctt aaataaattg 87480
tcaaaggggt ttgtactact aactaaataa gtgggtagag aagggctttc ctagcagctc 87540
cttgacatgc ttccatgtat acttttgagt tctgttcaca tgtcacattg tttaaatatt 87600
gaacccttgg aaagactcat tcgaaggtgc agaaaagggt gattctgctc acaataagct 87660
ttagagatac ttccttttag ggtatggaat ttagaatgac cagatgtagt cactgaatat 87720
atggctcaag gcaatcatta attcattctg caaatactta tcaggcactc tggtagtgtt 87780
gagactacaa caacgaataa aatgaacatg gagcttgtgt gtgttggggg ggaatacaaa 87840
ttaaacaatc acagcaatga atatataatg agaaactatg atcatttcta tatagaaaca 87900
ttatgttttt agagcatgta acaatcaagg ggatgagaga atattttgct gagaaagtga 87960
ccatttagta gagatttgaa ctaggcaaag agaggagaga aacttcttgg cggaggtcct 88020
taggcaaaag aaaatggaag aaaaagaaaa gaagctccag tgtgactgag gtggagagga 88080
tgaggagaag ggcagctctg gtgaggtagg tggagcccag atcaaaggat gatatagtgt 88140
gatgatgagc aaaaacaaaa gaaggaacaa caaaaacaac aaaaacccat gtggattttg 88200
aagtaacaca agccttaggt tcacatatga gtttggtcac ctatcaggtg tgtaacctga 88260

ttagctttaa cctctttctc cttagcttcc ttgttgttga aaacaaacct cattgagtta 88320
ctgtgaatat tgtaagagat ggtctctgat tcgtagtaga agtacatggt ataggtataa 88380
tacttctagg tgctttacag tgctctgttt gttagctgac agtcatctaa ttttaggcaa 88440
aacagaaaaa taattattgc ttttggagat tcgcagcttg aagcccggca acagtttcaa 88500
aaagtaaatt agtcaacatt ttttgaacac ttagtaggtt tggcttagtt cagttaaaaa 88560
ccatttttga gcacatactt tgtgcacgat gtaaagtaat actttggcaa tataagaatg 88620
aaccattttg aagttaatga ttaaatggat aattttataa caatgttgtg aaggatccag 88680
tagagttaaa ttaagcatgt gttttggtga aggaaagaaa ggaaatgatg tgtgggagac 88740
attctttgca gaaggaatta ttggggaagg ttatggaggg gaggaagtta cggccagtaa 88800
cccatgagag aggtgccaca ggagagagag agaattctag gactgtcctg caaaagactg 88860
ctaggagagg gcggtgagcc aagtcagtcc tgggcctcag cacttactgg atggctgctt 88920
acaaagagct cgggtctctc tggaaggaca tcctggatta tggtcatgag ttagtgctca 88980
actatagcca agaccaatgt ctccaggatt cacctgtgtg tttctttctc tgggcatgtt 89040
tatttaggga cttatttatg gaaatagtgt tcccgttgct ggaagtggct aggaacactg 89100
agacatgtat tttatcttat ccaaatcaat tgctatgaaa ctatgctcat caccctttgt 89160
agttgtagat cacatacccc tcagcccctc actatggggc catttaaatc ttccgtgttt 89220
tatttgcttt ggatccatcc tcttcagtca aaaccatctg actacattgc tttttttttt 89280
ttttaacccc tctgggtgtg tcagtattat actgcttaga aattgtctta gaaagcttct 89340
gtttcctgaa aaagaaaact ccttactcat gtcatcctct gatttactcc atccctgtta 89400
ttgcctatta aagtcttgtc catccttcaa gaacctgatc agatatttat ttctccatga 89460
agccttcctt aatcactcca gcttaaggga atttcttctc tctttataat attgtgtcac 89520
atactatttc taatattgca tccaatgctt gcttgattat tagtgttact cagagggaat 89580
tttttaaaaa tcactttgta ttctagaaca gttttttttt ttttgtctca ctctgtcgcc 89640
caggctggag tgcagtggca caatctcggc tcactgcaac ctccacctcc tgggttcaag 89700
tgattctcat gccttagcct cccaagtagc tgggattaca ggcacccacc accaagcctc 89760
gctaattttt gtatttctaa cagagacggg gtttcaccat gatggccagg ctggtcttga 89820
actcctgacc tcaggtgatc cacccgcctc ggccttccaa agttctggga ttacaggcgt 89880
gagccactgt gcctggcttt ctagaacagt tttatattta cataatcatt gtgaagctag 89940
tacagggaat ccttgtatat cccacatcca gtttccccta ttattatcat cttatattaa 90000

catagtacat ttgtcaaaat gtatgaactg atatagatac attattatta gctaaagtcc 90060
attctttatt ctgatttcct cagtttcccc taatgtcctt tgtctggata taacattaca 90120
cttagttatc atatctcctt aggcaccctg agctgcgata gtttctcaga ctttcttgtt 90180
tttaatgacc ttgacagttt tgaggattac tcatcaggaa ctttatagaa tttcccccaa 90240
ctgagacttt tctggtgatt tttttataag tagactgagg taataggttt ttgggaggaa 90300
gtccacagag gtaaagtgtc attttcatca catcatatca agaacacata ctatcaatat 90360
gacttatcaa tgttgatgtt aaccttgatt acctgtcttg aggaagtgtt tgtcagattt 90420
ctccactgaa aagtgaccct ttttctttct ctgtttttac actgtacttc ttgggacaaa 90480
gtaactatgt gcaacttact cttaaggagc tggggaaggg agatagtaat gcttcatttt 90540
cttaagagca gattatctac ataaattatt tggaattctt ttgcattgga aatgtgcata 90600
ttctccccat ttgtttattc aatcattaat ttatagcagc atgaactcat ggatatttat 90660
tttattcttt ggtttataat tcaatactgc tgtttttgag gggaggaact cattaagttg 90720
gctactgtgt ccctttgtca taccccatca tctttttgtg tgtacatttt ctcaatttct 90780
ggcactaaaa gatactccaa gtccatcttg tttatagtct gctccattcc cagaatcagc 90840
catttttctg aggaatcctg tctcctttta ttgaagaatg ttattagaaa tcaagatctg 90900
agcactaggt ttgctcattg ctactgggat gtcattgcct ctaggccttt tcagcggatg 90960
gaacatatgt gtgtatacta acccaggcat atacacatat ctataaatat ttccatatgt 91020
aactatttgt atccttatta agctaaacat aagttcatac tgatgtctcc aaatctaatc 91080
tattatcata gattagatat tatgaatcat tttggggaaa cctgaaaata cagaatccca 91140
ggtatcactc caaagataat gaatcgggat aaatattgaa cttatatgtt ttgaaaggca 91200
aacattaaaa gtaaatgctt ctcttggttg aatattggaa tcaatatcaa gattatgaaa 91260
acatcagttt acatgataaa tataagtttt actttaccat tattcaccat ttgggtaagt 91320
atttatctta ggaaaacttc gtgtgtctga ctatactttt gctaatttaa atttcatgag 91380
tgtgcaatct gatagagttt gaaaattatt atattaggtg attattggat gtgccattca 91440
actcccaaag tctgtgattc gaagtcccat atataaaaac aaacactgca ggaacacaaa 91500
attaattgtg agaattgtac tcattcatgt tggcagtatc tgcctcactc atttcctctc 91560
cttctctttc tgttttcttg cattattggt tggtggcaga ctccagttcc aacaccatct 91620
tctgagatga tcctgattcc cagaatgctc ttggtgctgt tcctgctgct gcctatcttg 91680

agttctgcaa aagctcaggt taatccaggt aacatggcta ttactcagct atatgctaga 91740
agaccagcag aatgtttaaa cccatcaatg ttcaatggtg ggtctaaaag ctgccaagag 91800
ggactacaaa gctctttctt tttaaatctc catggtgaaa tatgactaaa ataggaagaa 91860
aaatgaatat actttatata tatgtatttt aatacttaca acctgttgaa tattgtaaaa 91920
tattttctta tatagagtag gggtgcagta agagggcatt ttaaaaaatg ggaggtggca 91980
gaagcaacca tttgcttctg catcttctga gaaaaacagg acagaatttt tagatgtctt 92040
ggagaacttc ttcaggttgg tttaccagtt gttttgggct gtcttagact ttgattttac 92100
ttggatgtcc tgttatgctt ttgggatcct ggtcctgtgg gatctcaggc aggttctctt 92160
tcctctagga tgtggccatg ctaaaaatag ataggttagg agaatctttt gagcccaaat 92220
ttgtgggatt attttgtatt acatgcattt ttaaagttta aatggaagta aataattgta 92280
ttatttatta cacaataatg ctgcttaaca acctatccaa agctcaatgt ctttaaacga 92340
gcatttattt ggctcctagc tggacttgtt tggactgttc tctgttctgt gttgatttac 92400
tcaagcataa ctggaagtca gctggctatt ggctcatcta ggatggatgt ggcactgatg 92460
aatggggcaa ctttgctgtg ttctgtttca tcctcctgca gactaacctg ggtatgtttt 92520
cctggaaatt gcacaggaac aagaccagaa gccagaatac tcaaacattt ttcaagccac 92580
tgctgaaatc atgtttacta agtctcattg gccaaagcaa ttcatgtggc caagcccaga 92640
gtcagagtgg gagagcacaa aaaattagat ggtaaaggtc tgggatgcag gtattggaga 92700
agaattggag tcattttttt tttcattttg ctacaaatat gaacatggaa aagtatacag 92760
cttaaatgtt caattttatg aattcataaa ctgaacacat ctaaacagta cctggattaa 92820
gaaacagaac tataccagca ctttagaagc tctcttcatg ctcccttcca gtaacttttc 92880
tcaccacccc gcaaaggtag ccattatcct gctttctagc aacatagatt agttttgccc 92940
atttttgtac tttatataaa tggaattaga tgatgcataa atactctttg tgtctggctt 93000
ctttcactca acattttgtt tatgaaattc atcctactgt tgcatgcagt tgtcaactat 93060
tcattttcat tgttgtatat tactttgttt gaacatttta tttagccaat ctactgtaga 93120
tgggaatttg aataatttct agtttgggcc cattatgaac aaaactccta taattattct 93180
tatataaaac ttttggtaga gataagtact cagttccttt gagtatacac ttagaagtgg 93240
aacttcatat gtgtagcata tgatatacta ttctgcataa taccaaaaat tttttcaagt 93300
actttaccat tttaccatcc catagcagta tatgcaagtt ccagttgttc catgctatat 93360
tttgattctt gagggtgctt taattttgtc ctcttcatag cataagggta catttgtgaa 93420

aatgcttgct ctcctccctt cataaaagtt ccatgtgcat gggtttttct ttataccttc 93480
aataggaaat atgatcaaac atccttaaaa tcaaagaatt attggctgaa ggagcccagt 93540
gccttgttac tttctcgtaa taagctcagc tgttgcatct cagatcctaa cttgggtacc 93600
atttttttcct ttttctgttc tgtattcctc tcaatcaatt gttgatgttt ggttgcctcc 93660
tactacattc ttcatgctct gagttctaag gaagagtgct gtgagtgcat ggaatggtgg 93720
gatgttttgg tggtaagcat agtcatggag actggcatgc cacgaggaca tttaaatgac 93780
taaccaggag agctgatctt ctgtgttaga gtccacactg tggccctgga gcaggaaatg 93840
ggctatttgg agacagcaaa tagtcatctt aatttttttt tctatttctg gtgtcaggta 93900
tttttgttgg ctctttctga cagggaaaga gaaatagact tcttgctttg caaagacctt 93960
tataggaaaa caggacatgt tgaaggacta atccttctct ctgccatttc tttctatttt 94020
accaagtcaa accagagatg agtgtttgag gcagatgaga cattttcatt cattatcagt 94080
gataaagagt ctatcatgaa tcctgaggct catagaatgt tatagcttgc acactgctga 94140
gcataaagtc agcatatgat aaatgcatgt tgatttaagg atgactcttc aggagtcctt 94200
actggtgtca actgaaagac tgactataac tgaaagtatt gaaaggtaac tgaaggcact 94260
gaaagactga gtgaattctt ggccagttag gtttttccca cagaatggcc acatgatata 94320
acaaatagaa cttaagccag gagacctgag ttataagcaa atccaatatg caaccaatta 94380
ttaaattact gaaacatttt agttggttat tagctataac atattggttg gttaatagta 94440
actgagctaa gcattaaact caataaaata aaaataaaat aataattagt tgaagctcat 94500
tgttgaagat cttacaatga ctattggtca ataagccttc agtattagca tgatttagtt 94560
gataacaaac attaaacatg atatgatatg aaatatcaat cttggtgtct cagcagatac 94620
caagagagga taaataatct tgtttacatt taaaaaaact ctctagtatt tgaaatcata 94680
aatgtctcac aggtgtcatc catggaggct gcagagtagt ggtggtatga cagagatagt 94740
tggctgaagc acgaaggcag cagagttttt ggctcactgt tctaaagtat ctgagttttg 94800
gcaaagcagg cagtcaggca gatggtgtta gtggcatttc cagtaatggc agtgttaaag 94860
gtagcattgt caatggggcc tggagcgtag gctacgcaag tctttgtggg aaatgctgac 94920
atgaaggtgt atatacatag tcctgaattg tgggcagagt tctcagctat cttccagctg 94980
caacagtgca ccaggaactg gaacctaatc atcattaatc cagtgggaga ggttattgga 95040
agaattagag gcagtttctt atgtcaaaat catggctcag aaataccatt tagctagtga 95100

catccaaatc aaacaaataa tcatcccaat aatttctgaa aatcatttat acaaatcaac 95160
agatgattgg aatgttatta gttctgatga atgatttgga gttaacttag aaaaaagtat 95220
ttagcttaac ttttgagtaa caaccctaat tataatcctg acttggtcat aaattaattg 95280
tgtgacagtg agggagttgt tacgtgtctc tttatacttc cttaatattt tcacttgtag 95340
taagattaag ttggattaga taatctatgg gaccctttct gctacaaaat tactgtgatt 95400
tttatgatgt gagaacttta tagcccatac tggaagattt cggcttggaa gtatagcccc 95460
tgaagttgca tggctgtttt ttgcaagaaa gactacttgt ttccaactcc ctaccacttc 95520
ccaggctcag ggatgtgaag tctgaatttc aggagaatct tgcctttggg gctaagcaca 95580
caggaagtga ctgataaata cttaaaaatt ttaaacccta aaactctgct tagtggcagg 95640
tgaatgcaga aaccctgccc cagcctaacc tgcaggagag tcatagtgct acaagctacc 95700
ctgctccaga gcatcttttg ctctagaggt ggggccaaga ggtagcattt caggctgaag 95760
atatagcgta tgcaatggct gagaggaggg tgaggaacat ttggggctcc tggtggagga 95820
taatgagtga gagagccagc ggggctatgt aggagatcca ttcccctgtg ggatccttga 95880
gtcaagtttt cccttatttc cagcttgggc tgggactgcc aaacttgata ggagaactaa 95940
gggcatctcc atgggaaaga gaccccacaa tcatgcacaa aaatacgggg ctgcagtagt 96000
tgtttgagtg gacatcagtg tcaggctccc ctaggggata taaaaccact gtttcagtgg 96060
ttagtgggga actggcacct tacgtttttc caggtaggag gatgaataga actatcctga 96120
tgatcctgaa attctagccc agcagtctga agccctggga cccacccagc caattagagg 96180
attgttgtgt ataaaaaaca caggggtgct tgttcttatt cccctgactt ggcctgtgac 96240
aactgtttgt gctcaggaaa gggagaaaca tctcttcttc tcagcaggca aaccaggcct 96300
ttaggatcca tggtataaag ctgaggcagg gcagtgctgg aacttcccat tttcatgtca 96360
cactttcccc tcataggaag caggcaactt actaccagtt cagagtcctg gggtgggagt 96420
ccccctttgg tatccagaat gcttccactt tgaaaaagag tggcaaagcc attcagggtt 96480
tgggattagg agaggacgag cttttctcct cctggcccca aatttatttt aggttcacca 96540
ctgtggtttt tgttctttcc agcatcaatc ccacatgtca gtgagttctt agggtcaggc 96600
aactagaatt ctagactgtt catgttggaa tggaccttag aaatatctaa gtcactcttc 96660
ctcactttac agatgaggaa actgggagtc aaagcaggtg agtgatttgc ccatgctcca 96720
catttagcta atagttaagc taggacgagg acccagggct acctgtctcc caatcccagg 96780
tgacttctac ctgggccctc agggagcaat gtgctgaagt gacctttatc taaaaatcac 96840

atatatccat gcagcctcat gctttccttt ctgcaaccaa cttccaatgt atatttatgg 96900
ggatgagatg taattgagat ttgagggaca gcctgaggcc aaagaatgga gtcagacgaa 96960
taggatatac agtgctcaca cctagtctca tgctaaatga ggaaggctgg agctagaagc 97020
cctgcgggcc aacttacccc cacccagaaa ctgaagcacc agggatgctt tggttccctt 97080
ctgcaccagg ttgtgccttt atcaccgctc ccttgtcttc caccaagact gctatgtgtt 97140
aattcaccta cacatatttc caaattggaa actcattttt gtttccctgg cttctcgtca 97200
ttcgtgttcc cagagatgtg ctgagaaggt ttatgtggcc tcatctgtta acgagaaggt 97260
gatgctagtt tcaggtttgt gaaaccaggc ctgaggtact agtgttgtga tgtgtgtgta 97320
tgtgtgtgtg taggtgggtg gcgcgggggt gtagtgtgtg ttagacagcc tgaacaggcc 97380
ttgagaaaaa gataaaaggc cggcaaaaga cctctctggt tgaatttatc ttaagtgagc 97440
aatgctagaa gctgcccaga ttggctcttt cttgcttgtg tgtttgtttt ttaagtctag 97500
gctgacgcct gagagtggcc tttggcatcc ttcctgccag taaaagagcc tttctctggg 97560
taaaacagaa actcatctgc cccttgagcc ctcttggttg tgccaaatag ggaaaaagag 97620
aatgcaaaac tcaaagaata ttcaatgaac acttaatata ttctgggtac cattttaagt 97680
ggtgtcaaca aatttaatct tcacaataat cttattagta ttagtgttat tatataatag 97740
tagtattatt attccagttt tacagaggag aaagtgaggc caagaaacat taggtaactc 97800
atagcaggtt acagagaaag ctaagtggca gaaccggcat tcgactctgc tccagcaagc 97860
atgctctttt cttctacttc tttttcttt atcccgtttt tcaaatttcc aacaatgtat 97920
cagttaagag tttttcgctt gtttttttgt ttgtttttgg agagggcatg ttgctctgtc 97980
acccaggctg gagtgcagtg gcatgaacat gactcactgc agcctcaacc tcctgggctc 98040
aagccatcct cctgcctcag ctccccaagt agctgggact acaggcatgc gccaccacac 98100
ccaactagtt tgggtacttt ttgtagagaa ggggttttgc catgttggcc agactggtct 98160
tgaactcctg acctcgccct cctaaagtgc tgggattaca ggcatgagcc accacacccg 98220
gccacaagca tgctcttgac ctctgtgctc tactgccttt ggagtgtgag actcaaggaa 98280
tgtcagggca gaggaaaccc agattcatgg accagccacg aatgggctac agagacccgt 98340
gaatcccctg aaattgcatc ccaaacttaa tgtgtgaaga caaaagtaca tttgcaaaat 98400
gaaaaatgtt ttctattatc agatgctcaa aagactctgt gaccagttct agaaaagact 98460
aagaatggtg gatttaatct aatcttgtac aaatgaggaa actgaggttt agggagataa 98520

agtgatggga ggaaacatgt ttgaaaagac tattagcaga cctaaccagg ctttgcctac 98580
tcgctggacg agcatggtca gagttttggg ttctctgaac tgcagtcttc tcacctatca 98640
ctgatcgctg ccttaagggc tgttaggaag agtaagagaa tatatgtaaa gtacctagca 98700
tagtgtcaaa cacacagaat gtgattaata aatgctggaa cttctgtcct cttcctcaat 98760
tttgtatatt gtgctgtgat aatggcagac ttcattagtc tttccattat tctatactgt 98820
cctttatatt ttagtttaat catttcatta attaattatt taacttaatt actatactgt 98880
aaaaatattt cattttcttt gagggcagac ttcacctaat tcctgggctt tgtatcttgg 98940
gataaaaggg ttccaaatac atctactgca tcatcacctt tccatccaaa gtagccaggt 99000
gaattcccgg ttcataccct aaaactttgc cttcaccctg ttgttttgca ttctgaggcc 99060
tagattccta gttgtaaaag gcagtcagag gagctaaaag taatgcaaga aaaaacaaag 99120
ctccactttt tttctgttgt cagtagggat agaggcccag ggagacctag tctccttgag 99180
tctaaacttt ggggccagga gcatgatcat gggactccag gaagagcttc acacaaagtc 99240
ccttttctgg acctctcagt tttcacaacc acctgcagag ctcagttttg ctcagggctc 99300
tcgtgtggaa agactaagag tctacagaag aacaacagtg ttggcaggag cagacaagga 99360
gacaggaccc ctgcttacca tggaaactgg gcgcactggg gccattaggt ccgtgcatca 99420
ctgctgcttc acatatttgc ttattaatgt tttgttctga aagctgacaa gaactctagg 99480
ggcttcaagc ccataagatc aagacataca atatagcgat ataagtatgt aagaaacata 99540
gattgataca gatataaaat aggaaaagga tatgaaacaa atataatata tatgataaaa 99600
attataaata ttataataaa tatattataa atattataat atttaatatt attaatacat 99660
taaaatataa acatatattt atattttgta aatataaaca tatatttata ttttgtaaat 99720
ataaacatat atttatattt tgtaaatata aacatatatt tatattttgt aaatataaac 99780
atatatttat attttgtaaa tataaacata tatttatatt ttgtaaatat aaatataaat 99840
ataaatatat atataaaata tgtataaaaa taaacataat aaatattata ataaatatac 99900
acacatatat atgtgagtat atttatgtat gtatttatat atagatatat ctatatttat 99960
gtgtgtatat ttatgtaagt atatttatgt atgtatttat gtatatatag atatatctat 100020
atgttcacat gttacatgtt atatagatat atctatctat atatagagtt aaaaataaat 100080
aaataaataa ataaaaataa aaataaaaat aaaaataaat aaataaatac agagaagcct 100140
gggagtttcc ttgacttcct tttcccccct ccctcttctt attcatcaaa catttgttga 100200
ttgcctatat aagccaagca ctgtaatagg tgctggtcat gacgacacaa cagattatca 100260

ctacaccaga gacaaagacc aaagtcataa aaacctctga agcaggaaga aaaaaaaagt 100320
tgcataaatt tttttaacaa ctcatcacct cccacttagg ctcataaaaa aacctcactg 100380
attttagtgt cttaacttct actttactgt gtatgtgtcc aaactactga aaaattctgt 100440
gagggctatc ttgccacatc catactcttc agagacccac aggccaggac caagagctaa 100500
gtcgacaatc acactatata tatctatata tatatatata gatataatca cactatatat 100560
atctatatat atatagatat aatcacacta tatatatcta tatatatcta tatatagata 100620
taatcacact atatatatct atatatagat ataatcacac tatatatatc tatatataga 100680
tataatcaca ctatatatat ctatatatag atatatctat ataaatatat agatagatat 100740
ctctttatat atatatagat agatatatct ttatatatat atagatagat atatctttat 100800
atatatacat acatagtcca taaatatgta cataaatatg ttcacatttt acccaatcag 100860
gtcaaatctc caatgtcatg ctagttgaga tactgaaggt atgagagtta gctggagctg 100920
aaagaatttg ccaggccagg taaaaagagg aagggaaagc ttttgagctg aagaaaaggc 100980
acagaggcac aaatagaaaa acaagagtga ggatagtgat tattgagggg tcatgaaggc 101040
aaaggtgggg ttaggatggc accaaaagta ggaaccatat aatcttggga tcctcagtat 101100
atgacaaatg ttttatgatc aaaggccttg tcttcacctg gtgttaacat aatgctatgc 101160
catgctgtct ttcccagagt gcaggttcct tttgctatca catttcaaca aatcctggga 101220
caagcacatt ctaaaaatta gacaagcaat aatcctccct gggctctctg tccagattct 101280
tccccaactt ggtgattctt gaccctagtg atcacatgat aaacagggtg tgtgtgctgg 101340
tagcagggtg tgatgaccac gtgatacggt tactagacag actgtgacaa tggagatcaa 101400
gagatctaga ttctagttcc agctctgtca ctatctagct ttgtgacttt aggtaaatct 101460
ctgcacctca ctttgctcat ttataacatg gggtccataa actttgctct ctgaacttca 101520
tagggttact gcaggggatt gaaagagata atggaaataa atgggctttg gaaaattaca 101580
tgcaaatatc aaagactgtg atttgttact tttggaagaa aagaaaatga aaataaacac 101640
tatgaaacaa cagtttctat caagcagatg atgcaggagg aagaaagaga aaaaaactcc 101700
attagtgatc attaaggtcc cttgtggtcc ccaagaccga ctcaagttgc ttttggggaa 101760
tggggcttct aacagtcctg aactctggcc accctaactc taaagggatg caggaccctg 101820
tgctcaagcc aagcatatcc atttatatat atatatatat atattttttt tttttttttt 101880
tttccttggg agaatagtgt gattgttgac ttagctcttg gtcctggcct gtgggtctct 101940

gaagagtatg gatgtggcaa gataaccctc acagaatttt tcagtggttt ggacacatac 102000
acagtaaagt agaagttaag acactaaaat cagtgaggtt tttttatgag cctaagtggg 102060
aggtgatgag ttgttaaaaa aatttatgca actttttttt tcttcctgct tcagaggttt 102120
ttatgacttt ggtctttgtc tctggtgtag tgataatctg ttgtgtcgtc atgaccagca 102180
cctattacag tgcttggctt atataggcaa tcaacaaatg tttgatgaat aagaagaggg 102240
agggggaaaa aggaagtcaa ggaaactccc aggcttttct gtatttattt atttattttt 102300
atttatattt atttatttat ttttaaatgg agtttcactc ttattaccca ggctggagtg 102360
caatggcgca atctcggctc actgcaacct ccgcctccca ggttctagag attttcctgc 102420
ctcagcctct cgagtagctg ggattacagg catgcaccac cacgcctggc taattttgta 102480
tttttagtag ggacgaggtt tctccatgtt ggtcaggctg gtctcgaact cccgacctca 102540
ggtgatccac ccaccttggc ctcccaaagt gctgggatta cggcgtaagc caccatgcct 102600
ggcttttttt tttttttttg caaaaaaaaa aaaaaaaatc taaatgatgc catgtgagac 102660
ttgatctcag aaagatgttg ggaagctggg aaggaaccca taggaatatc atccctttaa 102720
atgatgagat gctgagtatg gtgtgagtaa attagaagga agataatgaa ccgaggccat 102780
ttcagctgaa gctaccaaag tgcagcacca ggtccagcct tctagtgtta gagaactggg 102840
agagcccatc tccttgcaga ggtagagcag cccgggactg caggggaggg tgaggccttc 102900
atgcttgttc ctaaatgcct aaggtcttcg tttaggctac aagcggagca ttttgattgt 102960
tatacataaa ggaaaatgtg gttaaattta attcaaatat atgtaaatat cttatgattg 103020
gcatttcaag ggaggaagtc agattcaatc ttttggctac tgtaacctat attaaagata 103080
ggaagtatgc aatgatgtgt actgtctatg tacaggtatt gctaacttga gaaatgtatt 103140
ggcatgggag gagccctggg cttgcccata ggacaaatcc acctcgaaag cagagcagag 103200
tgtcttcatt ctgggtagct ggtaggatgt aagcctgaat tcttggtctt ccagtgcttc 103260
ttaaaaactc ccacgtgctc tctttcctca ccaggtcctg aactctaatc tgtgtcctgt 103320
ccgactctca aaaaatcagc tcagctcttc agagggcttc tccttacatt ccccctccac 103380
acagccccag catttggcac atgtgctgag tgagatacac tgcatgctcg agggatacca 103440
ggtctccctc aaaaactctg ctaattttgt cccgtagaga ccgtctgcta gtgccaaacc 103500
cggagtcggc ctcctgcccc tgctccaaat gggcaaatgg ctgaaagtgg ctacagaagt 103560
tccctcacct ctctgagatt ctcccttctc tgaagtcttg gtctctgcat tctcattgcc 103620
tcagaaacaa tcagattcct tcaagtagat gatttatgaa ttttatttag ctcttccagt 103680

tacttttaga gggagcacta attactgcac actactccat cccacctaga aacagagata 103740
ttaataaaaa gtgaatttat actttattgc cactgtgatt catgttaact aatcatgttt 103800
tactttttga cttgacttct aggaagctca gagccaaagt tgaaacccat catcagccag 103860
tggtatagaa ccaaatgtaa tactctttct gtattaacct tagctctagc ttatctaatt 103920
gttcctattt tctttttcct ttatccaaat ttcttcactt atttatttat ttatttttat 103980
cttatgcaac tttggaagcc gactccaaac tttactgagt gagtcaaagt aaaaataatt 104040
aaaaataaat aatacctaac tttattgaat attagtgctc taagtgcttg acataattaa 104100
ctcatatatt ccttatggca ttcctataaa caagtgcaat tactaccatg aaattgagag 104160
gctgaaagct taagtaacct ttctaaggtc ataaaacctt ttaattgttt aaggaagagt 104220
gaatgtacta ttttaaacaa atagttctct cttctcctct tagatatcat ttattgagcc 104280
ccattacatt ctataaagta tgctaacttc tttgtataca ttatgttaac cttggcttgg 104340
caggattatg aaagatgaag aaaccgaagt tcatagaggt ttggtaactg gtagtggggt 104400
cccataattc agaagtggca gagccaagtc ttaatataaa ctttccatga ttccaaaccg 104460
tggcctcagt tttcttcttc actcctgatc tgcatagcta cccagtttcc tcttcagctt 104520
accttcttgt tggggaaagg agatactatt tactatgaaa gtttcactgt ctttgttaac 104580
caggaagaag aattcattat ctgatcccta tgttagtatc ctgaaaccca ctacagcttg 104640
gggactgctc tcctggggct ctgcaagaat tgtttctgga aagtattttg ggcttccagg 104700
aaaactgcaa tcctaaaggt ctattcctaa ggggaatttc aacttgtgca agaggtctgg 104760
gtgaccatta tacccaaaga atagccctgt aggctgaaga aagaaaccca aataatcttt 104820
cttggccccc ctgtggaatc ccattgtgtc aatatttatc tgcacaaagg accagtctga 104880
aacagcctgc tctgtgaagc ttgtgggaag cagggcctca gtgacagttt ctttaagtgg 104940
actaccttgt cctttcagaa actggaagaa ctagaccatg gtagcctagg gttggaggag 105000
gaagaaccag ggatcaggag acctgaattt cagtcctggt taccctagcc ctctgtgtga 105060
acttgagtaa gtcacttcac ctttctgggc ctcagaggtt ctgatattta acacggagac 105120
attagaggga ttggcttctt aaatttgctt ctgggctcca acgttttatg attgcagatg 105180
tccctttaga ttgtagactg gcttgagacc gctgacccca ataatgcccc tcagtcagtc 105240
ttgtctgggc ccagagaggc ccaggtgtca ggtctctgca cctcaggcct catgtgggcc 105300
atgtgtgtgt tttcctggaa gctgcatata ggacactcat tgacggggag cccgtggtct 105360

ggagctagta gtgagatggt aaacattatg gaacaagcag ccctttcagg ctctcagcct 105420
tggcaggata gtatgaaaaa taattcttgg gaagctgggg actggtggca gctgcttctc 105480
ctcagcctgc ctgccaggcc ctaccagagc ttggagggct ggagctccct gctgggcctg 105540
ccacagcctg tgctggtagt tttccactct gggaaactgg aaaagtgaat tggcttctct 105600
gtccgggaga ttggtgggta gcggaagcat cagaaaaccg ctaagacaac tcagcaggaa 105660
aaaccaggag acacttcttt gctgcagggt ctcttgcttc ccttgccatt cccagctttt 105720
tcatgcttct ttacagttca tgatagaaca aagacagact ggtgggattt gatgaacgag 105780
gaagtggaac tgggggaaag caaggagacc tgacatttag ggcttgggcc aacttggcag 105840
ttctgagtct tgactattgg ctgagatccc ttccacagaa ggagtttacc caaaataggc 105900
acattcaatg ggaaaaccac actggtacag ctcagaagtg ccacgttggt tgccctgggc 105960
ttttcctggt tccaaagatt cagtaggatg agaaaggtca tccaagatca tcttgtccat 106020
tttcctgtgc cttttctcta aggcccttgg gcatactgag tcactgagaa actttgccta 106080
ttttcttaat gtgactattg ctgatttact aagttcttat ctctgaattg tctatcctca 106140
tccttatatt actttgtcac aatttctaga cctccatcct aactttcgtt gtctggagat 106200
tgtgtgcaat acttgtagcc ctctctgcct tttcatctac ttatttattt gccaaacgtg 106260
tgttaagcat ctcatgggtg ctgtgtgttg gcaggtactg agggggaaaa ggaagaatct 106320
gacatgatta ctattctcaa agttgctcac aatttcaaca aggaggtaac caagcacaag 106380
agtaacagaa aatctccgtt ggagtttgtt acttaataga gctcgcctac acaccatcat 106440
cactttaatc ccataccacg ttttagtatt ttccatagct tcgtcacatt atctgaatag 106500
aaagagccag taatagctac tgtcaaatac ataatcaaat tgtgcctatt tgattatgaa 106560
aaatctattt tctttttcct gtctccccta ccggaatgta agatccatga gacaaattat 106620
tttatctgtc ttgcttacta ctgtatcttc agaagaacac ccgatacaca gcaaaaactt 106680
atttattaaa taaatacata cacatttgta aaagaaatac atttgtgaac tcaatacatg 106740
caggtaaaga taggttgcaa taaaatatgt catgactcag acttacaagg aagtttcctg 106800
tatctcctgt ctgctaaaac accattagcc aaggctaccc ttgaccaaac tcccaggact 106860
ttgtttttaa gccttgtaaa gtgatgaaca acagaaaaca cttcctttat cacttttcaa 106920
tagacgttta ttgagcactt ggttaatgcc agacatccta ctagaaattg gagatacaac 106980
agaggaagat agatatgatc cttgccctca taacacatgc tgtttagtgg gcagaccaaa 107040
attaaacaat tatttacatt acaagtctaa aaactgttat gaacgaaaac cagtttgtta 107100

taagagcata taatagaaat tcctaactta gctggggact tgggggaatt ccttggggga 107160
gtgactggct gacagctgaa ggatgagcag gagctagtga gtgaaggagg gaggatgcac 107220
atcccaggta aagggagcat catgcacaga gaccttgagg ctggaagata ctttgtgcat 107280
tcaggaactg aaataaggtc agcgtgcttg gtagagagca aagaacaggg aatagccaga 107340
gatgaggcca gagagtaggc tgagacccaa tcgtgtggac ctcaagggtc acattttctt 107400
aagaagaatg aaaacctgtt agaaggttca tgacagaata aagttttagg aagctaattt 107460
gggctattgt gtggagaagg gatgatagag gaacaacgaa gaagagctag aaggcattat 107520
agtggctcag atgggaggtc atggaggttg cgcactgcat gggaagaata gaggagagaa 107580
acagctggac tcaactgcag gaggtaggat ttgtagaact tgctaatgga ctggatggtt 107640
ctcaaaggcc aagcttactt ctcattcctt gactgccgcc tgttgagagt aacatcttac 107700
ctatcattcc tggactcttc ctctccaaga attctatttt ggtctccagg cagaggttga 107760
ctcagggaga gcctttccag ttttcatcaa tgtcattgcc actctgctgt ccaccacaca 107820
gttgacctaa accctctgta tgctggaaga gctggacgag tcgaggaaga ggggagcgct 107880
gacatgtgta acaagttatt tacatgaaat gaagcctcat gtcctcccct acttcctcta 107940
gttctcaagc ttgagtctgc attagggtca tctagagtag tggttaatac actgtttgct 108000
accccctccc agcaacccca ccagagtttc tgattcatta ggtcaaatgg agactgataa 108060
ttagcgttgc taatgagttt ccaggtgatg ctgatgctgc tggtcctgga agcatatttt 108120
gaggatcaca gctctaaatg gtaactgaaa tgcaatcaca atacatagat caaagagctt 108180
tcaggaagga aactggcact cctttgacac cacatagcag agtatcctat atggatctac 108240
ctccacatct gttatatagg aaaattcctg aatgggttca gttaggacac aaaatataaa 108300
gggtttctat gcctaggaaa tataatttaa ctgaaatgtg tcataaaagc agctgcagca 108360
gcagtagctc tgataaaact ccttagttgc cattctcaga atgagcagta cgttagaagg 108420
aaagttattt gcaacaatat catgattcac ctcatgtgac ccacattccc tatttctgct 108480
ttactttaga tattccaaga agcagaggct ctcattgcat tcatggccta gtgtttagag 108540
atgcattaaa gccagggatg aaagcccagt gggacggtca gtgtggcttt ctctggtgcc 108600
cagcatggtc ctagagaatc attacacgtc caggctgaag ggacctggga gcagtggctc 108660
cagagcctgg ctacacagca cagtcacctg gtggactgta aaaatatgca ggttactggg 108720
ctcagcttca ggtataccaa agcaaaatct ctaacaggag gctcacaaac atgtattcta 108780

aaacctctcc aggcttttct aatgctattg gtccatggac tggctttggg aatcactgcc 108840
ttacagaaag cctaatagaa ctctcttgct ttccacatga agaaacagac cgaaagagag 108900
ccaggagctt gctcagatcc cacatggtat aagtggtaga accgggagtg gatgcgaagt 108960
cttgattttc aagggttgtg tgctcagtgg aattacaggc tttagagcca agttctaaga 109020
tgagttagta tttactcaag gaatggcctt tgcagttaaa acttacctgt agaatgtgtg 109080
attagtggga aacatgaaat attgggtgga aaatgcttat catattgcct gacacttggt 109140
aaatgcttct aaatggatgt catcattact gcattttact gaatttaagc aactcacgac 109200
tctaagatgc atcattactt tatgtgcctc taagaaagaa agaaaaacta ctgccagtta 109260
aactatgaca ctatttgctt gcgaagtgca tccagatttc agagatgtca gtacgtgaaa 109320
gaatgggcag tgtggtattt cacttctctt ttctcaactc tatgaagatt cttactttac 109380
tgaattagac acttaacagt acatgaaaat tcctgaagga gaagtatact tagtgtgttt 109440
gaggaacaac aaggaggcca gtgtggctga tggggaaagt ggggtaagga caaggtccaa 109500
gacagccagg ggatgtctga ttccatgtct tcactattgt gaatagtgtg aacagaagag 109560
tttcatgata tgactttcat ttcttttatg actacatagt attccatgct gtatatgtac 109620
cacattttct tttcttttct cctttgcttt ttctttcttt cttttttttc gtttcttttt 109680
ttttattaca gttttatttt ttatttattt tttttggaat aatttttttt tttattatac 109740
tttaagtttt agggtacatg tgcacattgt gcaggttagt tacatatgta tacatgtgcc 109800
atgctggtgc gctgcaccca ctaacgtgtc atctagcatt aggtatctct cccaatgcta 109860
tccctccccc ctcccccgac cccaccacag tccccagagt gtgatattcc ccttcctgtg 109920
tccatgtgat ctcattgttc aattcccacc tatgagtgag aatatgcggt gtttgggttt 109980
ttgttcttgc aatagtttac tgagaatgat gttttccaat ttcatccatg tccctacaaa 110040
ggacatgaac tcatcatttt ttatggctgc atagtattcc atggtgtata tgtgccacat 110100
tttcttaatc cagtctaaca ttgttggaca tttgggttgg ttccaagtct ttgctattgt 110160
gaataatgcc gcaataaaca tacgtgtgca tgtgtcttta tagcagcatg atttatagtc 110220
atttgggtat atacccagta atgggatggc tgggtcaaat ggtatttcta gttctagatc 110280
cctgaggaat cgccacactg acttccacaa tggttgaact agtttacagt cccaccaaca 110340
gtgtaaaagt gttcctattt ctccacatcc tctccagcac ctgttgtttc ctgacttttt 110400
aatgattgcc attctaactg gtgtgagatg atatctcata gtggttttga tttgcatttc 110460
tctgatggcc agtgataatg agcatttttt catgtgtttt ttggctgcat aaatgtcttc 110520

ttttgagaag tgtccgttca tgacaaaccc acagccaata tcatactgaa tgggcaaaaa 110580
ctggaagcat tccctttgaa aactggcaca agacagggat gccctctctc accgctccta 110640
ttcaacattg tgttggaagt tctggccagg gcaatcaggc aggagaagga aataaagggt 110700
attcaattag gaaaagagga agtcaaattg tccctgtttg cagacgacat gattgtttat 110760
ctagaaaacc ccattgtctc agcccaaaat ctccttaagc tgataagcaa cttcagcaaa 110820
gtctcaggat acaaaatcaa tgtacaaaaa tcacaagcat tcttatacac caacaacaga 110880
caaacagaga gccaaatcat gagtgaactc ccattcacaa ttgcttcaaa gagaataaaa 110940
tacctaggaa tccaacttac aagggatgtg aaggacctct tcaaggagaa ctacaaacca 111000
ctgctcaagg aaataaaaga ggacacaaac aaatggaaga acattccatg ctcatgggta 111060
ggaagaatca atatcgtgaa aatggccata ctgcccaagg taatttacag attcaatgcc 111120
atccccatca agctaccaat gactttcttc acagaattgg aaaaaactac tttcaagttc 111180
atatggaacc aaaaaagagc ccgcatcgcc aagtcaatcc taagtcaaaa gaacaaagct 111240
ggaggcatca cactacctga cttcaaacta tactacaagg ctacagtaac caaaacagca 111300
tggtactggt accaaaacag agatatagat caatggaaca gaacagagcc ctcagaaata 111360
atgccgcata tctacaacta tctgatcttt gacaaacctg agaaaaacaa gcaatgggga 111420
aaggattccc tatttaataa atggtgctgg gaaaactggc tagccatatg tagaaagctg 111480
aaactggatc ccttccttac accttataca aaaatcaatt caagatggat taaagattta 111540
aacgttagac ctaaaaccat aaaaacccta gaagaaaacc taggcattac cattcaggac 111600
ataggcgtgg gcaagggctt catgtccaaa acaccaaaag caatggcaac aaaagccaaa 111660
attgacaaat gggatctaat taaactaaag agcttctgca cagcaaaaga aactaccatc 111720
agagtgaaca ggcaacctac aacatgggag aaaattttcg caacctactc atctgacaaa 111780
gggctaatat ccagaatcta cgatgaactc aaacaaattt acaagaaaaa aacaaacaac 111840
cccatcaaaa agtgggcaaa ggacatgaac agacacttct cgtttctttt tttttgagat 111900
ggagtctctc tctgtcgccc aggctggagt gcagtggtgt gatcactgca cctccgcctc 111960
ccagattcaa gcaattttcc tgcttcagcc tcctgagtaa ctgggattac atgggcacac 112020
caccatgcct ggctaatttt tgtatttttg atggagacag ggttttacca tgttggccag 112080
tctggtctca aactcctgac ctcaagtgat ccgcccacct gggctgccca aagtgctggg 112140
attacaggcg tgagccacca tgcctggccc acattttctt tatcgaatcc actattgatg 112200

ggcacctagg ctgatttaat gtctttgcta ttgtgaatag tgtgagcgga agaatttcat 112260
gatttgattt acattctaaa agatacatgg gaaaaggtgg acaaagggag atcagttagg 112320
aaattattgc actattcctg gcaatggaag acagtgaatt agcttattat gatagtaaaa 112380
tatgtgagaa gaagtaggtg ggatatggga atatttttag ggtagccagt agaatcttct 112440
ctcagtggat ggactgaata cagggtattg aaggagtcaa gaatggcctc aagatttttg 112500
gcttgagtcg ttgaaagaat ggaacctgaa aaaaaaaaaa aagactgaag gatgagcttt 112560
ggagggagtc tgtattttac tcaatgttat ctttattcct tatttacatc tatctctaaa 112620
gtttacctct ttttttaaat aaatattttt aaatacctat tttctcccca atatgcactg 112680
agaaataaaa atgaataaga tacagtggct ttattcccct caaaattttg ccacctgcag 112740
gggagatagg actcacacgt ggtacattac aaagcagtat gtatctaata gtataagaaa 112800
gatatttaaa atgtgagaaa tcagagaaag gaggcattca ttgtaattgg ccctatcctc 112860
ccctcaaaat gcttgctttt gttgagtaca agttcattac attggaaaag gagaatgcta 112920
gcataataac tttaactagt tttttcactt tgggtaaact gcctccttgc tgtacctcaa 112980
accacctcca caacaagcat gctttttcct caagtttacc tttgcatttg ctgttcccct 113040
tgcataaacc atcatccctg caagagtcac ctggttgctg tcttccttca ctcaggtctc 113100
tgctccgagt caccttatct aagaagccta ccttgatttc tttatataaa agaatgccta 113160
ttccttttgc atactttttc ttcacagcac ttgccatctt ctctcacatt atatatttat 113220
ttgctttatt ggctgtttcc ccacatgaga atgtaaactc agtgacaaaa ggcactttgt 113280
tttgtttacc actgtattcc cgcaaggcca atgaattgct taacgaatta tttaataaat 113340
ataggaatta aaataaacca tgggtttgga aagcaataag caaatggggt tggagatagg 113400
actggactct gaacacagcg ttggtttcag aagagagaga agacactaga gaacagctct 113460
ctgactaagc tacatgcctc tgtggcagcc tggaatcttg ggacacacgg cttgcaccag 113520
gccactcttt tggttaaagc aaaggttaaa gtctggtgta ttgggacaaa ctttggatag 113580
agatacagaa tgtgatctac gtcaatccct actacttaat agctgtgggc cttctgccag 113640
tttcctcata caggatgagt atcccttatc taaaatgctt gggaccagaa gtgtttcaga 113700
tttcagaatt cttggacttt ggaatataat ataatataat ataatataac ataacataat 113760
acaatacaat acaatataat ataatataat ataatataat ataatatagt ataatgtaat 113820
gtaatgtaat ataatataat ataatataat ataatataat ataatataat ataatataat 113880
ataaatttac ccaggtgagc atcccaaatc cacaaatcca aaatccgaaa tgctctaatg 113940

agcatttcct ttgaacgtca tgctggtgtt caaaaagcat cagattttgg attttagatt 114000
tttggatttg agatactcaa cctgtatata ttttgagagt aatagtgtgt actctgccaa 114060
ctacataggg ttattgcaag ggtaaagcaa agataagcca tgggacagca ctttgcaaac 114120
tgttaagaat tctattatca gggtcaagat ggcaatcagg gacactatga catggactac 114180
agtcaaaaga tccaggaatg cctgccttgg acagctagat cttctcccca ggaggtgcct 114240
gagggagaca tacacacagg tgacagtcac aaagggtaag tgaagttcta cattattagt 114300
agctgggctt gggtcctaaa tgccaatgtg agcgttctga taaactgacc tgggcatgta 114360
gcccaggtta aaggcctcca gaagcctctc tttattcatt aagtcactgg tgacaaggac 114420
taaaacactt agcacactca gcgacatgta agatcagtgt tgaggaggga ttagagcagg 114480
cccctgagag ggccattttc tttgtgtgat tttcaaaaag tacattgatc tcatccattg 114540
gaaattttgg ccccattagc atcctgtgtt agtgtttcct catccttact cttcccttca 114600
atttcacttc tcagaaagtg gaatttgaga atttgttgct tcatccccaa acagttcaga 114660
tttgccctta agattcaagc ataatgatta cctgtggcac acattacctc tactggagaa 114720
attatacttt tgataagaat aggcaaagtg atattacatt catatgttca tctcttgatt 114780
caaaactgac ttggaaatca tagtggatgg tgtgttaggc tgttcttgca ttgttataaa 114840
tacataccca agactggtca atttataaga aaagaggctt aattagctca taattctgca 114900
ggctgcacaa gcatggcatt ggaatctgtt tggcttctga ggaggcctca gggagcttat 114960
aatcatcgtg gacggtgaag tgggagcagg catgtcacat ggcgaaagca ggagccagac 115020
agagagagtg gggtggcggg gaagggactg cacactctta aatgaccaga tcccctgtga 115080
acttagagcg agagctcact tatcaccaag ggcatggccc aagccattca tgagagatct 115140
tcccccatga tccaaacacc ctacctcctc tcccacccac cagcccccac ctccagcatt 115200
ggggattact tttcagtatg agatttgggt gggcacaaat atccaaacta tatctgatgg 115260
tgtattaatc cattttcatg ctgctgataa agacataccc aagactggga agaaaaaggg 115320
gtttaattgg atttacagtt ccacatagcc ggggaggcct cagaatcatg gcacgaggcg 115380
aaaggcactt cttacatggc agcagcaaga gaaaatgaag aagaagcaaa agcagaaacc 115440
cctgataaac ccatcagatc tcgtgagact tattcactat tacaaggata gcacgggaaa 115500
gacgggcagg cccccataat tcagttacct ccccctaggt ccctcccaca acatgtggga 115560
actctgggag atacaattca agttgagact tggtggggac acagccaaac catatcagat 115620

ggtgaacata gaagtgttcc tgtgggtata tggccatgtt tcttgccaaa acttcctgca 115680
tatgattctg gttaattgca gtttctggaa gtatcagcta atccttggcc taaagctatc 115740
tttccctttt ttagggtgag ataagactcc aggagttctc cttggtatga tacaagcaga 115800
agggccctct tgccttcagt tcttcatggt tgtagtggat atcctccttc ttcaagccca 115860
tgatgttcct tagggaaggt agttctgctg tttctgtgcc aagcctgggc attatgctca 115920
cgtgaaacct ttttcccttg atttttgcta cttctccagg gcacttcctg ggaatggggc 115980
ttggtaccta ttaggactat aaatttgcct ctctctgacc cctccaagct gagagaagtc 116040
tcgcctttct ccctgccacc tctactccac cctgcagtcc ctttctgagg gcaggctttc 116100
taggaagttc tttgtcttca tgtagtttct tttttcttcc ctgtaaacat aaatgtcccc 116160
tagggtgaat gaacataaag gcctggccac aggctttgaa aaaggaagtg ataatacaca 116220
aaaatcttca aaattatctt tctccagaga ttatttcaaa aacagtttta ttgaagtaca 116280
atagacatac aatagactgc acacattaaa aatgcacaag ttggccagtt ttgacttaca 116340
tataaacctg tgaaatcatc accataatca agataatgac cacatccatc actaccaaaa 116400
gttgcttgtg tccctttatg attcctctgg ccgctcctcc ttcccccagg aaacaactga 116460
tctctttctg tcactataga ttaacttgtt tttgttttct aaagttacat ataaatggaa 116520
gcatacagta tgtgcttttt tggtctgact ttttttatta agcataattg ttttgaaatt 116580
tatccatgtt gttgtagcat gcatcaatag ttcattttat tgctgaatag caaggcatga 116640
tctagggtag gacaagtgaa gtgcctaggg ttaaaatgca gtcagttcct tatgatgata 116700
atcataatga tgatgactgt aaaaacattt aatgagacct gctctctgaa caagttttta 116760
agtgtaatat accgtatcgt taagtatagg cacaatgttg tacagtggat ctctagaacg 116820
tattaatctt gtataaccga actttatatc cctcgaatat gaattcctca tttccctttc 116880
ctcccaattc ctggcaacca tcattctact ttgtttctat gatttaggcc atttttgata 116940
tgtaacgtat gtggaatcag gcaatattta cccttctatg gctgggttat ttcacttaga 117000
attatttcta ccgggttcaa ccatttagtc aaaaatgtta ggatttcttt ctttcttctt 117060
ttttgttctt ttcattatta ttttttatta tcattatact ttaagttctg gggtacatgt 117120
gcataatgtg caggtttgtt acataggtat acacgtgcca tggtggtttg ctgcacccat 117180
caaaccatca tctacattag gtatttctcc taatgctgtc cctcccctag ccccccaccc 117240
aatttctttc ttttttaaag ctgaataaga ttccattgta ggtgtatccc acattttctg 117300
tatctactta tctgctgata gtcatttgtg ttgtttccat gtcttggctt ttgtgaatgg 117360

tgctgcaatg aacatggaag ggcagatatc tcttcaagat gctgatttag atcctcttgg 117420
gtatatacca gaagtgggac tgctggagca tatggtagtc ctatttttaa ttttttgagg 117480
agcctccata ctgactgcat cattttacat tcccactaac agtttataag ggttccagtt 117540
tctctacatc cttgccaaca tttgttatct tttgattttg ttgatgatag tcatcctgac 117600
atgtataagg tgatatctca ttatggtttt aatttgcatt tccctaatga ttagtgatgt 117660
tgaacacctt ttcgtgtaac ttggccattt gtatgtcttc tttgaagaaa tgtctattca 117720
acttctttga ccactttaaa atcatatttt ttgtttccta tcaagttgta tgagttccct 117780
atatatcttg gatattaacc ttcaaatata taatttgcaa atattttctc tcattccaaa 117840
ggtcttttca ctttgttcat tgtttccttt gctgtgcgga agtttttgtt gttgttgttc 117900
aattgtagtt ccgcctgttt ttgcttttgt tgcttatgtg tttggtatca tatccaagaa 117960
atcattgctt agaccaatgt ttttttcatg tatcttcttc caggagtttt atacttttag 118020
atatcatgtt taaatcttta atccattttg agttgatttt tgggtacaat gtatgataat 118080
agtttaattt tattatcttg catgtggata tccagttttc ccaacaccat ttattaatga 118140
gatttttttt tttgcccatt gtttattttt gacactcttg ttgaagatca gttgacctgt 118200
gtgggtgact tccaggctct ctattctgtc ctattggtct atatgtctgt tttttcaggt 118260
accatactgt tttaagtaac tttctaaaat attttgaaat taggacgtgt gatacttctg 118320
actgatttct tctttttcaa cattgctttg gatattttga aactttttgt ggttccatat 118380
aaattttagg gtttaaaatt atttcaaaat aatttctgta acaaatgtca ctgagatttt 118440
gaaatgcaat cagttgtgaa tccgtttcaa ttgttctttt ttttcttaca atggtttcta 118500
tttttctgct ttgtcaaatg cctgataatt ttttatagta tgggacacac ttacacctta 118560
aaactttttg ttagatggct gaataggaac agctccagtc tacagctccc agtgtgagca 118620
acgcagaaga tgggtgattt ctgcatttcc agctgaggta ctgggttcat ctcactggga 118680
cttgtcggaa agtgggtgca gcccacagag tgtgagctga agcagggtgg ggcatcgcct 118740
cacccaggaa gtgcaagtgg ttggggaatt ccctttccta gccaagggaa gctgtgacag 118800
atggtacctg gaaaatcggg acactcccag cctaatactg cgcttttcca atggtcttag 118860
caaacggcac accaggagat tatatcctgc gcctggcttg gagggtccca cgcccacgga 118920
gccttgctca ctgctagcac accagtccga gatcgaacta caaggtggca gcgaggctgg 118980
gggaggggcc tctgccattg ctgaggcttg agtaggtaaa caaagaagcc gggaaggtcg 119040

aactgggtgg tacccatcac agctcaacca ggcctgcctg cctctgtaga cgccacctct 119100
gtgggcaggg cgtagctgaa caaaaggcag cagaaacttc tgcagactta aacgtccctg 119160
tctgacagct ttgaagagag tggtggttct cccagcatgg agtttgagat ctgagaatgg 119220
acagcctgcc tcatcaagtg ggtccctgac ccccgagtag cctaactggg agacacctcc 119280
cagtaggggc ctacagacac ctcatacagc tgggtgcccc tctgagatga agcttccaga 119340
ggaaggatca ggcagcaaca tttgctgttt tgcaatattt gctgttttgc aatatttgct 119400
gttctgcagc ctgcactggt gatacccagg caaacagggt ctggagtgga cctccagcaa 119460
actccaacag acctgcagat gagggtcctg actgttagaa ggaaaactaa caaagagaaa 119520
ggaatagcat caacatcaac aaaaaggaca tccacaccaa agccccatct gtaggtcacc 119580
atcatcaaag actaaaggta gataaaacca caaagatggg gagaaaccag agcagaaaag 119640
ctgaaaattc taaaaatcag agcacctctt ctcctccaaa ggaacacagc tcctcaagag 119700
caatggaaca aggatggacg gagaatgact ttgacaagat gacagaagta ggcttaaaaa 119760
tatcagtaat aacaaacttc tccaaggaag tttggaggat gttcaaaccc atcgcaaaga 119820
agctaaaaac cttgaaaaaa gattagacga atggctaact agaataaaca gtgtagagaa 119880
gaccttaagt gacctggtgg agctgaaaac catggcacga gaactacgtg atgcatgcac 119940
aagcttcagt agccaatttg atcaagtgga agttcaagtg aatgaaatga agcgagaaga 120000
gaagtttaga gaaaaaagag taaaaaaaaa tgaacaaagc ctccaagaaa tatgagactg 120060
tgtgaaaaga tcaaatctac atctgactgg tgtacctgaa agtgacgggg agaatggaac 120120
caagttggaa aacactcttc agggtattat ccaggagaac ttccccaacc tagcaaggca 120180
ggccaacatt caaattcaag aaatacagag aacgccacaa agatactcct cgagaagagc 120240
aaccccaaga cacataattg tcagattcac caaggttgaa ataaaggaaa aaacgttaag 120300
ggtagccaga gagaaaggtc aggttaccca caaagggaag cccatcagcc taatagcgga 120360
tctctcagca gaaactttac aagccagaag agagtaaagg ccaatattca acgttcttaa 120420
agaaaagaat tttaacccca gaatttcata tccagccaaa ctaagcttca taagtgaagg 120480
agaaataaaa tcctttacag acaagcaaat gctgagagat tttgtcacca ccaggcctgc 120540
cctacaagag ctcctgaagg aagtgctaaa catggaaagg aaaaaccagt atcagccact 120600
gcaaaaacat gccaaattgt aaacaccatc gatgctagga agaaactgca tcaactaatg 120660
agcaaaataa ccagctaaca tcataatgac aggatcaaat tcacatgtaa caatattaac 120720
cttaaatgta aatgggctaa atgctccaat taaaagacac agactggcaa gttggataaa 120780

gagtcaagac ccatcagtgt gctgtattca ggagacccat ctcatgtgca gagacacaca 120840
taggctcaaa ataaagggat ggaggaagat ccaccaagca aatggaaaac aaaaaaaggc 120900
aggggttgca atcctagtct ctgataaaac agactttaaa ccagcaagga tcaaaagaga 120960
caaagaaggc cattacataa tggtaaaggg atcaattcaa caagaagagc taactatcct 121020
aaatatatat gcacccaata caggagcacc caaattcata aagcaagtcc ttaaagacct 121080
agaaacaaag agacttagac tcccacagaa tcataatggg agattttaac accccactgt 121140
caacattaga cagatcaaca agacagaaag ttaacaagga tatccaggac ttgaactcag 121200
ctctgcacca agtggaccta atagacatct acagaactct ccactacaaa tcagcagcat 121260
atacattctt ctcagcacca catcatactt attccaaaat tgaccacata gttggaagta 121320
aagcactcct cagcaaatgt aaaagaacag aaactataac aaactctgtc tcagaccaca 121380
gtgcaatcaa attagaactc aggattaaga aactcactca aaactgctca actacatgga 121440
aactgaacaa cctgctcctg aatgactact gggtacataa tgaaatgaag gcagaaataa 121500
agatgttctt tgaaaccaat gagaacaaag acacaacata ccagaatctc tgggacacat 121560
tcaaagcagt gcatagaggg aaatttatag cactaaatgc ccacaagaga aagcagaaaa 121620
gatctaaaat tgacactcta acatcacaat taaaagaact agagaagcaa gagcaaacac 121680
attcaaaagc tagcagaagg caagaaataa ctaagatcag agcagaactg aaggagatag 121740
agacacaaaa aacccttcaa aaaatcaatg aatccaggag ctggtttttt gaaaagatca 121800
acaaaattga tagaccgcta gcaagactaa caaagaagaa aggagagaag aatcaaatag 121860
atacaataaa aaatgataaa ggagttatca ccactgatcc cacagaatta caaactatca 121920
tcagagaata ctataaacac ctctatgcaa ataaactaga aaatctagaa gaaatggata 121980
aattcctgga catatacacc ctcccaagac taaaccagga agaagtcgaa tccctgaata 122040
gaccaataac aggctctgaa attgcggcaa taattaatag cctaccaacc aaaaaaagtc 122100
caggaccaga tggatgcaca gctgaattct accagaggta caaagaatag ctggtaccat 122160
tccttctgaa actattccag tcaatagaaa tacagggaat cctccctaac tcattttata 122220
aggccagcat catcctgaaa ccaaagacta gcagagacac aacaacaaca aaaaagagaa 122280
ttttagacca atatccctga tgaacattca tgcaaaaatc ctaaataaaa tactggcaaa 122340
ctgaatccag cagacaacaa aaagcttatc caccacaatc aagttggctt catccctggg 122400
atgcaaggct gcttcaacat atgcaaatca ataaacctaa tccgtcatat aaacagaacc 122460

aaagacaaaa accacatgat tatctcaata gatgcagaaa aggccttcaa caaaattcaa 122520
cagcccttca tgctaaaagc tctcagtaaa ctaggtattg atggtaagag ctatttatga 122580
caaacgcaca gccaatatca tactgaatgg gcaaaaactg gaagcattcc ctttgaaaac 122640
tggcacaaga cagggatgcc ctctctcacc actcctattc aacatagttt ggatgttctg 122700
gccagggcaa tcaggcagga gaaagaaata aagggtattt aattaggaaa agaggaagtc 122760
aaattgtccc tgtttgcaga tgacatgatt gtatatttag aaaaccccat catctcagcc 122820
ccaaatctcc ttaagctgat cagcaacttc agcaaagtct caggatacaa aatcaatgtg 122880
caaaaaatca caagcattcc tatacaccaa taagagacaa acagagagcc aaatcatgag 122940
tgaactccca ttcacaattg cttcaaagag aataaaatac ctaggaatcc aacttacaag 123000
ggatgtgaag gacctcttca aggagaacta caaaccactg ctcaacaaag taaaagagga 123060
cacaaacaaa tggaagaaca ttccatgctc gtgggtagga agaatcaata tcgtggaaat 123120
ggccatactg cccaaggtaa tttatagatt caatgccatc cccatcaagc taccaatga 123180
ctttcttcac agaattggaa aaaactactt taaagttcat atggaaccaa aaaagagccc 123240
gcattgccaa gacaatccta agccaaaaga acaaagctgg aggcatcaca ctacttgact 123300
tcaaactata ctacaaggct acagtaacca aaacagcatg gtactggtac caaaacagag 123360
atatagacca atggaacaga acacagccct cagaaataat accacacatc tacaaccatc 123420
tgatctttga caaacctgac aaaaacaagc aatggggaaa ggattcccta tttaataaat 123480
ggtgctggga aaactggcta gccatacgta gaaagctgaa attcgatccc ttccttacac 123540
cttatacaaa aattaattca agatggatca aagacttaaa tgttagaccc aaaaccataa 123600
aaaccctaga agaaaaccta ggcaatacca ttaaggacat aggcatgggc aaggacttca 123660
tgactaaaac accaaaaaca atggcaacaa aagccaaaat tgacaaatgg gatctaatta 123720
aactaaagag cttctgcaca gcaaaagaaa ctaccatcag agtgaacagg caacctacag 123780
aataggagaa aatttttgca atgtacccat ctgacaaagg gctaatatcc agagtgtaca 123840
aagaacttaa acatttataa gaaaaaaaat caaacaaccc catcaaaaaa tgggcaaaga 123900
atatgaatag aaatgtctca aaagaagata tttatgcagc caacagatac atgaaaaaat 123960
gctcatcacc actggtcatc agagaaatgc aaatcaaaac cacaatgaga taccatctca 124020
caccagttag aatggcaatc attaaaaagt caggaaacaa caggtgctgg agaggatgtg 124080
gagaaatagg aatgctttta cgctgttggt gggagtgtaa actagttaaa ccattgtgga 124140
agacagtgtg gcgattcctc aaagatctag aagtagaaat accatttgac ccagcaatcc 124200

cattactggg tatacaccca aaggattata aatcatgctg ctataaagac acatgcacat 124260
gtatgtttat tgtggcgcta ttcacaatag caaagacttg gaatcaaccc aaatgtccat 124320
caatgataga ctggattaag aaaatgtggc acatatacac catggaatac tatgcagcca 124380
taaaaaagga tgagttcttg tcctttgtag ggacatggat aaagatagaa accatcattt 124440
gagcaaacta tcgcaaggac tgaaggccaa gcaccgcatg ttctcactca tgagtgggaa 124500
ttgaacagtg agaacacctg gacacagggc ggagaacatc acacactggg gcctgtcgtg 124560
gggtgagggg acgggggagg aatggcatta agagaaatac ctattgtaaa tgacgagtta 124620
atgggtgcag cacaccaaca tggcacatgt atatatgtga caaacctgca cattgtgcac 124680
atgtacccta caacttaaag tataataaaa aaaaaagaga aaagaaaaaa aaaaactttt 124740
gttgcttatc cgaaatttaa atttaactag gtatactcta ttttatctgg caatcaggtg 124800
tcctgagact ctagcttcct ttacttctct ggactcccat ttttgtctcc tcaactcagg 124860
aaattctcca ggctctgcct gagttcccac tcattgtgtt gtatcctgga cacactctca 124920
tgccagtaag ctgatgccac cgtaggggtc aacttttttg tttcctgact ctcagattgt 124980
tgtccttcgt tgtcagatgc tcattatctt aaaacataat ttttcatata ttttgtcctt 125040
tttggttgtt tcacactgga gagcaaatct ggtctctgtt actccgtctt tggttgaaag 125100
tggaagtctg tatccagaag ttattgttta tagccaggag ccagaacatg gttctaacca 125160
gggccaaaaa tgtgtgtttg atcccttcat aggtgagtgt gtagtttctt tatagttttt 125220
atgcaattaa ctactgaaaa aaataattct ttgggtcata aaggcgcctg gtgattatga 125280
gggttcctca ctactgtcag gaaattgatt agaagtaaac atcttctaat aagagaatgg 125340
gatgggccac tggggttatt accaagtaca aagcataaat tggcacatta cattgtatta 125400
cattgattgt cagagtttga accaaaatat agatttggct gggggtcaaa tctcggaggc 125460
ccaaattgta tttggagtca gggccaggaa gacaagtctg atagagtcag ggtgatcaaa 125520
ctggggccat agaggaattt aggaagatgt aaattctctt attccttgtt caatattcag 125580
tgatattctt cctagggggc ttggaaataa aaataaaacc atgtcctctc ttttctcttt 125640
ggtttctctt ggtctagcta tatgccgcta tcctctgggc atgtcaggag gccagattcc 125700
agatgaggac atcacagctt ccagtcagtg gtcagagtcc acagctgcca aatatggaag 125760
gtgaggatgg ttacatcaag aaagcccatg ttctggggtt gggcagattt cctgggcaca 125820
gattcctgac cctaggggct ggggaaggtg gtgttattgg caggaactgt tgagaaatga 125880

aggacagaca gcaagtgggc ctgtcctgct ctgtatgttt tcagggagag aatttaggac 125940
ttagccacat gtactggtct ggcagcccca tctcaaccac agaggcccca gggccacatt 126000
acccacagtg aatgggttca ttgataggaa gtggatttgc tttccctgac tttctcattg 126060
tgagctgctg caggaggcag gcaacagtgg cacctatggg aaggtgcaga ttatgtagga 126120
cactgggagt tagcatccac tcctcttctc agtgggtcta tgaaacatca ctcactgcaa 126180
agcatcactc actgccaagc atcaagacac tgaggcctcc aacaagttct ccacctctct 126240
caaggatctc ttacacagaa gggccatcct tgccaacaga cagagtgtgg cagtttcctg 126300
gggcgattga tttcaggggt cttgtacatc aataaactcc ctactgctct actggttgga 126360
cccaatactc tacctggaat cttattattt tgtgaagtat tgattttatt aaaatggaat 126420
tacccataag aaaatgaatc ctgatgaacc tttcccctta atttcttagt gtatgaaaga 126480
ctagagtgat ggtaggtgga attggaaggg agcaacaaat gaatgggtaa tatctgtgag 126540
aaaagggagc aatccaaggg agaggaaaag tggtagggag ttctcaaagt aaactttata 126600
ataatttcac aattttgctc agtggtagct cagcctgaag gtaataaaat gaaaccagat 126660
tatttactac ttccttgaca tgctcttccc cctcatcctc cccatgtgtt tctatggctc 126720
cttgctggtc tgcaatgccc atcctgctgc attgcataac ccacactgga gaccctacta 126780
tccctcctcc ctattgccat aacttgatgt ccaagcaaag ggtggctagc tttgttaggc 126840
tgagtttgaa agagggaagg gaaatatgaa atgactcatg cgggaacaga gagtaggaag 126900
agttttattt ctctttatgc ttctaaatca ggatgggaag tgaaggctat gtgtgatctg 126960
aaggcctcag ctcactgtct ttcagggtct catggtactc cagggaaagg gatctgacca 127020
caaatgggta ctgtggtggc agtgaaaact gtggcaagaa cccaaaatgt ttataaggac 127080
agcctgctct ctcccctcag tacagggcag ctgcttgcct gtgaaccagt aaacagctct 127140
gtggtttcat ggttgctccc tctctcccca accctcacct ctcaaggctg gactcagaag 127200
aaggggatgg agcctggtgc cctgagattc cagtggaacc tgatgacctg aaggagtttc 127260
tgcagattga cttgcacacc ctccatttta tcactctggt ggggacccag gggcgccatg 127320
caggaggtca tggcatcgag tttgccccca tgtacaagat caattacagt cgggatggca 127380
ctcgctggat ctcttggcgg aaccgtcatg ggaaacaggt aggaagaaga gacatccaga 127440
tcctggatgt ccaagaccat attttgactt aggctaggag aagaagggta caggttttct 127500
gtgtggatat gtgtgtccac agaccaggca tgcccttgta aatgtgtgac ccagggtgag 127560
ggaggcaggg aaggaatatc aaggctgtgg ggaaagcaga gtagatgcat tctgctcctc 127620

gaattaagaa gagagagtcc atcaaaaacg tggtggggtg aagaaaagtg agcatgattt 127680
aataccacct cttcactcat tctcttctct ctcctcaggt gctggatgga aatagtaacc 127740
cctatgacat tttcctaaag gacttggagc cgcccattgt agccagattt gtccggttca 127800
ttccagtcac cgaccactcc atgaatgtgt gtatgagagt ggagctttac ggctgtgtct 127860
ggctaggtag gtcactagcc caggaagcct atagacttta ttaaaaactc caacttctgg 127920
gaaatgaagg caatcaaatc agaaagggat gggatcagtt actcctggaa tggtgatgcc 127980
ctcttgggaa gaatagagac agaagcattg ctcattaacc cagggcttag ggccccacac 128040
tgagtcatgc attcagagtc attacgttga ctgccatgga ggggcactcc tccaaagtct 128100
tccctcggtg tccagatgga gtcctgctgg acacgctgtg caagcttata cccttgaaac 128160
tcacatagtt ccctgaggta atgggcctga gcagtaggca ctcacttggc tgtgtttcct 128220
ttgcagatgg cttggtgtct tacaatgctc cagctgggca gcagtttgta ctccctggag 128280
gttccatcat ttatctgaat gattctgtct atgatggagc tgttggatac aggtaaatcc 128340
tgggaaactt tattagaatg ggaaattggc cactaagaaa gaataatatt ttatcatgtc 128400
ttgggatcaa taatcaatca accaatcagt atttatttag ttcttgtaca aggcatttgg 128460
aaataattta gatgaaggaa tacacatgac ctttatttcc agggaactta aagcccggtt 128520
tgggaagaca agttaattac ccagaaaaca atcaattaca caaagtaatg gctaaagttt 128580
gtgatatggc tctaagtata atagccatat ttttgaccct caaaacagga catgtagtct 128640
gacacaaata ttgacctact tatggaaaca aagggccaga gaattgaagt aataagtata 128700
atactctggg caagtcaaga agtcagaaag aatagacctt atacatataa atgctttgaa 128760
atttgtaaaa aggggactag taagacttac aatagacaag gaaagttcta ggttaagatg 128820
agaattgtag cagaggaagg taggtagaag gtagcagaag aaaggaagca gggctgaagg 128880
caagactgac ctggctatag tagaagctgg agttgagaag aacagaatgg aacaatggat 128940
aagcctgaag tggatcaaag gagtgccatg gcaaagaagt gtgaccagat atagtaggaa 129000
cttagggttg ggtacaggat gctcccacct atactgtttc acttcaagcc tttaagcctt 129060
gccattcttc tagcccacac taacactttt ctctctaagc ttggtctgca tctgcaaagt 129120
gagcctggga gagaattcag tccatcgtga agaccagacc agatgatgga ggttgacagg 129180
gctcgagaga aggagatact agtgagtctc aggaagggag tgtggaatta cagccttcta 129240
ctggcttagg tgtggagaat gaactattgc cctctagctg ccatcccctg ggtgaagacc 129300

tgattcatcc actcttctac tcctagtcac ttcctttcca tcttcttaat cactgttctt 129360
tcctttttgc ttaaacggtc ttggcataat ttcgctcagt cacaccccaa caggcatgta 129420
ttcaaggcac agaggaaatc agagatcaac cgaattcagt ttaatttaat aagcctttgt 129480
taatcaactg ttactatata caaggaattt ggccaggcat gttaggtatt caaaggtaat 129540
ggagtcattg atcctacctt taaagtgctc acaatttgga gtaggggcta aaacagaata 129600
taagttacta tgattcaagt atatgagcaa gagatagcct atgactgctc tggggttcaa 129660
aggaaaaagt aaactatagt aggaaagcac aaaaatgggg atgaaaacat ctggatttca 129720
tataataaaa gttacttctg tttgctacaa ggcttaaggg attaaaatgt gtgtgaaaag 129780
catttcgtaa gataaattgc tatgcacata taagttgtta tctgattatc atttttctga 129840
ctgaaggacc agtaaagata tggtagaaca gatgaaatct gaactgggtc ccaaagaatg 129900
ggtagtactt tcacaggtgc agaatcttga aatggaattt taggcagagg gaataatagc 129960
atgaggaaaa aagaatcatg gttataaagt ttaggggact gtctgggaga aatcaacagt 130020
ttatttaacc acgggccata tttatgtggc aaagtagtgc aggagaagca aaacaaagca 130080
gattatgcca tattttgaag gaacctgaag tcaaagccaa aaggagcaag aagtggagga 130140
ggaggaagag gagaaattgc tgttcttatt cttcataaac attatcatta atgtaatgag 130200
tcattgaaga tttttaaagc tgaggaagga catgattgga gctgtgcttt agacagatta 130260
atcaagcagc atgcaaaggt tagattggaa tggaatgagg aggctatgag gaacatttta 130320
aaactcttag ttctgccaag taaaaagaat taatgaccaa ggccataacc gtgactgtgg 130380
ggaatgagga aaagatattc atttaggatg tttgaaatct gattggatgt atgaggcaaa 130440
acagtagggt gagaaaaagg gtcagatgaa gtttgacagt ctaagtgact gaaacaatgg 130500
ggatgacatt tatttattcg acaaatatat tttgagtgct taccttgcgc caagcatttt 130560
ccattactat gaagagaagt ataatttatg gaaaagggaa accaaggaga aagacttagt 130620
tcagtagggg atgatgatgg atttagtttg gagtgtaggt ctgtgagact gagaagacat 130680
tcagtagaga aataaggaag gtagccggaa ttatggctct taactccagg ggaaagataa 130740
aaaaaattac aaaaatgtag acatcattgg ctttcattta ataattgaag taatgaaata 130800
tctggtggag gtggctgagt gaagagatcc tctcagccac tgagagatga catcagagtc 130860
tcaggaaata gtcatattag caaaagttac tttttaaatt taaaacaatt tcaagaataa 130920
tatatgctta ctggtgattt taataactct aaagtatata aatattgaac atttattttc 130980
ttatcccttc ttcacttgat ttaattttga ccaaggggga actgaacagg cattcctttt 131040

aatttctgtt gcttttcata aaactttcct gggatttatt tttctgagga taatgggaaa 131100
tatatttatt gcaagtaaac ttacgttctc ctagctttct gatctgtaga gagaagaaaa 131160
agatggcaaa accttggaag ggagatccag gctctggtca ctgtggataa ggccagtcaa 131220
attttataat ttaccaaaca ttttcaatga ttaaatatat tttaaaaggc tggcataatc 131280
acccttagta tttttttatt ttttgcgatt cacaaatttt acatattatt gatctagctc 131340
taaacctcaa gggtcacacc aattattgct tgagggtgag tatcccaatc tcgtcatttg 131400
ggaaatttgt gtcatatctg ccagagcttg agatgcagag cttgaggaaa gagaagttcc 131460
acatgatggt ttttgctctg tccctccata aaaagaagga aggagcctct tcgtgttctc 131520
taagtagtca aataagcata catgcagtta tattccatcc agcagtcagg cttctggaaa 131580
actcagcctt taaaacagag cctcttactc agaagtcaaa aaagcctgct aagctagaac 131640
acaaaactaa atccaatacc tgggattctc atgaaatcct tgcatcttac ttcctgggtc 131700
tagtatccta caaatttaat tagaattaaa tagaaattat tatttaaatt tagagcttgt 131760
attttccccc ttagtttaat atcaaactgg gttgttggat atgtggaata gaagagagtg 131820
tgactggatt ctctgaaatt gattcactga acaatgacaa tatgtagtta aaggagagtc 131880
agcaccaagc tctggaaaca tttatatgat agtggggcaa gtgctactat gtatggtagc 131940
attcattcta tgcatttatc catttcctca tttactctat gaattagttc atcaaatgca 132000
tttgataata ttaagtcatt atgtgaagac atattcagta tataagaata ggcaaggtgc 132060
aataaattat agaaagcttt tcatattaaa taagacattg tttaaaagtt ttactactaa 132120
aggagtaagc atcaaatatt ttataaaaat tactaatatg aaattcttag ttctccattg 132180
atgcaagata aacgagtcaa tactataatg caaatctgat tatttacaat ccttcaattc 132240
caagataata agctcataca aaatctggag tgaagatgcc gggtaaaagc tcttccacga 132300
atgtgtggtt aactcagatt tctctctcct tttcctcctc ttctcctggc ctgagcagca 132360
tgacagaagg gctaggccaa ttgaccgatg gtgtgtctgg cctggacgat ttcacccaga 132420
cccatgaata ccacgtgtgg cccggctatg actatgtggg ctggcggaac gagagtgcca 132480
ccaatggcta cattgagatc atgtttgaat ttgaccgcat caggaatttc actaccatga 132540
aggtcagtgg ggtcgggtgt ggtggaactt ctttaaggag gcacaaatca tagtgtggta 132600
gagaaaaggc atgctagaaa aaagtctagg ctgatgccag ttcaatgaca tattctgtta 132660
ctaactagct aaatgcattt cacaagtcac ttcatctcta agggcgtgat tctccatttg 132720

tgtgtaaggg agttagaata ggtcagtgag ctctcttttt tttgttgttt tgacactaaa 132780

ggtgacatat ccattaagcc aataacacag tgtccagggc ccacagattt ttaagacgca 132840

taaaaatgct ctaatttctt ttaatatcag gtgaaaaaaa taaattttta gggtcaaaga 132900

ttatcttttt accagcacag ttgtgtgtgt gtgtgtgtgt gtgtatacac acacaactat 132960

atatatatac acaatgatat atatatatac acaactacat atatataacc atatatacat 133020

atacaactat atatatacat atacaactat atatgtatat acatatacaa ctatatatat 133080

gtatatacat atacaactag atatatgtat atacatatac aactagatat atgtatatac 133140

atatacaact agatatatgt atatacatat acaactagat atatatatgt agttggtgtt 133200

ctgcagtctt ctgaattttt ccttctagga acctcacagt ggcagtgata gttggtcagg 133260

ccttataaaa cctgggctgc aaccctggtt acctgtatgc actgtctgcc cttggccacc 133320

tcacttcatg ttgctggact gcagttttct catctgttaa atgaggactg caccagcatc 133380

ttctcagccc ttccttgcct tgaactctct cgagtctata atcctctcag ccgcagttgc 133440

cgtctaccct gattcgtgtg tgtatctata cacatagata catacacaca tatgtgagaa 133500

tatgtgtatt ctcatttaga aatgagattt ctaaaaaaaa aaatgaaatt gttttagatt 133560

gtacactatt ttgggtttct cacctagccc tgtgaaaaga agaagactgg ctctgaaatg 133620

tacctaggag gaagcaggat ggcagtcttc ctcttacctc agttcagaat agctcgaatc 133680

agggtagacg gcaactactg agttggctgg cactgtgtct ccatgcacct tagcagggcc 133740

aacctatcac tcacatgcct ctttctctac caggtccact gcaacaacat gtttgctaaa 133800

ggtgtgaaga tctttaagga ggtacagtgc tacttccgct ctgaagccag tgagtgggaa 133860

cctaatgcca tttccttccc ccttgtcctg gatgacgtca accccagtgc tcggtttgtc 133920

acggtgcctc tccaccaccg aatggccagt gccatcaagt gtcaatacca ttttgcagat 133980

acctggatga tgttcagtga gatcaccttc caatcaggta gggagctcag gtcctctttg 134040

ggaagtggga gcaaggtgat gaaggaggaa tgcacatgcc cagaacttct cattagcagg 134100

tctctgagag gagtgggatt gtacaggcag cttctcattg acggatacgg tgaatccttt 134160

gtgtgactag ttttaactgt ggaggggatg ggatgagaat ggaagtgaac aaaatatgca 134220

cgtaactctt cttagaaaga atgtttttaa atgagagtac agtgtgttaa aaattttata 134280

taatttaagg tagttaacga gtgcgctgtt aaggaaaagc acacctaagc attactgaga 134340

tatttaaatg aaaatttcta aatatacaca aaagtaaaga gaacaataaa ccactcatca 134400

ctcagcttta ctaatcattg atattttgcc atatttgttt cacccattcc ttccttttgg 134460

ctgtagcatt ttaaagcaaa tatgagatac tacttgattt cattagcaaa taattggact 134520
gcatctaaga aaatagcatc attttcatag gtataatgcc atgataacat ctaacaacct 134580
ttattattaa ataatttctt aataccaccc aatatcctgt ccatattcaa gtatgactaa 134640
ctgtccatca atgtcttaat tcagttgact ttatcaaata aggatcaaaa taaggatctg 134700
ttggtgagtt tctttattct atttgctctg tgtctctttt aatctaaaaa attccttcca 134760
tttttatttc tttttaaatg gattctggct tggctgatgg ctttctgtgg tgtcatttta 134820
cttgagcttc tgttctctgt cattcctaat gactggtgct tacatctaca ggttaatttg 134880
attccagttc aatatttatt tttgtcaaga attctttata agcattctat atatctcagt 134940
aatgctgtat attttatgct gaacttcatc atggggatgt aatgctagat cctctcactt 135000
tttgtgatac taagattgat ttctggttcc tcatgaactt tttccctaag ttttatcctc 135060
catcaataac cactgtctga attatttatt tcactagggt tgctaaatga cgattttcta 135120
attcttttat tttttctgca tttcttagct gaatttcttc cgaatagagt aactattctt 135180
catcatgcac cagtttactc agaaatcaca ttgaaagagc agcatacgtg acttatcttc 135240
ccctttaatt gttaattttc agagtaatga cttgataccg tagcaaccct caataatgtc 135300
taattaattt tggtttttac ccttattttt ttggtatcat tataaactca ttgcttttta 135360
tcaatttaat gcttaatcga ttgcagttat tattattagt agtagtatgt ttatttttag 135420
acagagtctc gctctgtcac ccaggctgga atgcagtggt gtgatcttgg ctcactgcaa 135480
cccccgcctc ccgggatcga gtgattcttc tgcctcagcc tcccaagtag ctgggattac 135540
aggtgcctgc cactgcgcct ggctagtttt tctatgttta gtagagatag ggtttcgcca 135600
tgttggccag gctggtctcg aactcctgac ctcaagtgat ccgcctgcct tggcctccca 135660
aagtgctggg attacaggca tgagccaccg tgtctggcca ttattctttt taatactcaa 135720
gttgtgctgt ctttgactag agtttgcatt tttttttttt tgagacggta tcttgctctg 135780
ttgcccaggc tggagtacta tcttgagtca ttgcaacctc cacctcccag gttcaagtga 135840
ttctcctgcc tcagcttccc aaatagctgg gattacaggt gcctgctacc acgcccggct 135900
tttttttttt tttttttttt tttttttttt tttgcatttt tagtaaagac ggggtttcac 135960
catgctggcc aggctggtct cgaactccca acctcaggcg atccagccgc ctaggcctcc 136020
caaagtgctg ggattacaga tgtgagccac tgcacccagc ctagagtttg catttttaag 136080
gggctctttt gctcttttga tctgacccta gaagcctttg ataactttct tgttttccga 136140

aagaacaagg tatttcaagc tcattttgaa catgtacagc acccgacgtg gaatcaacca 136200
tttcaagaaa tcttgattcc ttttagtagg aaatggtatt tagaaaacac aaactgggta 136260
gtagggatgc tcattactaa taggttgtca ttgtttcttg gatttttcgg tggaagaaag 136320
caatgaaata tgtatattta aaaagaaaga ttgtgagttt acattgttac ttccaattca 136380
aattaaagat aaggttttta tttaaagttg attttatacg ttcatctcta ttatttatgc 136440
taaatatcat gtttcctaac aatgttaaca gcattttctt catcctatta ttcaaaaagc 136500
aataccaata ttattactag caacgtaact attgaacatt aagatttctt agcaattctc 136560
ttttctttgt cctgagaata tatcctacta agaatgtgca gtcaaagcac tatgttctaa 136620
ggtcagttgg gtaacagtgg gataattctt ttctgtatat agttatatca ccagtgtggt 136680
atacagttag ggtaacagtg ggataattct tttctgtatg tagttatgtc accaatgtga 136740
tatacagtta ggttcattgt attccatttg tttcaacctt tagggattcc ttttaaactt 136800
taaacactac ttttgattga acagtaactg cagcaaatag tgatttaact tactacagcc 136860
agaaactgtg ccaaatcttg aaaagttaaa aggtgtttag aacattgttc ataccattaa 136920
atttaaatgg gaagatgaaa gatacagaat gctttaaaaa aaaaaaaaaa aaaagaaatg 136980
tccaaagcaa tatgaacaat aaatggtcag gaggagagag gcactagcgt gggtttgtca 137040
tagacagcat caaaggaatg agacttgtac tgggcctgga agaaaagtcc aggtgtaaaa 137100
taacataaaa gagaggaaga gactaattta aagataaacc acactttggg aggccaaggc 137160
aggaggattg cttgtattca agagtttgag actagcctag gcaacatggc gaaatcctgt 137220
ctctataaaa aacacaaaaa ttagctgggt gtggtggcac gtgcttgcag tcccagctac 137280
ttgggaggct gaggtgggag gatcacctga gccctggagg cagagcttgc agtgggtcga 137340
gattgcacca ctgcactcca gcctgggtga cagagtgaga ccctgtttaa aaaaaaaaaa 137400
aaagaaagaa aagaaaaata aatcagaaga acccatcctt tcagatttga tttttaaggg 137460
aaaaaaattt caagatggtt tgcttattaa gatgtactga taagaaaata gagcatattt 137520
ttaggaaaac aggtttggtg tttaaatgcc ttctgtgcta atgatttaat ctaagagtaa 137580
gttgattgat tgtttcatct gaatactgag agtagaaatc aagatgctca aagtcagtgc 137640
cacttacact gtaagtgtta actgggggac aaatagattc ccacccatcc tcagggattc 137700
tcctggcata gtaactggta cacttattat agctaattat agctatgaac catggttttt 137760
actcctgggg tctcttgagc tttaaatcca atgcataata atattagttt tcaccggagc 137820
ctgacagccg aactccgaaa gcacatgcag aggccagata cctccttatt tatataataa 137880

tattaagaag aagaaaaatt aggatacctg gaagaaatgt ctcttagaaa ggaaatacat 137940
attctaattc ttgaccccct tgattagaca aatcagaggg aaaaactctg gcaaagctgc 138000
caacatcaat ctggcaaata cgcttttgtg gtttagtctc aaaagctgta aaatcacaga 138060
aaaagacact ccaatgtcag taaatatcta taaagattaa gccttaattc ataaattgaa 138120
gggctaggcc gatgaggcat agtggagact cttttataaa caatctaata tccttgaagg 138180
aaaaagcatc ttaacactat gtctgtccct tgcccattta cccgctatat tctgtctata 138240
ttttcactga atttgggtcc tgcttcttat gctttatgac aatgtgtggt acagcccttt 138300
gaatcactgt ttcttaaact tttttgtacc atagcatcta gtatgtaata tgtgaaacaa 138360
aaattttaca aaggaaaact tgcttttact atttagataa atggtgattt tttattcaac 138420
gtatttatga ttttatcttt ttaaaagact gatgacaact cactaaattg atcttgtaat 138480
gtgctaggtc acaatatgcc ttcagagaaa acactagctg tctgtcttct ccctggctct 138540
gactcaccct tgttttataa cagatgctgc aatgtacaac aactctgaag ccctgcccac 138600
ctctcctatg gcacccacaa cctatggtat atgtgattcc taattacaca aattaatttg 138660
aagggactta ctgggggatg aagaagggtg gagagttgca aagccctggc tgtgtgggag 138720
gctttacacc agttggcttt ggatgaagag tagccctgga aaggtaggac tgtctacctc 138780
caagttatgg atccaaatat actctcttca ctatggattc atgtgcttct tcagctgagg 138840
cagagtctgg agttgtccta atttctccat gaaagtttgg gaatttgggg tcagaaacag 138900
ggaaagggac attcctgggt cagacccata gtgggtattt ctaaggaggg gtggaaatca 138960
aagaaacaaa atgctgatat ctccaattgg agataaatat gccatgtatg cattgcttta 139020
ggcagtcatt ggtgacacta gccagcaatg tgggaaacat gtgggattga atgtgtccaa 139080
agaggacttt cagctacctt ggcatgtctc aaagaatgca gagagtgaaa ctggttcctc 139140
acctgttttt catgaggtgt gattaaggga ctacagactc aggccataat aatacagaga 139200
caaaattttg ggaagaatag aaaaactcac cattactctg tctgacacat ggagaggggg 139260
agactactgt cttatggaag agggaaatag gataggggaa caaagtaata aacagattgg 139320
gcgtggtggc tcacacctgt aatcccagca ctttgggagg cagaggcagg cagatcatga 139380
ggtcaggagt tcgagaccag actggccaac atggagaaac cccatctcta ctaaagatac 139440
aaaaattagc cgggcgtagt ggcgcatgcc tgtaatccca gctactcagg aggctgaggc 139500
agaattgctt gaacctggga gggggaggtt tcagtgagcc aagatcatgc cattgcactc 139560

catcctgagg gacagaacaa gactctatct caggaaaaaa aaaaagtaat aaaacagtag 139620
caagaaaaca aaacagtagc aagagcaaga ataagcagaa gtattaaggt ggcatcttcc 139680
ataattatcc tcaaggaaca gggtctacct ccatgtttcc agttcagtcc ctcatacttt 139740
tacttgacct gtgagatgat tgtattctct gccttctctc cctggtcaca gatccaatgc 139800
ttaaagttga tgacagcaac actcggatcc tgattggctg cttggtggcc atcatcttta 139860
tcctcctggc catcattgtc atcatcctct ggaggcagtt ctggcagaaa atgctggaga 139920
aggtgaggag gtgcagaatg gtcatgatat aagaaactgc tcctttcttt cttaagccac 139980
tgttgtccca ggagtaaaag gcaaattgca aacatttatt aatggttgga attaactgag 140040
gctaccaaga aacataggaa tgaagccaga atagaattag ctaagtctat tttctcattt 140100
ttagagtgga gatgttggaa ccttcctaac gttcctcgga aatgcagaaa actcttctta 140160
atacattgaa tctccattca ggcattttcc ttgaagattc tgattcactg gattttgggt 140220
agagtccagg catccatatt taaaaaaaaa tttaacaagt gcttctgatg cacattcgag 140280
gttaagaatt actctcctaa tgccaagctg aaatttctga taatgctgtt gaagttcctt 140340
ctcatctttt tcactttgct tgtctgtttg gtgtgtgtgt gtgtgtgtgt gtgtgtagaa 140400
aaacatccag cttcattaat attcaagcca agtttctcac tattctattt ctacttttgt 140460
gtacttgaag gttgtgatta tgtatccctc tgagtccctc ttcagactct gtctatgcca 140520
aatgatctga ataacttttt acattgtcta acactttaca ggaagcagaa tttttcctca 140580
tgcataattt tatatacacc tcattgagta ataactcata ggcattgtta tgtttaacta 140640
tttttctgat gagaaaatgt gatgtgctta aggtcataca actgatgtcc agtcttctat 140700
gctttttcca attgttaata ttgaaatgca ttgatttctt tcttcaaaac tatattcatt 140760
tcatcaaaga tggaatatct atgacttgtc ttggtttctt ggaccaaata gcaactccct 140820
tggaccaaat agcaactcac tgctgcttag tgtctgattc ttttggggcc tgctgacgac 140880
taagtgtttc tgcctttggc catgatcacc tctcctggtc aattagttcc tctcaataga 140940
catggactag tagaggccac aggaactctg agctgtgggg ctttgaactg cctcctgagg 141000
gcagggaata ggaaggagga gaaatcctga ttgatttctg tgtttggggg tggggactca 141060
gaggagacca atgcctgtgt ggaacctcta ttccagagga gaggaaaggg caccatgaga 141120
atgggcttga gtctctagag ctttctagat gcaggatggg agaaaaatgg ctcaaatcag 141180
gggtaaggag gagcaatcct gatgagtagc ctgcaattcc ttctgatctg tgccaccctc 141240
aggttcctga gatggttgat gtttgtttta aggtttttag gaaggaatat ttcatggaag 141300

agaaggtctc cttgcttccc acaagtatgt ctaccagtct gaaaacactt aaagctttca 141360
attcgcaaat agcaacagaa gactgaaata cagaatccac agagactcac tcgctgtcac 141420
gagtatgtct caaggcatga atgaggcaaa tatcaaaatt tttcctcctt tagaatgctg 141480
gaaaacacta gtgtttaatg aggtattcca gagtgagggt ggtggtgaac tgaaggttca 141540
ttgaactgct tgaagaggat atgtgacctt cttaaatagt ctgttaggac cacattttag 141600
ccaaggggga aagtctgtgt tctctgctat atgagctctt tttctcttta tttgagccac 141660
aatttaccat acttctctga gtctttattc tgaggcctgt tggaatatct tcatgggcta 141720
agagagaaat gttggaatct ccaggtaaaa ggtagagcaa acatttgccc cttgaatatt 141780
taacttggtc ctccaagaaa ttctacccct gtacatcctg agtaagatga ctcaaggtgc 141840
tctggaatga cacccagtgt ttcttcctgg gaacaaaatt ttatgtggaa aatcattaag 141900
tacacacata cattctaaga acattactta acatagctgt agactgtatg tagcattatt 141960
tgtaggataa ttttggtggt gtcctcaaca ttccttcttt gagagattta acccacatat 142020
attgagtacc tactttgttc tgagcttcat gcttagtact ggggatatta ctgcaacaaa 142080
agaccttcag tctagtggaa aagtgcacat ctaagtttcg ccattgagca ccaaagctgt 142140
gtttgttgga gtggagcatt ttacatgttt aaacccaaat agtcacatac atgcaaatgc 142200
tggcatttgc cttttgatgg agcaggcagg ttatattaga taagatacca cagaagtagt 142260
tgctttgttg ttctccctcc cttattaggt gtaagtcaca ttgttatctc tggacaccca 142320
gggctttcct gtcccatgtt gtagccattt tatttttggt tagtgaaaat atgggtcagt 142380
ggtgggtatt atcctaatct agggtatcaa aatgatcaag tagaactact gattcatcat 142440
gttatagcac atgcattaag ttaataaaca atttatgtct attccaaaac caagtagtta 142500
aagggtaagc caggaaaacc catcatttaa taaaacatag aaaactctat aagattgaga 142560
tctttggcta ctgatgggtt cttatagcaa cagggagata atcttttttt tttaagtgtt 142620
tcagaaagca catcatagaa attagaagat caaattctac cttgaagaaa tgaaggatta 142680
ttctaagttc ctacaacaca ggcatttgtg gaaccagact gcattcctag cacgtgcagt 142740
gttgctgggg ttaaacagta gtaaagagtt atttcatttg agtgggagag ctgagtttaa 142800
gaagaggagg cattgaccat ctcttttgtg ccaacatgcc tttctccttg ctcttctctt 142860
ccaggcttct cggaggatgc tggatgatga aatgacagtc agcctttccc tgccaagtga 142920
ttctagcatg ttcaacaata accgctcctc atcacctagt gaacaagggt ccaactcgac 142980

ttacgatcgc atctttcccc ttcgccctga ctaccaggag ccatccaggc tgatacgaaa 143040
actcccagaa tttgctccag gggaggagga gtcaggtgag gatgatgtgg tgggcagggt 143100
gtcaagggag aaacctcagc aagcatcagg taggtatgac acgcctgctc ccagttcatt 143160
gcatctattt ttagtctctg ctaacctcct gagaagtcca cattctcttt ctagatttgg 143220
tctgccgctg ctcctggcct aatttgagca actctccttt gctactgaat ataagatgtg 143280
tggagggacc cacataccag aaaagaaaga aaaaaataat aaaacatgtg aattaggtgt 143340
tgacaccaga agaacctaaa cagtccaacc agtctaacct cattttacat gtcgggaaag 143400
tgagaaagtc cagatagagt actcacccaa caccaaattt catgagtggg taaaggcagg 143460
actagatttt tacctggaga aataaataaa taaatgaagc taaagacatt ccaaaattcc 143520
cagtggtgta gccatttaaa taactaaaat aaataactac ttgagttatc tattttttgt 143580
gaatttctct ttggtaaacg tttattagag taatatgaga ttttattatg agagtaaaac 143640
atttctcacc ttttctcaat aaaagcattg aaataaaaca ataaaacagg taaaacagta 143700
agtagtgtca gttgattcaa atttcgatgt ctttcaaaaa atgatgcaga gtcatatcag 143760
aagggaagct ggagtcacct ctttagctca gagcaacttt tctataagat catcaacctg 143820
agggacccga gtcagtagaa aagaacttgt agtgttcagg agagaggagc taggcctgag 143880
ctggatgtag aagcagacag gactaaattc aagagcatag gattttatag gaaggagcct 143940
gaaggggtct tttcaaagga taaaaggaaa cttgcatcta ttttattatt caatggatag 144000
aagacatgaa agagcatgct aaattcaagt aatttcacag agacagcaag aataaaggat 144060
ataaactaat ttatttcttg gggtgaatgg aagtaattta cctatttcct aagtctaaaa 144120
catcccccac ttcatatttt aaggcttttt taagttagaa tatgtcttaa gctagatcat 144180
atgtctttaa tctgaaagtt ttttatacct aaaatcagtt tttaagtcaa taacagtgtc 144240
ttaaaattaa ggaaataagt tattaaacct tcaaccttta atattcccac cctgatatac 144300
catgagcctt cctatagctt caccacacaa ttccacaagt ttagccacag aatttgttaa 144360
tgatcatgta ttatgaggaa aagaatgaga aataaaatga tgttctacat tctgtaggcc 144420
acaagaaaat ctctagagga agcaaaacca ttttccaagt aagaatgtca tgaagcaggt 144480
taggctttca caggggcatg ttttagccct cctctcagag ttccttcctg aagagatctc 144540
caaagacact ccacggaatg agggctcgtt gcccttgtct tcccaggctg cagcggtgtt 144600
gtgaagccag tccagcccag tggccctgag ggggtgcccc actatgcaga ggctgacata 144660
gtgaacctcc aaggagtgac aggaggcaac acatactcag tgcctgccgt caccatggac 144720

ctgctctcag gaaaagatgt ggctgtggag gagttcccca ggaaactcct aactttcaaa 144780
gagaagctgg gagaaggaca gtttggggag gtgagttgat tctttgattc ccttatagct 144840
cgaagaaggt ggttggataa aaatgatcag tagaacaaag agtcccttcc agaggtggat 144900
tcacaacaga atgtctcttc catttgtctc tccttgcatt gtcctctgga tttcagtatt 144960
gggtcaacct aatctttgtc aaatagctgt ggagttcctg ggacttctga tttctatata 145020
ggagttttta aggcaggcaa gtcactagac ttcagatatc ttgaaaaaca ttcaatattt 145080
tcaaaaatgt ttacaataga ttatggaggg tgggatccag aagatgtgat catcttttat 145140
gaactgtcca ggaacattct gctactcaat agccactttg tttttttccc ctttaatggg 145200
agttatatgt aaaatggtca aaaacaacct cgcccctgcc tttggtccca gaatcagcca 145260
ctagggaatg aatcaccaac taagataagg aggagtactc ttttcatttg ccctcagagt 145320
gatgcatgca ttattaaact tttcattctt gcatcagtca catgaacctt ctgggagctc 145380
aaagcaatgt tttgaattct aataggaact cttcaccact gccgtctaaa ctccaaataa 145440
aacatttggt ttcctaagac atgttattgg ttttcagtgt ggatctcatg ttctctcctt 145500
agataggtct ggtgttgcaa gaggttgagg gacccatgtt atctgttcat ggtgtgacat 145560
gtactcattc tgtcccctgg gttgaaaacc cacatctgtt atactgttac taatagacag 145620
gagtttctta taatgctaaa ccctatacta agggattatt ttaggaaatt gccttacgtt 145680
ttacatgatc cagtgactaa tgaatcgaag ttcaagcatt tgtaataaaa gacattttat 145740
attgggctat gctgtaatat aatattgtta ttgattcatt cattcactta gtgttccaag 145800
caatgtgtat ttctaaggca ggagagtggc acattagctg ggtactttac cattgtttta 145860
aatcttatca attatttgaa tcatagatat gttacacata tcttcttatt ggtcttttga 145920
tcattttgcc tgagttgtaa gagttagttt atctccagga aatgcccagc aagagtactg 145980
agacatcttc aggagaaatg atgatgctga gactagatga cttttgtcta ggttcatctc 146040
tgtgaagtgg agggaatgga aaaattcaaa gacaaagatt ttgccctaga tgtcagtgcc 146100
aaccagcctg tcctggtggc tgtgaaaatg ctccgagcag atgccaacaa gaatgccagg 146160
tctgtggtct acattttgaa ttttccttta ggtatttcat ctttaggacc aggaatactc 146220
ctggatactt ctgagcaatt ttttcttttg agatgggaag gtcagtgagc tgcctcccat 146280
ttccactgtt atcctttttc ttattccgat ggggtcggca gtcctcatct ttttctccac 146340
ctctctcaaa cacaaaggta cattttccat tgtacttgcc acatatttac ttcctaaccc 146400

cctgtgcaca agcactgtac ttgttcccaa catatacaac tgtctaaatg tgtacttctt 146460
tggtctttat acacagacat actcctgcat gtcattacct cctcttgaca aagtcttcag 146520
aatcacacat gactttcttg ctaagcctct tccagtctcc ttttttcaca tttctattta 146580
ttttctcatc ccttttttct cttttatgct ccttttcttc tatttgagca accctcaaaa 146640
tgagaagcta cctctatttg gaaaattatc tatgaacctc aaagcaaaag gatccacagt 146700
tctctagcag ttttccaaac tggctaataa tctcttggat ctttttctac ataaattgca 146760
gtttccagcc acacttcatt gtctgacagc ctttccatta caataatggt gagttaatcc 146820
tcacccagag agacaagctg gttgaaagct ggctgtatgt gtcccagaga aatctccaca 146880
cctggataac atatgcattt tcccatttgt gttgtcttga tttgtctcac tgatattcat 146940
agtgaaaaaa agtttattat tcttattttc caaattatca taaattccaa gaatggtgat 147000
agacttcatt aaaagtcttg caatggacaa gatgtaagat aaagaagaga gaaaggtgag 147060
gaaatgaaac tggaggcttt agaaatttaa ctgataattt caatctttaa gatactggta 147120
agaataaaaa gtgacattct ctgggtctta tagagagacg aagtatttct tatattaatc 147180
ttagcacatg attggagaaa gtggcatgag gggaaacaaa accaaaccaa accacaggac 147240
ataggatatg acaaagcctt tctccgttgt tccgatcggt gaagtttgcc attcctccac 147300
ctgtgtcctt agcaatgatg gatggccatg actgcaaacc ctcccaagcc atggaaaccc 147360
ctggtttgag acggaagggg gaatgaaaga ggggaggtgt tgcatcagga cggcattgat 147420
tccagtggtt tatattgacg tagtatgact tccagtccag gattcctagc aaactcttgg 147480
agggcttata actcatctgc tccaacagtt ttgggaatgt gggctagagg cctcactaac 147540
aaaatcttgc tactttatga cccaagggtg tctgtcacca tgtactcaaa caattattag 147600
tcttggtcaa atgaatactt cttttgacat gattaccacc acagccttta aagagctctg 147660
ttttcgacag gctactaaat acatgttgca actgactgat ggttcgtact gcattaagaa 147720
aagtcagtat gagaaaataa agagtaatca gataagccaa agatggttgt tcacatcata 147780
caaagtttct tcactagtaa tagagtgact ccatggcttt aaaaaaaaaa aggtaaatat 147840
cctcgtgcac ttaagatatg attttcagtg aaacatttta ctctgtagag tggtttttag 147900
cagcctattt ttgattcagc aaatattaat cactttctgt gtggcaggga ttttgttaga 147960
tgtgagggat agaaatgtga ataagtcatg gatcttgttc tcaaaaacat tacaattgaa 148020
taagggccca tgatatttca ggaggaatag aatgagacag atccaggtgt caatttctct 148080
gtcctctcaa aagttatcca aagggaaaca agatttggca taatgtccag gaataggcct 148140

tggtgtgcat tcttctctct cttgattctg actctggcaa cttctgagtt tatctatgtc 148200
tgtatcctcc caaggaatga ttttcttaag gagataaaga tcatgtctcg gctcaaggac 148260
ccaaacatca tccatctatt agctgtgtgt atcactgatg accctctctg tatgatcact 148320
gaatacatgg agaatggaga tctcaatcag tttctttccc gccacgagcc ccctaattct 148380
tcctccagcg atgtacgcac tgtcaggtaa acaagccagg tcttccttct cctccctgtg 148440
gtcatgagag taacctggga tctgaaaata gggagcagtg agccttaaag tgtatcaatg 148500
ttctgggatg gtgggggaag tcagtgtgca gggaataatg gagcagctgc cctttgggga 148560
aacgcaaggg attcatcaag agtagagaaa gaatgttgag ctttcaaccc tagtttgttg 148620
ataccatttt agaatgtgta cctttcacat cttcctgttt ccataggcta ccttctgtct 148680
tcttgtctat ttcctcagtt acaccaatct gaagtttatg gctacccaaa ttgcctctgg 148740
catgaagtac ctttcctctc ttaattttgt tcaccgagat ctggccacac gaaactgttt 148800
agtgggtaag aactacacaa tcaagatagc tgactttgga atgagcagga acctgtacag 148860
tggtgactat taccggatcc agggccgggc agtgctccct atccgctgga tgtcttggga 148920
gagtatcttg ctggtaagtt ctcagcattt taaagccctg tctaacaact ggcttgtggg 148980
ctcacatgca tgacacgtgg agacaaacct gagacagcag gaggcaaact caatagactg 149040
tagtattttc tctgatttga aattattttc tttgtgtact aattcttatt atttaatgct 149100
tgcctatttc tgctaaaaaa ttaacacaca taaaaattta gggagagaag ttaaaaagta 149160
taagagattt attatctttg tttctcttta tcatttctac ctagtgattg ttcttaattt 149220
aagatattaa aaaccttatc atttaaagaa atgttaccta tacaaaatat tttcttctcc 149280
attagtttca taagcatgtt tctcataatt acatagacta attcctataa ttgtatttca 149340
atacttttca acatgttctt tttaagttca tcattctttg ctttataatt tttatttgat 149400
tcttgatatt ccataccata agtgagtaat ttttcccaaa tatctctatt agaggtatat 149460
tttctgtcca tgcatacctg agacttttta ttgttgattt tataaccact cagctgattt 149520
atagactcat tagactatac acttttactt cctacaatgc catgtacatg ttattttatc 149580
tttttttcttg tgtgacattg cagagaaaag tctgagacca atttggtttt gttcatttat 149640
gattaatctg cttatttcct gctaatattc ttataggatt tgtaaattaa ttttgaaata 149700
aaaaatgtat agttattccc ttgcactcac caggataacc actatcaata ctttggtata 149760
ttttcttaga ggattttttt ctatacatat atttttttcta tgcatgcaat ttttttttctg 149820

tgcatgcatt tatatacata tgtacatata tgtaatttat attacataat tgggatgaca 149880
ctatgccata caattctcta tctctatttt taacttaaca ttttattatc tgcataatat 149940
tccatgtgta gatatatact ataacttaat aatgcctcta taatttgata tttaagtact 150000
ttccaatttc ctgctattat gaatgatgct gtgatgtaca ccagtttaca taaatcctta 150060
taaagatctt tttcacctat atatgtgaat tgctatattt tacccatttc taatgggata 150120
gttaggcatt actctcccta aaggtattat atggtgttca aaacaatata ttagactgac 150180
attcagagga ccttggttca aatccagttt ttccactact aatggtctta ttaaccagaa 150240
gaatttggga aacctctcct gtttcatttt tctgatctaa aaaataagga taactggcat 150300
ggtggtatgt acctgtagtc ccagctactc aggaggctga ggcaggagga tgaaaatctg 150360
ggctgcgctg cgctatgaca attgggtgtc ctcactaagt ctggcattaa tatggtcacc 150420
cctaggagca gtggaccacc aggttgccta aagagggatg agccagccca ggtcagaaac 150480
agaggaggtc aaaactcccg tgttgatcag taatgagatc aaacgtgtga ctggccattg 150540
cactccaggc tgggcaacat aatgagaccc catttctcaa aggaaaaaat atttaataat 150600
aaataatatc atccacttca cagtgtggtt gtctgtattg agtagaaaat gtgagtagtc 150660
aatgcatgct atttacacct gaatctcatt gctgtcactt gttaaagtct atctttcaac 150720
actgtgatta tcactgaact ggtaaatgag agtcctggat tcactgcagg cacttaaact 150780
agattcctct atctctaaca taaaaaccat tgacttccca ttcagatttt gcctttctca 150840
ggagcatttt cccaaggcta ccctagagtt gaaaggttga cctaacactc agttgcagag 150900
tcaaatagct tatggctgcg tttttttcag ctcttggatc tcatccccag agaaacagaa 150960
ccaagctgag atcatgagaa tgaaagaatg ggctgtttct aaatactcca tgcacatctt 151020
ggcattttca gaattccttg cctgtggtgg gggaaggggt atagctgcag attatgaaat 151080
ttaacagggt gttgttgtgc acaggttatt ctgatttccc attcttttct ttacttaaat 151140
agggcaagtt cactacagca agtgatgtgt gggcctttgg ggttactttg tgggagactt 151200
tcaccttttg tcaagaacag ccctattccc agctgtcaga tgaacaggtt attgagaata 151260
ctggagagtt cttccgagac caagggaggc aggtaagaac tgttggggat gaatggatgt 151320
ggacctgtgt accttgaagg atggagaatg gcaagtcctg ccttagcagg agtgggccga 151380
gatggaagac agactatcca ggtgttagtc tttgtcacta actatccaga tgatgtgaaa 151440
gaccagtatc ctccccaggc atccatacac tttgctttat ctatgtgtca actcattgac 151500
tcttggagag cataaagggt catgtcaggt ttctccccag ctcaaaggta gcaggaaaca 151560

aagtcctatc tctcatgatt tatctatttc ttgatctaga ccatgtggtt tttcttctgg 151620

gtcaaaaaat aaaagtgtaa aaggatgtta ttagcattca ttaaaaatat agcttagtat 151680

aagaaatggg gttggttttg actatctagc aatgtttcta gacttggatg tctgaaaatg 151740

ggcagatata tcctaaactt gttgctcttg gaaggcatct ctgggcccag tattgtgaca 151800

tctcaggact cagggaaaga tgagttccca agctccccag gagtctcaat tcctagccaa 151860

gatactccaa gctttttttt gggtggctta gatttaaaat tcctgtgaga tctggaattt 151920

aaagcttgcc agtaactaca gcaccgcact tccccaaagg ggcctggcaa cagcataccc 151980

ttggattatg gtatctttac caggaaaagg ataatagctg ggggaaggat cttgcagaca 152040

ataaaaagtg gatcttctgt ggcttgaaca gatccaagca agccaaagat agataaggta 152100

ttgacaagag ataatacatt atgcatatta aaaataagag aggggtctga gaatttaatt 152160

gtatcttgat actctgtaaa tattttggca gcagggagtc taaattagct atcttggtta 152220

tgaggcaatt tgccttctta ctatagtcaa aggaaaattt actcctgagt acatgagata 152280

agtgagtggg ccaaagaaaa atgttgtttt ctgcctctgc catgatcggc tcagccaaga 152340

tgaacttcaa tatgggttgc ccataaatgg aagatttggt ctctttttgg ttgtttcatc 152400

ttttgcatga aggtagggac cacataatgg cccatcaaaa aattctgctg cagacaactg 152460

cagaagacaa atggaacagc ttagaaaagg gctgatatga ttcagaactt tcacgagtag 152520

gaaaaatgga cactacaccc atttcagtta tcaagttcaa gaaagtgggc agggggcaaa 152580

tcaaaccatg atgcaaagat acttcccttt cccccgtctt tgtaatattc tctctctctc 152640

tctcttttgt tttcctttat ttttgttccc aaagacttac ctccctcaac cagccatttg 152700

tcctgactct gtgtataagc tgatgctcag ctgctggaga agagatacga agaaccgtcc 152760

ctcattccaa gaaatccacc ttctgctcct tcaacaaggc gacgagtgat gctgtcagtg 152820

cctggccatg ttcctacggc tcaggtcctc cctacaagac ctaccactca cccatgccta 152880

tgccactcca tctggacatt taatgaaact gagagacaga ggcttgtttg ctttgccctc 152940

ttttcctggt cacccccact ccctacccct gactcatata tacttttttt tttttttaca 153000

ttaaagaact aaaaaaggaa aaaaaaaagc ctagggcaga tacaatctag taaaagaaaa 153060

tctttgatat accaaagtgt tggataacaa aggctagaaa attcagataa tttataaagg 153120

ttaactatac ttgtgcttat aaatgtgcag attctacaat atttttccat gtcattctaa 153180

aagaatcttc agaaagaaaa acttgaagaa tactaatgtc ttgggaaaca tgagattaag 153240

tttagggaaa acacttgata gatgtggaga atctgaggac tcagaattca gcaactaatc 153300
acaggtggtt ttctaatttg gcctctggac atgtctcact gtttgtattt ctctctcctg 153360
tcaaagtgaa tgatatattc ttgaaaaccc ccaatttctt gaaatgggtg ttctgttcat 153420
tcatgggcag gggtcaggat tgaagttcat atatgaaaca actggggatg tagataggaa 153480
agaatgtctg ctgcaagagt gtatgaaggg ggattgtcat ttacaaaaaa aaagtacctc 153540
atggatggaa ggattcatga atagataatg gacataggaa aggaggtcaa tggaggacac 153600
ataacagaca tgctatccac catttatttg tgattttgta agagggttct cttctttgtc 153660
ttgttcagat attttcatgt gtgtgtttta gtgagaatct gagtttttaa tcagtaaagt 153720
ctgatgtttt gatatcttga tgttttgatg gtagactctt gagttcatct tgtccaaatc 153780
ttctagcatt ttcaaggatg aagcctctgt gggatctttg ggcttggggc tgaatttctc 153840
aggcataaat acctaatggc cttgtctaag agggaataga tctgttctga gtgtccccaa 153900
aaaagttgtc ctaggaactg atgtggagaa gttacaggga gactgatttc cattccttat 153960
tggggaaaac gaagcactca gaacatacaa ggataaatgg gctgccacca gaggtgagga 154020
gtctcttgtc actggagagg ttaagataga ggctcaagaa caatgcaagg aaaatggagg 154080
ttttcaaaca caaagtggat ggtggtatga gacaatgttt aatgtccttc taactccgag 154140
agtccttgat tctggagagg cacaggatga acatctgttg ctactgctga gtatacacac 154200
tcttgcctct gagatggcgt gaccagcaga aaagaaagct cagggctgtg gcttcagaat 154260
tcatggaaac agagccactg tcaagaagga atctgctcag agcagattct gagttttcac 154320
actccacaaa tcctcagatt tagtgggaag ctggaaaaag gaactcttaa ttccaattga 154380
ggagaaacaa gaaaacatta caaatcagtt tcccaagctg agaaggatta gccttgaccc 154440
cttgggtctg cctctgttgc cctcaccagt ttaccttcta caacagtttc tcaggaacat 154500
gtgtatttcc ccattcagct ttcagcattg ctccacgcca cagcataagc atagatccca 154560
agtccacagg ctccattttg caggtcatct tctgatccta gcaaatgtcc tttccccata 154620
gttgtcctat gcctttgggc tttagtctat cccaggacta actgtggaga aatcattggt 154680
ttgagagtca agagagcatt ggtttgggag ctttaatcct ctttctgctt cacactaagt 154740
gtgtcatctt ggctaaatca cttggtcttt ctgcattttg ttttcttatt tataggatga 154800
ggaaattaga ttaaatggtt ttgaggtcct ttcttgttct gatatgtcca gtactcactg 154860
gaaaattgga tctataactg atgggtttag taatctggtc atttcttgct ctgaaaattg 154920
tagtcagcaa aagagatcat ggaagaaatc actgtaatgg tagtaatagt aacacatgcc 154980

atttgtattg tgccttaggt ttaccaggtg tttccaaata cattagca 155028

<210> 2
<211> 2568
<212> DNA
<213> Homo sapiens

<220>
<221> CDS
<222> (1)..(2565)

<400> 2
atg atc ctg att ccc aga atg ctc ttg gtg ctg ttc ctg ctg ctg cct 48
atc ttg agt tct gca aaa gct cag gtt aat cca gct ata tgc cgc tat 96
cct ctg ggc atg tca gga ggc cag att cca gat gag gac atc aca gct 144
tcc agt cag tgg tca gag tcc aca gct gcc aaa tat gga agg ctg gac 192
tca gaa gaa ggg gat gga gcc tgg tgc cct gag att cca gtg gaa cct 240
gat gac ctg aag gag ttt ctg cag att gac ttg cac acc ctc cat ttt 288
atc act ctg gtg ggg acc cag ggg cgc cat gca gga ggt cat ggc atc 336
gag ttt gcc ccc atg tac aag atc aat tac agt cgg gat ggc act cgc 384
tgg atc tct tgg cgg aac cgt cat ggg aaa cag gtg ctg gat gga aat 432
agt aac ccc tat gac att ttc cta aag gac ttg gag ccg ccc att gta 480
gcc aga ttt gtc cgg ttc att cca gtc acc gac cac tcc atg aat gtg 528
tgt atg aga gtg gag ctt tac ggc tgt gtc tgg cta gat ggc ttg gtg 576
tct tac aat gct cca gct ggg cag cag ttt gta ctc cct gga ggt tcc 624
atc att tat ctg aat gat tct gtc tat gat gga gct gtt gga tac agc 672
atg aca gaa ggg cta ggc caa ttg acc gat ggt gtg tct ggc ctg gac 720
gat ttc acc cag acc cat gaa tac cac gtg tgg ccc ggc tat gac tat 768
gtg ggc tgg cgg aac gag agt gcc acc aat ggc tac att gag atc atg 816
ttt gaa ttt gac cgc atc agg aat ttc act acc atg aag gtc cac tgc 864
aac aac atg ttt gct aaa ggt gtg aag atc ttt aag gag gta cag tgc 912
tac ttc cgc tct gaa gcc agt gag tgg gaa cct aat gcc att tcc ttc 960
ccc ctt gtc ctg gat gac gtc aac ccc agt gct cgg ttt gtc acg gtg 1008
cct ctc cac cac cga atg gcc agt gcc atc aag tgt caa tac cat ttt 1056
gca gat acc tgg atg atg ttc agt gag atc acc ttc caa tca gat gct 1104
gca atg tac aac aac tct gaa gcc ctg ccc acc tct cct atg gca ccc 1152
aca acc tat gat cca atg ctt aaa gtt gat gac agc aac act cgg atc 1200
ctg att ggc tgc ttg gtg gcc atc atc ttt atc ctc ctg gcc atc att 1248
gtc atc atc ctc tgg agg cag ttc tgg cag aaa atg ctg gag aag gct 1296
tct cgg agg atg ctg gat gat gaa atg aca gtc agc ctt tcc ctg cca 1344
agt gat tct agc atg ttc aac aat aac cgc tcc tca tca cct agt gaa 1392
caa ggg tcc aac tcg act tac gat cgc atc ttt ccc ctt cgc cct gac 1440
tac cag gag cca tcc agg ctg ata cga aaa ctc cca gaa ttt gct cca 1488
ggg gag gag gag tca ggc tgc agc ggt gtt gtg aag cca gtc cag ccc 1536
agt ggc cct gag ggg gtg ccc cac tat gca gag gct gac ata gtg aac 1584
ctc caa gga gtg aca gga ggc aac aca tac tca gtg cct gcc gtc acc 1632
atg gac ctg ctc tca gga aaa gat gtg gct gtg gag gag ttc ccc agg 1680
aaa ctc cta act ttc aaa gag aag ctg gga gaa gga cag ttt ggg gag 1728
gtt cat ctc tgt gaa gtg gag gga atg gaa aaa ttc aaa gac aaa gat 1776
ttt gcc cta gat gtc agt gcc aac cag cct gtc ctg gtg gct gtg aaa 1824
atg ctc cga gca gat gcc aac aag aat gcc agg aat gat ttt ctt aag 1872
gag ata aag atc atg tct cgg ctc aag gac cca aac atc atc cat cta 1920
tta gct gtg tgt atc act gat gac cct ctc tgt atg atc act gaa tac 1968
atg gag aat gga gat ctc aat cag ttt ctt tcc cgc cac gag ccc cct 2016
aat tct tcc tcc agc gat gta cgc act gtc agt tac acc aat ctg aag 2064
ttt atg gct acc caa att gcc tct ggc atg aag tac ctt tcc tct ctt 2112
aat ttt gtt cac cga gat ctg gcc aca cga aac tgt tta gtg ggt aag 2160
aac tac aca atc aag ata gct gac ttt gga atg agc agg aac ctg tac 2208
agt ggt gac tat tac cgg atc cag ggc cgg gca gtg ctc cct atc cgc 2256
tgg atg tct tgg gag agt atc ttg ctg ggc aag ttc act aca gca agt 2304
gat gtg tgg gcc ttt ggg gtt act ttg tgg gag act ttc acc ttt tgt 2352
caa gaa cag ccc tat tcc cag ctg tca gat gaa cag gtt att gag aat 2400
act gga gag ttc ttc cga gac caa ggg agg cag act tac ctc cct caa 2448
cca gcc att tgt cct gac tct gtg tat aag ctg atg ctc agc tgc tgg 2496
aga aga gat acg aag aac cgt ccc tca ttc caa gaa atc cac ctt ctg 2544
ctc ctt caa caa ggc gac gag tga 2568

<210>  3
<211>  855
<212>  PRT
<213>  Homo sapiens

<400>  3

<210>  4

<211> 2568
<212> DNA
<213> Homo sapiens

<220>
<221> CDS
<222> (1)..(2565)

<220>
<221> misc_feature
<222> (2041)..(2043)
<223> n is a, c, g, or t

<400> 4
atg atc ctg att ccc aga atg ctc ttg gtg ctg ttc ctg ctg ctg cct 48
Met Ile Leu Ile Pro Arg Met Leu Leu Val Leu Phe Leu Leu Leu Pro
1               5                   10                  15

atc ttg agt tct gca aaa gct cag gtt aat cca gct ata tgc cgc tat 96
Ile Leu Ser Ser Ala Lys Ala Gln Val Asn Pro Ala Ile Cys Arg Tyr
            20                  25                  30

cct ctg ggc atg tca gga ggc cag att cca gat gag gac atc aca gct 144
Pro Leu Gly Met Ser Gly Gly Gln Ile Pro Asp Glu Asp Ile Thr Ala
        35                  40                  45

tcc agt cag tgg tca gag tcc aca gct gcc aaa tat gga agg ctg gac 192
Ser Ser Gln Trp Ser Glu Ser Thr Ala Ala Lys Tyr Gly Arg Leu Asp
    50                  55                  60

tca gaa gaa ggg gat gga gcc tgg tgc cct gag att cca gtg gaa cct 240
Ser Glu Glu Gly Asp Gly Ala Trp Cys Pro Glu Ile Pro Val Glu Pro
65                  70                  75                  80

gat gac ctg aag gag ttt ctg cag att gac ttg cac acc ctc cat ttt 288
Asp Asp Leu Lys Glu Phe Leu Gln Ile Asp Leu His Thr Leu His Phe
                85                  90                  95

atc act ctg gtg ggg acc cag ggg cgc cat gca gga ggt cat ggc atc 336
Ile Thr Leu Val Gly Thr Gln Gly Arg His Ala Gly Gly His Gly Ile
            100                 105                 110

gag ttt gcc ccc atg tac aag atc aat tac agt cgg gat ggc act cgc 384
Glu Phe Ala Pro Met Tyr Lys Ile Asn Tyr Ser Arg Asp Gly Thr Arg
        115                 120                 125

tgg atc tct tgg cgg aac cgt cat ggg aaa cag gtg ctg gat gga aat 432
Trp Ile Ser Trp Arg Asn Arg His Gly Lys Gln Val Leu Asp Gly Asn
    130                 135                 140

agt aac ccc tat gac att ttc cta aag gac ttg gag ccg ccc att gta 480
Ser Asn Pro Tyr Asp Ile Phe Leu Lys Asp Leu Glu Pro Pro Ile Val
145                 150                 155                 160

gcc aga ttt gtc cgg ttc att cca gtc acc gac cac tcc atg aat gtg 528
Ala Arg Phe Val Arg Phe Ile Pro Val Thr Asp His Ser Met Asn Val

                165                 170                 175

tgt atg aga gtg gag ctt tac ggc tgt gtc tgg cta gat ggc ttg gtg 576
Cys Met Arg Val Glu Leu Tyr Gly Cys Val Trp Leu Asp Gly Leu Val
            180                 185                 190

tct tac aat gct cca gct ggg cag cag ttt gta ctc cct gga ggt tcc 624
Ser Tyr Asn Ala Pro Ala Gly Gln Gln Phe Val Leu Pro Gly Gly Ser
        195                 200                 205

atc att tat ctg aat gat tct gtc tat gat gga gct gtt gga tac agc 672
Ile Ile Tyr Leu Asn Asp Ser Val Tyr Asp Gly Ala Val Gly Tyr Ser
    210                 215                 220

atg aca gaa ggg cta ggc caa ttg acc gat ggt gtg tct ggc ctg gac 720
Met Thr Glu Gly Leu Gly Gln Leu Thr Asp Gly Val Ser Gly Leu Asp
225                 230                 235                 240

gat ttc acc cag acc cat gaa tac cac gtg tgg ccc ggc tat gac tat 768
Asp Phe Thr Gln Thr His Glu Tyr His Val Trp Pro Gly Tyr Asp Tyr
                245                 250                 255

gtg ggc tgg cgg aac gag agt gcc acc aat ggc tac att gag atc atg 816
Val Gly Trp Arg Asn Glu Ser Ala Thr Asn Gly Tyr Ile Glu Ile Met
            260                 265                 270

ttt gaa ttt gac cgc atc agg aat ttc act acc atg aag gtc cac tgc 864
Phe Glu Phe Asp Arg Ile Arg Asn Phe Thr Thr Met Lys Val His Cys
        275                 280                 285

aac aac atg ttt gct aaa ggt gtg aag atc ttt aag gag gta cag tgc 912
Asn Asn Met Phe Ala Lys Gly Val Lys Ile Phe Lys Glu Val Gln Cys
    290                 295                 300

tac ttc cgc tct gaa gcc agt gag tgg gaa cct aat gcc att tcc ttc 960
Tyr Phe Arg Ser Glu Ala Ser Glu Trp Glu Pro Asn Ala Ile Ser Phe
305                 310                 315                 320

ccc ctt gtc ctg gat gac gtc aac ccc agt gct cgg ttt gtc acg gtg 1008
Pro Leu Val Leu Asp Asp Val Asn Pro Ser Ala Arg Phe Val Thr Val
                325                 330                 335

cct ctc cac cac cga atg gcc agt gcc atc aag tgt caa tac cat ttt 1056
Pro Leu His His Arg Met Ala Ser Ala Ile Lys Cys Gln Tyr His Phe
            340                 345                 350

gca gat acc tgg atg atg ttc agt gag atc acc ttc caa tca gat gct 1104
Ala Asp Thr Trp Met Met Phe Ser Glu Ile Thr Phe Gln Ser Asp Ala
        355                 360                 365

gca atg tac aac aac tct gaa gcc ctg ccc acc tct cct atg gca ccc 1152
Ala Met Tyr Asn Asn Ser Glu Ala Leu Pro Thr Ser Pro Met Ala Pro
    370                 375                 380

aca acc tat gat cca atg ctt aaa gtt gat gac agc aac act cgg atc 1200
Thr Thr Tyr Asp Pro Met Leu Lys Val Asp Asp Ser Asn Thr Arg Ile
385                 390                 395                 400

ctg att ggc tgc ttg gtg gcc atc atc ttt atc ctc ctg gcc atc att 1248
Leu Ile Gly Cys Leu Val Ala Ile Ile Phe Ile Leu Leu Ala Ile Ile
                405                 410                 415

gtc atc atc ctc tgg agg cag ttc tgg cag aaa atg ctg gag aag gct 1296
Val Ile Ile Leu Trp Arg Gln Phe Trp Gln Lys Met Leu Glu Lys Ala
            420                 425                 430

tct cgg agg atg ctg gat gat gaa atg aca gtc agc ctt tcc ctg cca 1344
Ser Arg Arg Met Leu Asp Asp Glu Met Thr Val Ser Leu Ser Leu Pro
        435                 440                 445

agt gat tct agc atg ttc aac aat aac cgc tcc tca tca cct agt gaa 1392
Ser Asp Ser Ser Met Phe Asn Asn Asn Arg Ser Ser Ser Pro Ser Glu
    450                 455                 460

caa ggg tcc aac tcg act tac gat cgc atc ttt ccc ctt cgc cct gac 1440
Gln Gly Ser Asn Ser Thr Tyr Asp Arg Ile Phe Pro Leu Arg Pro Asp
465                 470                 475                 480

tac cag gag cca tcc agg ctg ata cga aaa ctc cca gaa ttt gct cca 1488
Tyr Gln Glu Pro Ser Arg Leu Ile Arg Lys Leu Pro Glu Phe Ala Pro
                485                 490                 495

ggg gag gag gag tca ggc tgc agc ggt gtt gtg aag cca gtc cag ccc 1536
Gly Glu Glu Glu Ser Gly Cys Ser Gly Val Val Lys Pro Val Gln Pro
            500                 505                 510

agt ggc cct gag ggg gtg ccc cac tat gca gag gct gac ata gtg aac 1584
Ser Gly Pro Glu Gly Val Pro His Tyr Ala Glu Ala Asp Ile Val Asn
        515                 520                 525

ctc caa gga gtg aca gga ggc aac aca tac tca gtg cct gcc gtc acc 1632
Leu Gln Gly Val Thr Gly Gly Asn Thr Tyr Ser Val Pro Ala Val Thr
    530                 535                 540

atg gac ctg ctc tca gga aaa gat gtg gct gtg gag gag ttc ccc agg 1680
Met Asp Leu Leu Ser Gly Lys Asp Val Ala Val Glu Glu Phe Pro Arg
545                 550                 555                 560

aaa ctc cta act ttc aaa gag aag ctg gga gaa gga cag ttt ggg gag 1728
Lys Leu Leu Thr Phe Lys Glu Lys Leu Gly Glu Gly Gln Phe Gly Glu
                565                 570                 575

gtt cat ctc tgt gaa gtg gag gga atg gaa aaa ttc aaa gac aaa gat 1776
Val His Leu Cys Glu Val Glu Gly Met Glu Lys Phe Lys Asp Lys Asp
            580                 585                 590

ttt gcc cta gat gtc agt gcc aac cag cct gtc ctg gtg gct gtg aaa 1824
Phe Ala Leu Asp Val Ser Ala Asn Gln Pro Val Leu Val Ala Val Lys
        595                 600                 605

atg ctc cga gca gat gcc aac aag aat gcc agg aat gat ttt ctt aag 1872
Met Leu Arg Ala Asp Ala Asn Lys Asn Ala Arg Asn Asp Phe Leu Lys
    610                 615                 620

gag ata aag atc atg tct cgg ctc aag gac cca aac atc atc cat cta 1920
Glu Ile Lys Ile Met Ser Arg Leu Lys Asp Pro Asn Ile Ile His Leu
625                 630                 635                 640

tta gct gtg tgt atc act gat gac cct ctc tgt atg atc act gaa tac 1968
Leu Ala Val Cys Ile Thr Asp Asp Pro Leu Cys Met Ile Thr Glu Tyr
                645                 650                 655

atg gag aat gga gat ctc aat cag ttt ctt tcc cgc cac gag ccc cct 2016
Met Glu Asn Gly Asp Leu Asn Gln Phe Leu Ser Arg His Glu Pro Pro
            660                 665                 670

aat tct tcc tcc agc gat gta cgc nnn gtc agt tac acc aat ctg aag 2064
Asn Ser Ser Ser Ser Asp Val Arg Xaa Val Ser Tyr Thr Asn Leu Lys
        675                 680                 685

ttt atg gct acc caa att gcc tct ggc atg aag tac ctt tcc tct ctt 2112
Phe Met Ala Thr Gln Ile Ala Ser Gly Met Lys Tyr Leu Ser Ser Leu
    690                 695                 700

aat ttt gtt cac cga gat ctg gcc aca cga aac tgt tta gtg ggt aag 2160
Asn Phe Val His Arg Asp Leu Ala Thr Arg Asn Cys Leu Val Gly Lys
705                 710                 715                 720

aac tac aca atc aag ata gct gac ttt gga atg agc agg aac ctg tac 2208
Asn Tyr Thr Ile Lys Ile Ala Asp Phe Gly Met Ser Arg Asn Leu Tyr
                725                 730                 735

agt ggt gac tat tac cgg atc cag ggc cgg gca gtg ctc cct atc cgc 2256
Ser Gly Asp Tyr Tyr Arg Ile Gln Gly Arg Ala Val Leu Pro Ile Arg
            740                 745                 750

tgg atg tct tgg gag agt atc ttg ctg ggc aag ttc act aca gca agt 2304
Trp Met Ser Trp Glu Ser Ile Leu Leu Gly Lys Phe Thr Thr Ala Ser
        755                 760                 765

gat gtg tgg gcc ttt ggg gtt act ttg tgg gag act ttc acc ttt tgt 2352
Asp Val Trp Ala Phe Gly Val Thr Leu Trp Glu Thr Phe Thr Phe Cys
    770                 775                 780

caa gaa cag ccc tat tcc cag ctg tca gat gaa cag gtt att gag aat 2400
Gln Glu Gln Pro Tyr Ser Gln Leu Ser Asp Glu Gln Val Ile Glu Asn
785                 790                 795                 800

act gga gag ttc ttc cga gac caa ggg agg cag act tac ctc cct caa 2448
Thr Gly Glu Phe Phe Arg Asp Gln Gly Arg Gln Thr Tyr Leu Pro Gln
                805                 810                 815

cca gcc att tgt cct gac tct gtg tat aag ctg atg ctc agc tgc tgg 2496
Pro Ala Ile Cys Pro Asp Ser Val Tyr Lys Leu Met Leu Ser Cys Trp
            820                 825                 830

aga aga gat acg aag aac cgt ccc tca ttc caa gaa atc cac ctt ctg 2544
Arg Arg Asp Thr Lys Asn Arg Pro Ser Phe Gln Glu Ile His Leu Leu
        835                 840                 845

ctc ctt caa caa ggc gac gag tga 2568

Leu Leu Gln Gln Gly Asp Glu
850                 855

<210> 5
<211> 855
<212> PRT
<213> Homo sapiens

<220>
<221> misc_feature
<222> (681)..(681)
<223> The 'Xaa' at location 681 stands for Lys, Asn, Arg, Ser, Ile,
      Met, Glu, Asp, Gly, Ala, Val, Gln, His, Pro, Leu, Tyr, Trp,
      Cys, or Phe.

<400> 5

Met Ile Leu Ile Pro Arg Met Leu Leu Val Leu Phe Leu Leu Leu Pro
1               5                   10                  15

Ile Leu Ser Ser Ala Lys Ala Gln Val Asn Pro Ala Ile Cys Arg Tyr
            20                  25                  30

Pro Leu Gly Met Ser Gly Gly Gln Ile Pro Asp Glu Asp Ile Thr Ala
        35                  40                  45

Ser Ser Gln Trp Ser Glu Ser Thr Ala Ala Lys Tyr Gly Arg Leu Asp
    50                  55                  60

Ser Glu Glu Gly Asp Gly Ala Trp Cys Pro Glu Ile Pro Val Glu Pro
65                  70                  75                  80

Asp Asp Leu Lys Glu Phe Leu Gln Ile Asp Leu His Thr Leu His Phe
                85                  90                  95

Ile Thr Leu Val Gly Thr Gln Gly Arg His Ala Gly Gly His Gly Ile
            100                 105                 110

Glu Phe Ala Pro Met Tyr Lys Ile Asn Tyr Ser Arg Asp Gly Thr Arg
        115                 120                 125

Trp Ile Ser Trp Arg Asn Arg His Gly Lys Gln Val Leu Asp Gly Asn
    130                 135                 140

Ser Asn Pro Tyr Asp Ile Phe Leu Lys Asp Leu Glu Pro Pro Ile Val
145                 150                 155                 160

Ala Arg Phe Val Arg Phe Ile Pro Val Thr Asp His Ser Met Asn Val
                165                 170                 175

Cys Met Arg Val Glu Leu Tyr Gly Cys Val Trp Leu Asp Gly Leu Val
            180                 185                 190

Ser Tyr Asn Ala Pro Ala Gly Gln Gln Phe Val Leu Pro Gly Gly Ser
        195                 200                 205

Ile Ile Tyr Leu Asn Asp Ser Val Tyr Asp Gly Ala Val Gly Tyr Ser
    210                 215                 220

Met Thr Glu Gly Leu Gly Gln Leu Thr Asp Gly Val Ser Gly Leu Asp
225                 230                 235                 240

Asp Phe Thr Gln Thr His Glu Tyr His Val Trp Pro Gly Tyr Asp Tyr
                245                 250                 255

Val Gly Trp Arg Asn Glu Ser Ala Thr Asn Gly Tyr Ile Glu Ile Met
            260                 265                 270

Phe Glu Phe Asp Arg Ile Arg Asn Phe Thr Thr Met Lys Val His Cys
        275                 280                 285

Asn Asn Met Phe Ala Lys Gly Val Lys Ile Phe Lys Glu Val Gln Cys
    290                 295                 300

Tyr Phe Arg Ser Glu Ala Ser Glu Trp Glu Pro Asn Ala Ile Ser Phe
305                 310                 315                 320

Pro Leu Val Leu Asp Asp Val Asn Pro Ser Ala Arg Phe Val Thr Val
                325                 330                 335

Pro Leu His His Arg Met Ala Ser Ala Ile Lys Cys Gln Tyr His Phe
            340                 345                 350

Ala Asp Thr Trp Met Met Phe Ser Glu Ile Thr Phe Gln Ser Asp Ala
        355                 360                 365

Ala Met Tyr Asn Asn Ser Glu Ala Leu Pro Thr Ser Pro Met Ala Pro
    370                 375                 380

Thr Thr Tyr Asp Pro Met Leu Lys Val Asp Asp Ser Asn Thr Arg Ile
385                 390                 395                 400

Leu Ile Gly Cys Leu Val Ala Ile Ile Phe Ile Leu Leu Ala Ile Ile
                405                 410                 415

Val Ile Ile Leu Trp Arg Gln Phe Trp Gln Lys Met Leu Glu Lys Ala
            420                 425                 430

Ser Arg Arg Met Leu Asp Asp Glu Met Thr Val Ser Leu Ser Leu Pro
        435                 440                 445

Ser Asp Ser Ser Met Phe Asn Asn Asn Arg Ser Ser Ser Pro Ser Glu
    450                 455                 460

Gln Gly Ser Asn Ser Thr Tyr Asp Arg Ile Phe Pro Leu Arg Pro Asp
465                 470                 475                 480

Tyr Gln Glu Pro Ser Arg Leu Ile Arg Lys Leu Pro Glu Phe Ala Pro
                485                 490                 495

Gly Glu Glu Glu Ser Gly Cys Ser Gly Val Val Lys Pro Val Gln Pro
            500                 505                 510

Ser Gly Pro Glu Gly Val Pro His Tyr Ala Glu Ala Asp Ile Val Asn
        515                 520                 525

Leu Gln Gly Val Thr Gly Gly Asn Thr Tyr Ser Val Pro Ala Val Thr
    530                 535                 540

Met Asp Leu Leu Ser Gly Lys Asp Val Ala Val Glu Glu Phe Pro Arg
545                 550                 555                 560

Lys Leu Leu Thr Phe Lys Glu Lys Leu Gly Glu Gly Gln Phe Gly Glu
                565                 570                 575

Val His Leu Cys Glu Val Glu Gly Met Glu Lys Phe Lys Asp Lys Asp
            580                 585                 590

Phe Ala Leu Asp Val Ser Ala Asn Gln Pro Val Leu Val Ala Val Lys
        595                 600                 605

Met Leu Arg Ala Asp Ala Asn Lys Asn Ala Arg Asn Asp Phe Leu Lys

    610                 615                 620

Glu Ile Lys Ile Met Ser Arg Leu Lys Asp Pro Asn Ile Ile His Leu
625                 630                 635                 640

Leu Ala Val Cys Ile Thr Asp Asp Pro Leu Cys Met Ile Thr Glu Tyr
                645                 650                 655

Met Glu Asn Gly Asp Leu Asn Gln Phe Leu Ser Arg His Glu Pro Pro
            660                 665                 670

Asn Ser Ser Ser Ser Asp Val Arg Xaa Val Ser Tyr Thr Asn Leu Lys
        675                 680                 685

Phe Met Ala Thr Gln Ile Ala Ser Gly Met Lys Tyr Leu Ser Ser Leu
    690                 695                 700

Asn Phe Val His Arg Asp Leu Ala Thr Arg Asn Cys Leu Val Gly Lys
705                 710                 715                 720

Asn Tyr Thr Ile Lys Ile Ala Asp Phe Gly Met Ser Arg Asn Leu Tyr
                725                 730                 735

Ser Gly Asp Tyr Tyr Arg Ile Gln Gly Arg Ala Val Leu Pro Ile Arg
            740                 745                 750

Trp Met Ser Trp Glu Ser Ile Leu Leu Gly Lys Phe Thr Thr Ala Ser
        755                 760                 765

Asp Val Trp Ala Phe Gly Val Thr Leu Trp Glu Thr Phe Thr Phe Cys
    770                 775                 780

Gln Glu Gln Pro Tyr Ser Gln Leu Ser Asp Glu Gln Val Ile Glu Asn
785                 790                 795                 800

Thr Gly Glu Phe Phe Arg Asp Gln Gly Arg Gln Thr Tyr Leu Pro Gln
                805                 810                 815

Pro Ala Ile Cys Pro Asp Ser Val Tyr Lys Leu Met Leu Ser Cys Trp
            820                 825                 830

Arg Arg Asp Thr Lys Asn Arg Pro Ser Phe Gln Glu Ile His Leu Leu
        835                 840                 845

Leu Leu Gln Gln Gly Asp Glu
    850                 855

<210>  6
<211>  15
<212>  DNA
<213>  Homo sapiens

<400>  6
tgtacgcayt gtcag                                                                15